# FASCICULE DE BREVET EUROPEEN

(11) **EP 1 458 697 B1**
(45) Date de publication et mention de la délivrance du brevet: **06.09.2006**
(21) Numéro de dépôt: 02805790.9
(22) Date de dépôt: 09.12.2002
(51) Int. Cl.: C07D 277/34, C07D 417/12, C07C 233/87, C07C 235/78, C07C 235/66, A61K 31/426, A61K 31/427, A61K 8/49, A61Q 19/00, A61P 17/00

(54) **BIPHENYLMETHYL-THIAZOLIDINEDIONES ET ANALOGUES ET LEUR UTILISATION COMME ACTIVATEUR PPAR-GAMMA**
BIPHENYLMETHYLTHIAZOLIDINDIONE UND ANALOGA UND DEREN VERWENDUNG ALS PPAR-GAMMA-AKTIVATOREN
BIPHENYLMETHYL-THIAZOLIDINEDIONES AND ANALOGUES AND THEIR USE AS PPAR-GAMMA ACTIVATORS

(30) Priorité: 21.12.2001 FR 0116750
(43) Date de publication de la demande: 22.09.2004
(73) Titulaire: Galderma Research & Development, S.N.C., 06560 Valbonne, Sophia Antipolis (FR)
(72) Inventeur: BERNARDON, Jean-Michel, F-06000 Nice (FR); CLARY, Laurence, 06480 La Colle Sur Loup (FR); TERRANOVA, Eric, F-06520 Magagnosc (FR)
(74) Mandataire: Bernstein, Claire Jacqueline
(86) Numéro de dépôt international: PCT/FR2002/004232
(87) Numéro de publication internationale: WO 2003/055867

(56) Documents cités:
- EP-A- 1 067 109
- WO-A-01/14349
- WO-A-02/12210
- NOMURA M ET AL: "(3-Substituted benzyl)thiazolidine-2,4-diones as structurally new antihyperglycemic agents" BIOORGANIC & MEDICINAL CHEMISTRY LETTERS, vol. 9, no. 4, 22 février 1999 (1999-02-22), pages 533-538, XP004156083

## Description

L'invention se rapporte, à titre de produits industriels nouveaux et utiles, à des composés bi-aromatiques activateurs des récepteurs de type Peroxisome Proliferator-Activated Receptor de sous-type γ (PPARγ). Elle se rapporte également à leur procédé de préparation et à leur utilisation dans des compositions pharmaceutiques destinées à un usage en médecine humaine ou vétérinaire, ou bien encore dans des compositions cosmétiques.

L'activité des récepteurs de type PPARs a fait l'objet de nombreuses études. On peut citer à titre indicatif la publication intitulée "Differential Expression of Peroxisome Proliferator-Activated Receptor Subtypes During the Differentiation of Human Keratinocytes", Michel Rivier *et al.,* J. Invest. Dermatol 111, 1998, p. 1116-1121, dans laquelle est répertorié un grand nombre de références bibliographiques concernant les récepteurs de type PPARs. On peut également citer à titre indicatif, le dossier intitulé "The PPARs : From orphan receptors to Drug Discovery", Timothy M. Willson, Peter J. Brown, Daniel D.Sternbach, et Brad R. Henke, J. Med.Chem., 2000, Vol. 43, p. 527-550.

Les récepteurs PPARs activent la transcription en se liant à des éléments de séquences d'ADN, appelés les éléments de réponse des proliférateurs de peroxysome (PPRE), sous forme d'un hétérodimère avec les récepteurs X des rétinoides (appelés les RXRs).

Trois sous-types de PPARs humains ont été identifiés et décrits : les PPARα, PPARγ et PPARδ (ou NUC1).
PPARα est principalement exprimé dans le foie alors que PPARδ est ubiquitaire.

PPARγ est le plus étudié des trois sous-types. L'ensemble des références suggèrent un rôle critique des récepteurs PPARγ dans la régulation de la différentiation des adipocytes, où il est fortement exprimé. II joue également un rôle clé dans l'homéostasie lipidique systémique.

Il a été notamment décrit dans la demande de brevet WO 96/33724 que des composés sélectifs des PPARγ, tels qu'une prostaglandine -J2 ou -D2, sont des actifs potentiels pour le traitement de l'obésité et du diabète.

La demande de brevet WO 01/14349 décrit quant à elle des dérivés substitués de benzylthiazolidine-2,4-dione comme ligands des récepteurs PPAR.

Par ailleurs, la Demanderesse a déjà décrit dans la demande de brevet WO 99/34783 l'utilisation de composés activateurs de PPARγ dans la préparation d'une composition pharmaceutique, la composition étant destinée à traiter les désordres cutanés liés à une anomalie de la différentiation des cellules épidermiques.

Un des buts de la présente invention est de proposer de nouveaux composés activateurs des PPARγ présentant une meilleure activité biologique que des composés de l'art antérieur.

Ainsi, la présente invention concerne des composés bi-aromatiques répondant à la formule générale suivante : 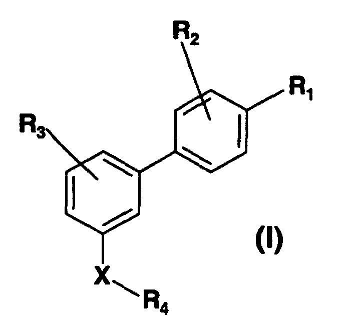 dans laquelle :
- R₁ représente un radical de formules (a) ou (b) suivantes : 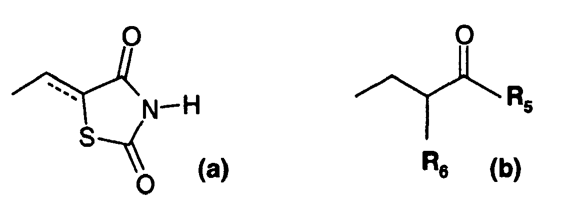 R₅ et R₆ ayant les significations données ci-après,
- R₂ et R₃ identiques ou différents représentent un atome d'hydrogène, un radical alkyle ayant de 1 à 6 atomes de carbone, un radical aryle, un atome d'halogène, un radical -OR₇, un radical polyéther, un radical nitro ou un radical amino pouvant éventuellement être substitué par des radicaux alkyles ayant de 1 à 6 atomes de carbone ;
   R₇ ayant la signification donnée ci-après,
- X représente les liaisons de structures suivantes :

   -CH₂-N(R₈)-CO-

   -N(R₈)-CO-N(R₉)-

   -N(R₈)-CO-CH₂-

   -N(R₈)-CH₂-CO-

   pouvant être lues de gauche à droite ou inversement
   R₈ et R₉ ayant les significations données ci-après,
- R₄ représente :
   - un radical phényle, benzyle, phénéthyle, thiényle, furyle ou pyridyle, tous ces radicaux étant substitués par un groupement R₁₀,
      R₁₀ ayant les significations données ci-après,
   - un radical pyrrolyle, pyrazinyle, naphthyle, biphényle, indolyle, indényle, benzothiényle, benzofuryle, benzothiazolyle ou quinolyle, tous ces radicaux pouvant être mono ou di-substitués par un groupement R₁₁ et/ou R₁₂,
      R₁₁ et R₁₂ ayant les significations données ci-après,
   - un radical -(CH₂)ₙ-(CO)_{q}R₁₃,
      n, q et R₁₃ ayant les significations données ci-après,
   - un radical adamantyle, diphénylméthyl, diphényléthyle, diphénylpropyle, diphénylbutyle, cyclopropylméthyle, cyclopentyléthyle, 2-benzimidazolyléthyle, cyclohexyl méthyle, phénoxyphényl, 9H-fluorényl, benzyloxyphényl, 4-heptyloxyphényl, ou 4-(6-methyl-2-benzothiazolyl)phenyl,
   - un radical -(CH₂)ₙ-O-R₁₃,
      n et R₁₃ ayant les significations données ci-après,
- R₅ représente un radical hydroxy ou un radical alkoxy ayant de 1 à 9 atomes de carbone,
- R₆ représente un radical alkyle ayant de 1 à 6 atomes de carbone, un radical OR₁₄ ou un radical SR₁₄,
   R₁₄ ayant les significations données ci-après,
- R₇ représente un atome d'hydrogène, un radical alkyle ayant de 1 à 6 atomes de carbone, un radical aryle ou un radical aralkyle,
- R₈ représente un atome d'hydrogène ou un radical alkyle ayant de 1 à 6 atomes de carbone,
- R₉ représente un atome d'hydrogène ou un radical alkyle ayant de 1 à 6 atomes de carbone,
- R₁₀ représente : un radical -S(O)ₘR₁₅
   un radical -(CH2)ₚ-COR₁₆
   un radical -O-R₁₇
   m, p, R₁₅, R₁₆, R₁₇ ayant les significations données ci-après,
- R₁₁ et R₁₂ représentent un atome d'halogène, un radical CF₃, un radical alkyle ayant de 1 à 12 atomes de carbone, un radical alkoxy ayant de 1 à 9 atomes de carbone, un radical polyéther, une fonction nitro, un radical hydroxyle éventuellement protégé par un groupe acétyle, benzoyle, une fonction amino éventuellement substituée par au moins un alkyle ayant de 1 à 12 atomes de carbone ou par un radical -CONH-R₂₄, ou protégée par un groupe acétyle ou benzoyle, un radical -S(O)ₘR₁₅, un radical (CH₂)ₚ-COR₁₆ ou un radical -OR₁₇,
   m, p, R₁₅, R₁₆, R₁₇, R₂₄ ayant les significations données ci-après,
- n peut prendre les valeurs allant de 1 à 9,
- q peut prendre les valeurs 0 ou 1,
- R₁₃ représente un radical -OR₁₈, un radical -N(R₁₉)(R₂₀), un radical aryle, un radical aralkyle ou un radical hétéroaryle,
   R₁₈, R₁₉, R₂₀ ayant les significations données ci-après,
- m peut prendre les valeurs 0, 1 ou 2,
- p peut prendre les valeurs 0, 1 ou 2,
- R₁₄ représente un radical alkyle ayant de 1 à 12 atomes de carbone, un radical CF₃, un radical aryle ou un radical aralkyle,
- R₁₅ représente un radical alkyle ayant de 1 à 12 atomes de carbone, un radical aryle ou un radical aralkyle,
- R₁₆ représente un radical alkyle ayant de 1 à 12 atomes de carbone, un radical - OR₂₁, un radical -N(R₂₂)(R₂₃), un radical aryle ou un radical aralkyle,
   R₂₁, R₂₂, R₂₃ ayant les significations données ci-après,
- R₁₇ représente un radical aryle ou un radical aralkyle,
- R₁₈ représente un atome d'hydrogène ou un radical alkyle ayant de 1 à 12 atomes de carbone,
- R₁₉ et R₂₀ identiques ou différents représentent un atome d'hydrogène, un radical alkyle ayant de 1 à 12 atomes de carbone, ou pris ensemble peuvent former un hétérocycle,
- R₂₁ représente un atome d'hydrogène ou un radical alkyle ayant de 1 à 12 atomes de carbone,
- R₂₂ et R₂₃ identiques ou différents représentent un atome d'hydrogène, un radical alkyle ayant de 1 à 12 atomes de carbone, ou pris ensemble peuvent former un hétérocycle,
- R₂₄ représente un radical phényl, diphénylméthyl, diphénylpropyle, diphénylbutyle, biphénylyl, phénoxyphényl, 9H-fluorényl, 4-benzyloxyphényl, 4-heptyloxyphényl, ou 4-(6-methyl-2-benzothiazolyl)phenyl.

La présente invention concerne également les sels des composés de formule (I) lorsque R₁ contient une fonction acide carboxylique ainsi que les isomères optiques et géométriques desdits composés de formule (I).

Lorsque les composés selon l'invention se présentent sous forme d'un sel, il s'agit de préférence d'un sel d'un métal alcalin ou alcalino-terreux, ou encore d'un sel de zinc ou de sels d'une amine organique.

Selon la présente invention :

Par radical alkyle ayant de 1 à 6 atomes de carbone, on entend de préférence les radicaux méthyle, éthyle, isopropyle, butyle, tertiobutyle, hexyle.

Par radical alkyle ayant de 1 à 12 atomes de carbone, on entend de préférence les radicaux méthyle, éthyle, isopropyle, butyle, tertiobutyle, hexyle, octyle, décyle, dodécyle.

Par radical polyéther, on entend de préférence un radical ayant de 1 à 6 atomes de carbone interrompu par au moins un atome d'oxygène tel que les radicaux méthoxyméthoxy, éthoxyméthoxy, méthoxyéthoxyméthoxy.

Par atome d'halogène, on entend de préférence un atome de fluor, de chlore, de brome.

Par radical alkoxy ayant de 1 à 9 atomes de carbone, on entend de préférence les radicaux méthoxy, éthoxy, isopropyloxy, tertio-butoxy, hexyloxy.

Par radical aryle, on entend de préférence un radical phényle, biphényl, cinnamyle ou naphtyle pouvant être mono ou di-substitué par un atome d'halogène, un radical CF₃, un radical alkyle ayant de 1 à 12 atomes de carbone, un radical alkoxy ayant de 1 à 7 atomes de carbone, une fonction nitro, un radical polyéther, un radical aryle, un radical benzoyle, un groupement alkyl ester, un acide carboxylique, un radical hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

Par radical aralkyle, on entend de préférence un radical benzyle ou phénethyle pouvant être mono ou di-substitué par un atome d'halogène, un radical CF₃, un radical alkyle ayant de 1 à 12 atomes de carbone, un radical alkoxy ayant de 1 à 6 atomes de carbone, une fonction nitro, un radical polyéther, un radical hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

Par radical hétéroaryle, on entend de préférence un radical aryle interrompu par un ou plusieurs hétéroatomes, tel le radical pyridyle, furyle, thiényle, isoxazolyle, oxadiazolyle, oxazolyle, benzimidazole, indolyle, benzofurane, éventuellement substitué par au moins un halogène, un alkyle ayant de 1 à 12 atomes de carbone, un alkoxy ayant de 1 à 7 atomes de carbone, un radical aryle, une fonction nitro, un radical polyéther, un radical aryle, un radical benzoyle, un groupement alkyl ester, un acide carboxylique, un hydroxyle éventuellement protégé par un groupe acétyle, benzoyle ou une fonctio amino éventuellement protégée par un groupe acétyle, benzoyle ou éventuellement substituée par au moins un alkyle ayant de 1 à 12 atomes de carbone.

Par hétérocycle, on entend de préférence un radical morpholino, pipéridino, pipérazino, 2-oxo-pipéridin-1-yle et 2-oxo-pyrrolidin-1-yle, substitués éventuellement par au moins un alkyle ayant de 1 à 12 atomes de carbone, un alkoxy ayant de 1 à 7 atomes de carbone, un radical aryle, une fonction nitro, un radical polyéther, un radical aryle, un radical benzoyle, un groupement alkyl ester, un acide carboxylique, un hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

Parmi les composés répondant à la formule générale (I) ci-dessus, on peut citer les suivants, seuls ou en mélange :
1- 7-{[4'-(2,4-Dioxo-thiazolidin-5-ylmethyl)-biphenyl-3-ylmethyl]-methyl-carbamoyl}-heptanoate de méthyle ;
2- 9-{[4'-(2,4-Dioxo-thiazolidin-5-ylmethyl)-biphenyl-3-ylmethyl]-methyl-carbamoyl}-nonanoate de méthyle ;
3- N-[4'-(2,4-Dioxo-thiazolidin-5-ylmethyl)-biphenyl-3-ylmethyl]-N-methyl-terephthalamate de méthyle ;
4- 3-Cyclopentyl-N-[4'-(2,4-dioxo-thiazolidin-5-ylmethyl)-biphenyl-3-ylmethyl]-N-methyl-propionamide ;
5- N-[4'-(2,4-dioxo-thiazolidin-5-ylmethyl)-biphenyl-3-ylmethyl]-N-methyl-naphthalene-1-carboxamide ;
6- N-[4'-(2,4-dioxo-thiazolidin-5-ylmethyl)-biphenyl-3-ylmethyl]-N-methyl-naphthalene-2-carboxamide ;
7- N-[4'-(2,4-Dioxo-thiazolidin-5-ylmethyl)-biphenyl-3-ylmethyl]-N-methyl-2-phenoxy-Acetamide ;
8- N-[4'-(2,4-dioxo-thiazolidin-5-ylmethyl)-biphenyl-3-ylmethyl]-N-methyl-1-Methyl-1H-pyrrole-2-carboxamide ;
9- N-[4'-(2,4-dioxo-thiazolidin-5-ylmethyl)-biphenyl-3-ylmethyl]-N-methyl-adamantane-1 - carboxamide ;
10- N-[4'-(2,4-dioxo-thiazolidin-5-ylmethyl)-biphenyl-3-ylmethyl]-N-methyl-biphenyl-4-carboxamide ;
11- N-[4'-(2,4-dioxo-thiazolidin-5-ylmethyl)-biphenyl-3-ylmethyl]-N-methyl-benzo[b]thiophene-2-carboxamide;
12- N-[4'-(2,4-dioxo-thiazolidin-5-ylmethyl)-biphenyl-3-ylmethyl]-N-methyl-6-oxo-6-phenyl-hexanamide ;
13- 4-Dimethylamino-N-[4'-(2,4-dioxo-thiazolidin-5-ylmethyl)-biphenyl-3-ylmethyl]-N-methyl-naphthalene-1-carboxamide ;
14- N-[4'-(2,4-Dioxo-thiazolidin-5-ylmethyl)-biphenyl-3-ylmethyl]-4-methanesulfonyl-N-methyl-benzamide ;
15- N-[4'-(2,4-Dioxo-thiazolidin-5-ylmethyl)-biphenyl-3-ylmethyl]-N-methyl-4-(1-phenyl-methanoyl)-benzamide ;
16- 6-(2-Methoxy-ethoxymethoxy)-N-[4'-(2,4-dioxo-thiazolidin-5-ylmethyl)-biphenyl-3-ylmethyl]-N-methyl-naphthalene-2-carboxamide ;
17- 6-Hydroxy-N-[4'-(2,4-dioxo-thiazolidin-5-ylmethyl)-biphenyl-3-ylmethyl]-N-methyl-naphthalene-2-carboxamide ;
18- N-[4'-(2,4-Dioxo-thiazolidin-5-ylmethyl)-biphenyl-3-ylmethyl]-N-methyl-4-methylsulfanyl-benzamide ;
19- Acide (S)-3-(3'-{[(1-Biphenyl-4-yl-methanoyl)-methyl-amino]-methyl}-biphenyl-4-yl)-2-ethoxy-propionique ;
20- Acide (S)-2-Ethoxy-3-(3'-{[methyl-(6-oxo-6-phenyl-hexanoyl)-amino]-methyl}-biphenyl-4-yl)-propionique ;
21- 1-[4'-(2,4-Dioxo-thiazolidin-5-ylmethyl)-biphenyl-3-yl]-1-methyl-3-naphthalen-2-yl-urée ;
22- 3-(4-Dimethylamino-phenyl)-1-[4'-(2,4-dioxo-thiazolidin-5-ylmethyl)-biphenyl-3-yl]-1-methyl-urée ;
23- Acide (S)-2-Ethoxy-3-{3'-[({1-[6-(2-methoxy-ethoxymethoxy)-naphthalen-2-yl]-methanoyl}-methyl-amino)-methyl]-biphenyl-4-yl}-propionique ;
24- 6-(Methoxymethoxy)-N-[4'-(2,4-dioxo-thiazolidin-5-ylmethyl)-biphenyl-3-ylmethyl]-N-methyl-naphthalene-2-carboxamide ;
25- 6-(Methoxycarbonyl)-N-[4'-(2,4-dioxo-thiazolidin-5-ylmethyl)-biphenyl-3-ylmethyl]-N-methyl-naphthalene-2-carboxamide ;
26- 6-(Propyloxy)-N-[4'-(2,4-dioxo-thiazolidin-5-ylmethyl)-biphenyl-3-ylmethyl]-N-methyl-naphthalene-2-carboxamide ;
27- 6-(Hexyloxy)-N-[4'-(2,4-dioxo-thiazolidin-5-ylmethyl)-biphenyl-3-ylmethyl]-N-methyl-naphthalene-2-carboxamide ;
28- 6-(Nonyloxy)-N-[4'-(2,4-dioxo-thiazolidin-5-ylmethyl)-biphenyl-3-ylmethyl]-N-methyl-naphthalene-2-carboxamide ;
29- N-[4'-(2,4-dioxo-thiazolidin-5-ylmethyl)-biphenyl-3-ylmethyl]-N-methyl-4'-propyl-biphenyl-2-carboxamide ;
30- N-[4'-(2,4-dioxo-thiazolidin-5-ylmethyl)-biphenyl-3-ylmethyl]-N-methyl-4-phenoxy-benzamide ;
31- N-[4'-(2,4-dioxo-thiazolidin-5-ylmethyl)-biphenyl-3-ylmethyl]-N-methyl-7-oxo-7-phenyl-heptanamide ;
32- Acide (6-{[4'-(2,4-Dioxo-thiazolidin-5-ylmethyl)-biphenyl-3-ylmethyl]-methyl-carbamoyl}-naphthalen-2-yloxy)-acetique ;
33- Ester méthylique de l'acide (6-{[4'-(2,4-Dioxo-thiazolidin-5-ylmethyl)-biphenyl-3-ylmethyl]-méthyl-carbamoyl}-naphthalen-2-yloxy)-acetique ;
34- 6-Methoxy-N-[4'-(2,4-dioxo-thiazolidin-5-ylmethyl)-biphenyl-3-ylmethyl]-N-methyl-naphtalène-2-carboxamide ;
35- 6-Acetoxy-N-[4'-(2,4-dioxo-thiazolidin-5-ylmethyl)-biphenyl-3-ylmethyl]-N-methyl-naphtalène-2-carboxamide ;
36- 6-Amino-N-[4'-(2,4-dioxo-thiazolidin-5-ylmethyl)-biphenyl-3-ylmethyl]-N-methyl-naphtalène-2-carboxamide ;
37- 6-Acetylamino-N-[4'-(2,4-dioxo-thiazolidin-5-ylmethyl)-biphenyl-3-ylmethyl]-N-methyl-naphtalène-2-carboxamide ;
38- 1-Hydroxy-N-[4'-(2,4-dioxo-thiazolidin-5-ylmethyl)-biphenyl-3-ylmethyl]-N-methyl-naphtalène-2-carboxamide ;
39- 1-Methoxy-N-[4'-(2,4-dioxo-thiazolidin-5-ylmethyl)-biphenyl-3-ylmethyl]-N-methyl-naphtalène-2-carboxamide ;
40- 6-Bromo-N-[4'-(2,4-dioxo-thiazolidin-5-ylmethyl)-biphenyl-3-ylmethyl]-N-methyl-naphtalène-2-carboxamide ;
41- Acide 6-carboxylique-N-[4'-(2,4-dioxo-thiazolidin-5-ylmethyl)-biphenyl-3-ylmethyl]-N-methyl-naphtalène-2-carboxamide ;
42- Ester methylique de l'acide 6-carboxylique-N-[4'-(2,4-dioxo-thiazolidin-5-ylmethyl)-biphenyl-3-ylmethyl]-N-methyl-naphtalène-2-carboxamide ;
43- 6-(3-Phenyl-ureido)-N-[4'-(2,4-dioxo-thiazolidin-5-ylmethyl)-biphenyl-3-ylmethyl]-N-methyl-naphtalène-2-carboxamide ;
44- 3-Hydroxy-N-[4'-(2,4-dioxo-thiazolidin-5-ylmethyl)-biphenyl-3-ylmethyl]-N-methyl-naphtalène-2-carboxamide ;
45- 3-Methoxy-N-[4'-(2,4-dioxo-thiazolidin-5-ylmethyl)-biphenyl-3-ylmethyl]-N-methyl-naphtalène-2-carboxamide ;
46- N-[4'-(2,4-dioxo-thiazolidin-5-ylmethyl)-biphenyl-3-ylmethyl]-N-methyl-4'-hydroxy-biphenyl-4-carboxamide ;
47- N-[4'-(2,4-dioxo-thiazolidin-5-ylmethyl)-biphenyl-3-ylmethyl]-N-methyl-4'-methoxy-biphenyl-4-carboxamide ;
48- N-[4'-(2,4-dioxo-thiazolidin-5-ylmethyl)-biphenyl-3-ylmethyl]-N-methyl-4'-propyloxy-biphenyl-4-carboxamide ;
49- N-[4'-(2,4-dioxo-thiazolidin-5-ylmethyl)-biphenyl-3-ylmethyl]-N-methyl-4'-hexyloxy-biphenyl-4-carboxamide ;
50- N-[4'-(2,4-dioxo-thiazolidin-5-ylmethyl)-biphenyl-3-ylmethyl]-N-methyl-4'-acetoxy-biphenyl-4-carboxamide ;
51- Acide (4'-{[4'-(2,4-Dioxo-thiazolidin-5-ylmethyl)-biphenyl-3-ylmethyl]-methyl-carbamoyl}-biphenyl-4-yloxy)-acetique ;
52- Ester méthylique de l'acide (4'-{[4'-(2,4-Dioxo-thiazolidin-5-ylmethyl)-biphenyl-3-ylmethyl]-methyl-carbamoyl}-biphenyl-4-yloxy)-acetique ;
53- N-[4'-(2,4-dioxo-thiazolidin-5-ylmethyl)-biphenyl-3-ylmethyl]-N-methyl-4'-methoxymethoxy-biphenyl-4-carboxamide ;
54- N-[4'-(2,4-dioxo-thiazolidin-5-ylmethyl)-biphenyl-3-ylmethyl]-N-methyl-4'-nonyloxybiphenyl-4-carboxamide ;
55- N-[4'-(2,4-dioxo-thiazolidin-5-ylmethyl)-biphenyl-3-ylmethyl]-N-methyl-4'-(2-methoxy-ethoxy)-biphenyl-4-carboxamide ;
56- 3-Biphenyl-4-yl-1-[4'-(2,4-dioxo-thiazolidin-5-ylmethyl)-biphenyl-3-yl]-1-methyl-urea ;
57- 1-[4'-(2,4-Dioxo-thiazolidin-5-ylmethyl)-biphenyl-3-yl]-3-(9H-fluoren-2-yl)-1-methylurea ;
58- 1-[4'-(2,4-Dioxo-thiazolidin-5-ylmethyl)-biphenyl-3-yl]-3-(9H-fluoren-9-yl)-1-methylurea ;
59- 3-Benzhydryl-1-[4'-(2,4-dioxo-thiazolidin-5-ylmethyl)-biphenyl-3-yl]-1-methyl-urea ;
60- 1-[4'-(2,4-Dioxo-thiazolidin-5-ylmethyl)-biphenyl-3-yl]-1-methyl-3-(3-phenoxy-phenyl)-urea ;
61- 1-[4'-(2,4-Dioxo-thiazolidin-5-ylmethyl)-biphenyl-3-yl]-3-(4-heptyloxy-phenyl)-1-methyl-urea ;
62- 3-(4-Benzyloxy-phenyl)-1-[4'-(2,4-dioxo-thiazolidin-5-ylmethyl)-biphenyl-3-yl]-1-methyl-urea ;
63- 1-[4'-(2,4-Dioxo-thiazolidin-5-ylmethyl)-biphenyl-3-yl]-1-methyl-3-[4-(6-methyl-benzothiazol-2-yl)-phenyl]-urea ;
64- 4'-(2-methoxy-ethoxymethoxy)-N-[4'-(2,4-dioxo-thiazolidin-5-ylmethyl)-biphenyl-3-ylmethyl]-N-methyl-biphenyl-4-carboxamide ;
65- 4'-hydroxy-N-[4'-(2,4-dioxo-thiazolidin-5-ylmethyl)-biphenyl-3-ylmethyl]-N-methyl-biphenyl-4-carboxamide ;
66- 1-[4'-(2,4-dioxo-thiazolidin-5-ylmethyl)-biphenyl-3-yl]-3-(4-hexyloxy-phenyl)-1-methyl-urée.

Selon la présente invention les composés de formule (I) plus particulièrement préférés sont ceux pour lesquels :
- R₁ représente le radical de formule (a) ou le radical de formule (b) avec R₅ représente un radical hydroxyle et R₆ représente le radical OR₁₄, et/ou
- X représente la liaison de structure -CH₂-N(R₈)-CO- ou -N(R₈)-CO-N(R₉)- lue de gauche à droite ou inversement.

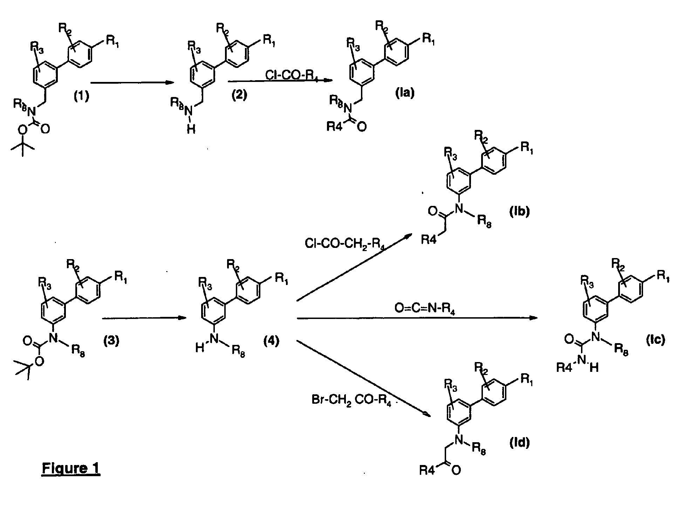

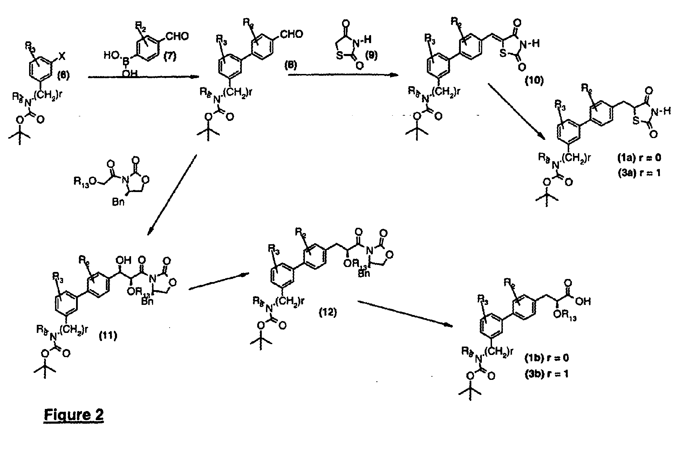

La présente invention a également pour objet les procédés de préparation des composés de formule (I), en particulier selon les schémas réactionnels donnés aux figures 1 et 2.

Dans la figure 1, les dérivés de formule (1a) et (1b) peuvent être obtenus respectivement à partir des dérivés (2) et (4) par acylation de la fonction amine avec une forme activée d'un acide carboxylique, par exemple un chlorure d'acide (Cl-CO-R₄), en présence d'une amine tertiaire (par exemple triéthylamine ou pyridine) dans un solvant anhydre de préférence le THF. Les dérivés (2) et (4) pouvant être obtenus respectivement à partir des composés (1) et (3) par déprotection de la fonction amine en présence d'anhydride trifluoroacétique ou d'acide chlorhydrique dans un solvant telle THF ou le dichlorométhane.

Les dérivés de formule (1c) peuvent être obtenus (Figure 1) à partir des dérivés (4) par réaction avec un isocyanate de formule O=C=N-R₄ dans un solvant tel le dichlorométhane en présence d'une base telle la triéthylamine.

Les dérivés de formule (Id) peuvent être obtenus (Figure 1) à partir des dérivés (4) par réaction avec une alpha-bromocétone (Br-CH₂-CO-R₄) dans un solvant tel l'acétone ou la méthyléthylcétone en présence d'une base telle le carbonate de potassium.

Les dérivés (1a) et (3a) peuvent être obtenus (Figure 2) à partir des composés (10) soit par hydrogénation en présence de Palladium sur charbon ou de Nickel Raney dans un solvant tel l'acétate d'éthyle, le dioxanne, le DMF ou l'alcool éthylique soit par réduction en présence de borohydrure de lithium et de pyridine dans le THF. Les composés (10) peuvent être obtenus à partir des composés (8) par réaction avec la 2,4-thizolidinedione (9) en présence d'acétate de pipéridine dans un solvant alcoolique tel l'éthanol ou dans le toluène. Les composés (8) peuvent être obtenus à partir des dérivés halogénés (6), de préférence iodés ou bromés par une réaction de couplage de type Suzuki avec un acide boronique (7). Cette réaction est effectuée en présence d'un catalyseur au palladium, par exemple le tétrakis(triphénylphosphine)palladium selon les conditions décrites par N. Miyaura *et al.* Synthetic Communications (1981) 11 (7), 513-519. Les dérivés boroniques (7) pouvant être obtenus à partir des dérivés halogénés correspondants (de préférence iodés ou bromés) tout d'abord par protection de la fonction aldéhyde sous forme d'acétal puis transformation en lithien, réaction avec le borate de triméthyle ou de trüsopropyle et hydrolyse en milieu acide (acide chlorhydrique).
Les dérivés halogénés (6) sont obtenus à partir des amines primaires correspondantes. Ces dernières sont protégées par couplage avec du di-*tert*-butyl carbonate dans un solvant comme le dichlorométhane. Le carbamate résultant est alkylé via l'utilisation d'une base telle que l'hydrure de sodium et d'un halogénure d'alkyle pour donner le dérivé (6).

Les dérivés (1b) et (3b) peuvent être obtenus (Figure 2) à partir des dérivés (8) par une suite de réactions selon les conditions décrites par B. Hulin et al. J. Med. Chem. (1996) 39, 3897-3907.

Lorsque R₁ comporte une fonction acide, les composés sont préparés en protégeant R₁ par un groupe protecteur de type alkyle, allylique, benzylique ou tert-butylique. Le passage à la forme libre peut être effectué :
- dans le cas d'un groupe protecteur alkyle, au moyen de soude ou d'hydroxyde de lithium dans un solvant alcoolique tel le méthanol ou dans le THF ;
- dans le cas d'un groupe protecteur allylique, au moyen d'un catalyseur tel certains complexes de métaux de transition en présence d'une amine secondaire telle la morpholine ;
- dans le cas d'un groupe protecteur benzylique, par débenzylation en présence d'hydrogène au moyen d'un catalyseur tel que le palladium sur charbon ;
- dans le cas d'un groupe protecteur de type tert-butylique au moyen d'iodure de triméthylsilane.

Les composés selon l'invention présentent des propriétés d'activation des récepteurs de type PPARγ.

Par activateur des récepteurs de type PPARγ, on entend selon l'invention tout composé qui présente un pourcentage d'activation des récepteurs PPARγ d'au moins 20%, à la concentration de 1 µM, dans un test de transactivation tel que décrit dans l'exemple 35.

Les composés préférés de la présente invention présentent un pourcentage d'activation des récepteurs PPARγ supérieur ou égal à 40% et avantageusement supérieur ou égal à 70%.

De préférence, l'activateur des récepteurs de type PPARγ est spécifique, c'est à dire qu'il présente un rapport du pourcentage d'activation des récepteurs PPARγ, au pourcentage d'activation des récepteurs PPARα (calculé par rapport à un composé de référence, le Wy 14643, activant les PPARα de 100%) supérieur ou égal à 3. De préférence, ce rapport est supérieur ou égal à 5 et plus avantageusement supérieur ou égal à 10.

L'affinité des dérivés PPARs pour le récepteur PPARγ humain a également été déterminée dans un test de binding tel que décrit dans l'exemple 36. Par ligand des récepteur PPARγ, on entend tout composé selon l'invention présentant une valeur de Kd inférieure à 10000 nM. De préférence, les composés selon l'invention présentent une valeur de Kd inférieure à 1000 nM et avantageusement inférieure à 100 nM.

La présente invention a également pour objet les composés de formule (I) tels que décrits ci-dessus à titre de médicament.

Les composés selon l'invention conviennent particulièrement bien dans les domaines de traitement suivants :
1) pour traiter les affections dermatologiques liées à un désordre de la kératinisation portant sur la différenciation et sur la prolifération cellulaire notamment pour traiter les acnés vulgaires, comédoniennes, polymorphes, rosacées, les acnés nodulokystiques, conglobata, les acnés séniles, les acnés secondaires telles que l'acné solaire, médicamenteuse ou professionnelle ;
2) pour traiter d'autres types de troubles de la kératinisation, notamment les ichtyoses, les états ichtyosiformes, la maladie de Darrier, les kératodermies palmoplantaires, les leucoplasies et les états leucoplasiformes, le lichen cutané ou muqueux (buccal) ;
3) pour traiter d'autres affections dermatologiques avec une composante immuno-allergique inflammatoire, avec ou sans trouble de la prolifération cellulaire, et notamment toutes les formes de psoriasis, qu'il soit cutané, muqueux ou unguéal, et même le rhumatisme psoriasique, ou encore l'atopie cutanée, telle que l'eczéma ou l'atopie respiratoire ou encore l'hypertrophie gingivale ;
4) pour traiter toutes les proliférations dermiques ou épidermiques qu'elles soient bénignes ou malignes, qu'elles soient ou non d'origine virale telles que verrues vulgaires, les verrues planes et l'épidermodysplasie verruciforme, les papillomatoses orales ou florides, le lymphome T, et les proliférations pouvant être induites par les ultra-violets notamment dans le cas des épithélioma baso et spinocellulaires, ainsi que toute lésion précancéreuse cutanée telle que les kératoacanthomes ;
5) pour traiter d'autres désordres dermatologiques tels que les dermatoses immunes telles le lupus érythémateux, les maladies immunes bulleuses et les maladies du collagène, telle la sclérodermie ;
6) dans le traitement d'affections dermatologiques ou générales à composante immunologique ;
7) dans le traitement de désordres cutanés dus à une exposition aux rayonnements U.V. ainsi que pour réparer ou lutter contre le vieillissement de la peau, qu'il soit photo-induit ou chronologique ou pour réduire les pigmentations et les kératoses actiniques, ou toutes pathologies associées au vieillissement chronologique ou actinique, telle la xérose ;
8) pour lutter contre les troubles de la fonction sébacée tels que l'hyperséborrhée de l'acné ou la séborrhée simple ;
9) pour prévenir ou traiter les troubles de la cicatrisation, ou pour prévenir ou pour réparer les vergetures ;
10) dans le traitement des désordres de la pigmentation, tel l'hyperpigmentation, le mélasma, l'hypopigmentation ou le vitiligo ;
11) dans le traitement des affections du métabolisme des lipides, tel l'obésité, l'hyperlipidémie, ou le diabète non insulino-dépendant ;
12) dans le traitement d'affections inflammatoires telles que l'arthrite ;
13) dans le traitement ou la prévention des états cancéreux ou précancéreux ;
14) dans la prévention ou le traitement de l'alopécie de différentes origines, notamment l'alopécie due à la chimiothérapie ou aux rayonnements ;
15) dans le traitement des troubles du systèmes immunitaire, tel l'asthme, le diabète sucré de type I, la sclérose en plaque, ou autres disfonctionnements sélectifs du système immunitaire ; et
16) dans le traitement d'affections du système cardiovasculaire telles que l'artériosclérose
ou l'hypertension.

La présente invention a également pour objet une composition pharmaceutique comprenant, dans un milieu physiologiquement acceptable, au moins un composé de formule (I) tel que défini ci-dessus.

La présente invention a aussi pour objet l'utilisation des composés de formule (I) pour fabriquer une composition destinée au traitement des affections susmentionnées, en particulier pour réguler et/ou restaurer le métabolisme des lipides cutanés.

L'administration de la composition selon l'invention peut être effectuée par voie entérale, parentérale topique ou oculaire. De préférence, la composition pharmaceutique est conditionnée sous une forme convenant à une application par voie topique.

Par voie entérale, la composition, peut se présenter sous forme de comprimés, de gélules, de dragées, de sirops, de suspensions, de solutions, de poudres, de granulés, d'émulsions, de suspensions de microsphères ou nanosphères ou de vésicules lipidiques
ou polymériques permettant une libération contrôlée. Par voie parentérale, la composition peut se présenter sous forme de solutions ou suspensions pour perfusion ou pour injection.

Les composés selon l'invention sont généralement administrés à une dose journalière d'environ 0,001 mg/kg à 100 mg/kg de poids corporel, en 1 à 3 prises.

Les composés sont utilisés par voie systémique à une concentration généralement comprise entre 0,001% et 10% en poids, de préférence entre 0,01% et 1% en poids, par rapport au poids de la composition.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de suspensions de microsphères ou nanosphères ou de vésicules lipidiques ou polymériques ou de patches polymériques et d'hydrogels permettant une libération contrôlée. Cette composition par voie topique peut se présenter sous forme anhydre, sous forme aqueuse ou sous la forme d'une émulsion.

Les composés sont utilisés par voie topique à une concentration généralement comprise entre 0,001 % et 10% en poids, de préférence entre 0,01 % et 1 % en poids, par rapport au poids total de la composition.

Les composés de formule (I) selon l'invention trouvent également une application dans le domaine cosmétique, et plus particulièrement pour réguler et/ou restaurer le métabolisme des lipides cutanés, notamment en vue de prévenir et/ou de traiter les signes cutanés du vieillissement et/ou de la peau sèche.

L'invention a donc également pour objet une composition comprenant, dans un support cosmétiquement acceptable, au moins un des composés de formule (I).

L'invention a aussi pour objet l'utilisation cosmétique des composés de formule (I) pour l'hygiène corporelle ou capillaire.

La composition cosmétique selon l'invention contenant, dans un support cosmétiquement acceptable, au moins un composé de formule (I) ou l'un de ses isomères optiques ou géométriques ou l'un de ses sels, peut se présenter notamment sous forme d'une crème, d'un lait, d'une lotion, d'un gel, de suspensions de microsphères ou nanosphères ou vésicules lipidiques ou polymériques, d'un savon ou d'un shampooing.

La concentration en composé de formule (I) dans la composition cosmétique est de préférence comprise entre 0,001% et 3% en poids, par rapport au poids total de la composition.

Les compositions pharmaceutiques et cosmétiques telles que décrites précédemment peuvent en outre contenir des additifs inertes, ou même pharmacodynamiquement actifs pour ce qui concerne les compositions pharmaceutiques, ou des combinaisons de ces additifs, et notamment :
- des agents mouillants ;
- des agents d'amélioration de la saveur ;
- des agents conservateurs tels que les esters de l'acide parahydroxybenzoïque ;
- des agents stabilisants ;
- des agents régulateurs d'humidité ;
- des agents régulateurs de pH ;
- des agents modificateurs de pression osmotique ;
- des agents émulsionnants ;
- des filtres UV-A et UV-B ;
- des antioxydants, tels que l'α-tocophérol, le butylhydroxyanisole ou le butylhydroxytoluène, la Super Oxyde Dismutase, l'Ubiquinol ou certains chélatants de métaux ;
- des agents dépigmentants tels que l'hydroquinone, l'acide azélaïque, l'acide caféïque ou l'acide kojique ;
- des émollients ;
- des agents hydratants comme le glycérol, le PEG 400, la thiamorpholinone, et ses dérivés ou l'urée ;
- des agents antiséborrhéiques ou antiacnéiques, tels que la S-carboxyméthylcystéine, la S-benzyl-cystéamine, leurs sels ou leurs dérivés, ou le péroxyde de benzoyle ;
- des antibiotiques comme l'érythromycine et ses esters, la néomycine, la clindamycine et ses esters, les tétracyclines ;
- des agents antifongiques tels que le kétoconazole ou les polyméthylène-4,5
- isothiazolidones-3 ;
- des agents favorisant la repousse des cheveux, comme le Minoxidil (2,4-diamino-6-pipéridino-pyrimidine-3-oxyde) et ses dérivés, le Diazoxide (7-chloro 3-méthyl 1,2,4-benzothiadiazine 1,1-dioxyde) et le Phénytoïn (5,4-diphényl-imidazolidine 2,4-dione) ;
- des agents anti-inflammatoires non stéroïdiens ;
- des caroténoïdes et, notamment, le β-carotène ;
- des agents anti-psoriatiques tels que l'anthraline et ses dérivés;
- des acides eicosa-5,8,11,14-tétraynoïque et eicosa-5,8,11-triynoïque, leurs esters et amides ;
- des rétinoïdes, c'est à dire des ligands des récepteurs RAR ou RXR, naturels ou synthétiques ;
- des corticostéroïdes ou des oestrogènes ;
- des α-hydroxy acides et des α-céto acides ou leurs dérivés, tels que les acides lactique, malique, citrique, glycolique, mandélique, tartrique, glycérique, ascorbique, ainsi que leurs sels, amides ou esters, ou des β-hydroxy acides ou leurs dérivés, tels que l'acide salicylique ainsi que ses sels, amides ou esters ;
- des bloqueurs de canaux ioniques tels que les canaux potassiques ;
- ou encore, plus particulièrement pour les compositions pharmaceutiques, en association avec des médicaments connus pour interférer avec le système immunitaire (par exemple, la cyclosporine, le FK 506, les glucocorticoïdes, les anticorps monoclonaux, les cytokines ou les facteurs de croissance...).

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

On va maintenant donner, à titre d'illustration et sans aucun caractère limitatif, plusieurs exemples d'obtention de composés actifs de formule (I) selon l'invention, des résultats d'activité biologique ainsi que diverses formulations concrètes à base de tels composés.

### EXEMPLE 1 : 7-{[4'-(2,4-Dioxo-thiazolidin-5-ylmethyl)-biphenyl-3-ylmethyl]-methyl-carbamoyl}-heptanoate de méthyle.

### (a) (3-Bromo-benzyl)-carbamate de tert-butyle.

Dans un ballon et sous courant d'azote, on introduit 40,7 g (183 mmoles) de chlorhydrate de 3-bromobenzylamine, 26 ml de triéthyhmine (183 mmoles) et 450 ml de dichlorométhane. On ajoute par petites quantités à température ambiante 40 g (183 mmoles) de dicarbonate de di tert-butyle et agite pendant une nuit. On verse le milieu réactionnel dans l'eau glacée, extrait avec du dichlorométhane, décante la phase organique, sèche sur sulfate de magnésium, évapore. On recueille 46 g (88%) de produit attendu.

### (b) (3-Bromo-benzyl)-N-méthylcarbamate de tert-butyle.

Dans un ballon et sous courant d'azote, on introduit 128 g (447 mmoles) de (3-Bromo-benzyl)-carbamate de tert-butyle et 800 ml de DMF. On ajoute par petites quantités 19 g (475 mmoles) d'hydrure de sodium (60% dans l'huile) et agite jusqu'à cessation du dégagement gazeux. On ajoute ensuite 29,3 ml (470 mmoles) d'iodure de méthyle et agite pendant une nuit. On verse le milieu réactionnel dans l'eau glacée, extrait avec de l'acétate d'éthyle, décante la phase organique, sèche sur sulfate de magnésium, évapore. On recueille 152,5 g (92%) de produit attendu.

### (c) (4'-Formyl-biphenyl-3-ylmethyl)-methyl-carbamate de tert-butyle.

Dans un tricol et sous argon, on introduit 61,5 g (205 mmoles) de (3-Bromo-benzyl)-N-méthylcarbamate de tert-butyle, 40 g (260 mmoles) d'acide 4-formylbenzèneboronique et 800 ml de toluène. On ajoute goutte à goutte 205 ml d'une solution aqueuse de carbonate de potassium (2M), dégaze le milieu réactionnel avec de l'argon et ajoute 7 g de chlorure de tétrakistriphénylphosphinepalladium(0) et chauffe à 90°C pendant 24 heures. On verse le milieu réactionnel dans l'eau, extrait avec du dichlorométhane, décante la phase organique, sèche sur sulfate de magnésium, évapore. Le résidu obtenu est purifié par chromatographie sur colonne de silice élué avec un mélange heptane et acétate d'éthyle (70-30). Après évaporation des solvants, on recueille 38 g (57%) du produit attendu.

### (d) [4'-(2,4-Dioxo-thiazolidin-5-ylidenemethyl)-biphenyl-3-ylmethyl]-methyl-carbamate de tert-butyle.

Dans un ballon et sous courant d'azote, on introduit 75,4 g (232 mmoles) de (4'-Formylbiphenyl-3-ylmethyl)-methyl-carbamate de tert-butyle, 32,5 g (278 mmoles) de 2,4-thiazolidinedione, 7,3 g (50 mmoles) d'acétate de pipéridine et 1 l de toluène. On chauffe à reflux pendant cinq heures et sépare l'eau formée à l'aide d'un dean-stark. On refroidit le milieu réactionnel, filtre le précipité formé. On recueille 84 g (86%) du produit attendu.

### (e) [4-(2,4-Dioxo-thiazolidin-5-ylmethyl)-biphenyl-3-ylmethyl]-methyl-carbamate de tert-butyle.

Dans un tricol, on introduit 30 g (70,7 mmoles) de [4'-(2,4-Dioxo-thiazolidin-5-ylidenemethyl)-biphenyl-3-ylmethyl]-methyl-carbamate de *tert*-butyle dans 500 ml de dioxanne. On dégaze le milieu réactionnel, ajoute 30 g de palladium sur charbon (10%) et hydrogène sous pression de 3 bars à 60°C. On filtre le milieu réactionnel, évapore et purifie le résidu obtenu par chromatographie sur colonne de silice élué avec un mélange dichlorométhane et méthanol (99-1). On recueille après évaporation des solvants 18 g (60%) du produit attendu.

### (f) 5-(3'-Methylaminomethyl-biphenyl-4-ylmethyl)-thiazolidine-2,4-dione.

Dans un ballon et sous courant d'azote, on introduit 18 g (42 mmoles) de [4'-(2,4-Dioxothiazolidin-5-ylmethyl)-biphenyl-3-ylmethyl]-methyl-carbamate de tert-butyle dans 250 ml de dichlorométhane et ajoute 16 ml (208 mmoles) d'acide trifluoroacétique. On agite à température ambiante pendant une nuit et hydrolyse le milieu réactionnel avec une solution saturée de carbonate de potassium. On extrait avec du dichlorométhane, décante la phase organique, lave à l'eau, sèche sur sulfate de magnésium, évapore. Le résidu obtenu est trituré dans de l'acétate d'éthyle et on obtient 14,4 g (78%) du produit attendu.

### (g) 7-{[4'-(2,4-Dioxo-thiazolidin-5-ylmefhyl)-biphenyl-3-ylmethyl]-methyl-carbamoyl}-heptanoate de méthyle.

Dans un ballon et sous courant d'azote, on introduit 600 mg (1,36 mmoles) de 5-(3'-Methylaminomethyl-biphenyl-4-ylmethyl)-thiazolidine-2,4-dione, 10 ml de THF et 600 µl (4,3 mmoles) de triéthylamine. On ajoute goutte à goutte 220 µl (1,55 mmoles) de 8-chloro-8-oxooctanoate de méthyle et agite pendant une heure. On verse le milieu réactionnel dans l'eau, extrait avec du dichlorométhane, décante la phase organique, lave à l'eau, sèche sur sulfate de magnésium, évapore. Le résidu obtenu est purifié par chromatographie sur colonne de silice élué avec un mélange dichlorométhane et acétate d'éthyle (80-20). Après évaporation des solvants, on recueille 350 mg (50%) 7-{[4'-(2,4-Dioxo-thiazolidin-5-ylmethyl)-biphenyl-3-ylmethyl]-methyl-carbamoyl}-heptanoate de méthyle, sous la forme d'une huile.
RMN ¹H (CDCl₃) : 0,88-1,40 (m, 4H) ; 1,62-1,70 (m, 4H) ; 2,31 (t, J = 7,4 Hz, 2H) ; 2,40 (t, J = 7,3 Hz, 2H) ; 2,96-2,99 (m, 3H) ; 3,15 (m, 1H) ; 3,58 (m, 1 H) ; 3,66 (s, 3H) ; 4,57 (m,1H) ; 4,60-4,66 (m, 2H) ; 7,23-7,55 (m, 8H) ; 9,15 (m, 1H).

### EXEMPLE 2: 9-{[4'-(2,4-Dioxo-thiazolidin-5-ylrnethyl)-biphenyl-3-ylmethyl]-methyl-carbamoyl}-nonanoate de méthyle.

De manière analogue à l'exemple 1(g), par réaction de 600 mg (1,36 mmoles) de 5-(3'-Methylaminomethyl-biphenyl-4-ylmethyl)-thiazolidine-2,4-dione obtenu en 1(f) avec 340 µl (1,52 mmoles) de 10-chloro-10-oxodecanoate de méthyle, on obtient après purification par chromatographie sur colonne de silice élué par un mélange dichlorométhane et acétate d'éthyle (80/20), 500 mg (70 %) de 9-{[4'-(2,4-Dioxo-thiazolidin-5-ylmethyl)-biphenyl-3-ylmethyl]-methyl-carbamoyl}-nonanoate de méthyle sous la forme d'une huile.
RMN ¹H (CDCl₃) : 1,26-1,32 (m, 8H) ; 1,59-1,65 (m, 4H) ; 2,20-2,43 (m, 4H) ; 2,95-2,99 (m, 3H) ; 3,16 (m,1H) ; 3,55 (m, 1 H) ; 3,85 (s, 3H) ; 4,54 (m, 1 H) ; 4,60-4,65 (m, 2H) ; 7,19-7,54 (m, 8H) ; 9,75 (m, 1H).

### EXEMPLE 3: N-[4'-(2,4-Dioxo-thiazolidin-5-ylmethyl)-biphenyl-3-ylmethyl]-N-methyl-terephthalamate de méthyle.

De manière analogue à l'exemple 1(g), par réaction de 600 mg (1,36 mmoles) de 5-(3'-Methylaminomethyl-biphenyl-4-ylmethyl)-thiazolidine-2,4-dione obtenu en 1(f) avec 310 mg (1,54 mmoles) de 4-chlorocarbonyl-benzoate de méthyle, on obtient après purification par chromatographie sur colonne de silice élué par un mélange dichlorométhane et acétate d'éthyle (90/10), 370 mg (60 %) de N-[4'-(2,4-Dioxo-thiazolidin-5-ylmethyl)-biphenyl-3-ylmethyl]-N-methyl-terephthalamate de méthyle, sous la forme d'un solide blanc de point de fusion 186°C.

### EXEMPLE 4: 3-Cyclopentyl-N-[4'-(2,4-dioxo-thiazolidin-5-ylmethyl)-biphenyl-3-ylmethyl]-N-methyl-propionamide.

De manière analogue à l'exemple 1(g), par réaction de 500 mg (1,13 mmoles) de 5-(3'-Methylaminomethyl-biphenyl-4-ylmethyl)-thiazolidine-2,4-dione obtenu en 1(f) avec 200 mg (1,30 mmoles) de chlorure de 3-cyclopentyl-propionyle, on obtient après purification par chromatographie sur colonne de silice élué par un mélange dichlorométhane et méthanol (99/1), 170 mg (25%) de 3-Cyclopentyl-N-[4'-(2,4-dioxo-thiazolidin-5-ylmethyl)-biphenyl-3-ylmethyl]-N-methyl-propionamide, sous la forme d'un solide.
RMN ¹H (CDCl₃) : 1,10-1,14 (m, 2H) ; 1,26 (t, J = 7,1 Hz, 3H) ; 1,50-1,79 (m, 6H) ; 2,42 (t, J = 7,6 Hz, 2H) ; 2,97 (m, 3H) ; 3,15 (m, 1 H) ; 3,58 (m, 1 H) ; 4,52 (m, 1 H) ; 4,61-4,66 (m, 2H) ; 7,20-7,55 (m, 8H) ; 9,48 (s, 1 H).

### EXEMPLE 5: N-[4'-(2,4-dioxo-thiazolidin-5-ylmethyl)-biphenyl-3-ylmethyl]-N-methyl-naphthalene-1-carboxamide.

De manière analogue à l'exemple 1 (g), par réaction de 1 g (2,3 mmoles) de 5-(3'-Methylaminomethyl-biphenyl-4-ylmethyl)-thiazolidine-2,4-dione obtenu en 1(f) avec 380 µl (2,5 mmoles) de chlorure de 1-naphthoyle, on obtient après purification par chromatographie sur colonne de silice élué par un mélange heptane et acétate d'éthyle (80/20), 460 mg (41%) de N-[4'-(2,4-dioxo-thiazolidin-5-ylmethyl)-biphenyl-3-ylmethyl]-N-methyl-naphthalene-1-carboxamide, sous la forme d'un solide blanc de point de fusion 120°C.

### EXEMPLE 6: N-[4'-(2,4-dioxo-thiazolidin-5-ylmethyl)-biphenyl-3-ylmethyl]-N-methyl-naphthalene-2-carboxamide.

De manière analogue à l'exemple 1(g), par réaction de 1 g (2,3 mmoles) de 5-(3'-Methylaminomethyl-biphenyl-4-ylmethyl)-thiazolidine-2,4-dione obtenu en 1(f) avec 480 mg (2,5 mmoles) de chlorure de 2-naphthoyle, on obtient après purification par chromatographie sur colonne de silice élué par un mélange heptane et acétate d'éthyle (70/30), 400 mg (40%) de N-[4'-(2,4-dioxo-thiazolidin-5-ylmethyl)-biphenyl-3-ylmethyl]-N-methyl-naphthalene-2-carboxamide sous la forme d'un solide de point de fusion 218°C.

### EXEMPLE 7: N-[4'-(2,4-Dioxo-thiazolidin-5-ylmethyl)-biphenyl-3-ylmethyl]-N-methyt-2-phenoxy-acetamide.

De manière analogue à l'exemple 1(g), par réaction de 500 mg (1,53 mmoles) de 5-(3'-Methylaminomethyl-biphenyl-4-ylmethyl)-thiazolidine-2,4-dione obtenu en 1 (f) avec 210 µl (1,52 mmoles) de chlorure de phenoxyacétyle, on obtient après purification par chromatographie sur colonne de silice élué par un mélange heptane et acétate d'éthyle (60/40), 640 mg (91%) de N-[4'-(2,4-Dioxo-thiazolidin-5-ylmethyl)-biphenyl-3-ylmethyl]-N-methyl-2-phenoxy-acetamide, sous la forme d'un solide blanc de point de fusion 140°C.

### EXEMPLE 8: N-[4'-(2,4-dioxo-thiazolidin-5-ylmethyl)-biphenyl-3-ylmethyl]-N-methyl-1-Methyl-1H-pyrrole-2-carboxamide.

*(a) Chlorure de l'acide 1-méthyl-1H-pyrrole-2-carboxylique.* Dans un ballon et sous courant d'azote, on introduit 500 mg (4 mmoles) de l'acide 1-methyl-2-pyrrolecarboxylique dans 5 ml de dichlorométhane. On additionne, goutte à goutte, 790 µl (4 mmoles) de dicyclohexylamine et 30 minutes plus tard, 290µl (4 mmoles) de chlorure de thionyle. Le milieu est agité 1 heure à température ambiante puis chauffé 2 heures à 50°C. On dilue alors avec de l'éther et le précipité est filtré. Le filtrat est évaporé et on obtient une huile brune.
*(b) N-[4'-(2,4-dioxo-thiazolidin-5-ylmethyl)-biphenyl-3-ylmethyl]-N-methyl-1-methyl-1H-pyrrole-2-carboxamide.* De manière analogue à l'exemple 1(g), par réaction de 500 mg (1,53 mmole) de 5-(3'-Methylaminomethyl-biphenyl-4-ylmethyl)-thiazolidine-2,4-dione obtenu en 1(f) avec 220 mg (1,53 mmole) de chlorure de l'acide 1-Methyl-1H-pyrrole-2-carboxylique, on obtient, après purification par chromatographie sur colonne de silice élué par un mélange heptane et acétate d'éthyle (60/40), 316 mg (47%) de N-[4'-(2,4-dioxothiazolidin-5-ylmethyl)-biphenyl-3-ylmethyl]-N-methyl-1-methyl-1 H-pyrrole-2-carboxamide sous la forme d'un solide blanc de point de fusion 184°C.

### EXEMPLE 9: N-[4'-(2,4-dioxo-thiazolidin-5-ylmethyl)-biphenyl-3-ylmethyl]-N-methyl-adamantane-1-carboxamide.

De manière analogue à l'exemple 1(g), par réaction de 500 mg (1,53 mmole) de 5-(3'-Methylaminomethyl-biphenyl-4-ylmethyl)-thiazolidine-2,4-dione obtenu en 1 (f) avec 310 mg (1,56 mmole) de chlorure de l'acide adamantane-1-carboxylique, on obtient après purification par chromatographie sur colonne de silice élué avec un mélange heptane et acétate d'éthyle (70/30), 390 mg de N-[4'-(2,4-dioxo-thiazolidin-5-ylmethyl)-biphenyl-3-ylmethyl]-N-methyl-adamantane-1-carboxamide sous la forme d'une poudre blanche de point de fusion 77°C.

### EXEMPLE 10 : N-[4'-(2,4-dioxo-thiazolidin-5-ylmethyl)-biphenyl-3-ylmethyl]-N-methyl-biphényl-4-carboxamide.

De manière analogue à l'exemple 1(g), par réaction de 500 mg (1,53 mmole) de 5-(3'-Methylaminomethyl-biphenyl-4-ylmethyl)-thiazolidine-2,4-dione obtenu en 1(f) avec 330 mg (1,52 mmole) de chlorure de l'acide 4-biphenylcarboxylique, on obtient après trituration dans l'éther 681 mg (88%) de N-[4'-(2,4-dioxo-thiazolidin-5-ylmethyl)-biphenyl-3-ylmethyl]-N-methyl-biphenyl-4-carboxamide sous la forme d'une poudre blanche de point de fusion de 204°C.

### EXEMPLE 11 : N-[4'-(2,4-dioxo-thiazolidin-5-ylmethyl)-biphenyl-3-ylmethyl]-N-methyl-benzo[b]thiophene-2-carboxamide.

De manière analogue à l'exemple 1(g), par réaction de 500 mg (1,53 mmole) de 5-(3'-Methylaminomethyl-biphenyl-4-ylmethyl)-thiazolidine-2,4-dione obtenu en 1(f) avec 300 mg (1,52 mmole) de chlorure de l'acide benzo[b]thiophene-2-carboxylique, on obtient après trituration dans le dichlorométhane 509 mg (68%) de N-[4'-(2,4-dioxo-thiazolidin-5-ylmethyl)-biphenyl-3-ylmethyl]-N-methyl-benzo[b]thiophene-2-carboxamide sous la forme d'une poudre blanche de point de fusion 187°C.

### EXEMPLE 12 : N-[4'-(2,4-dioxo-thiazolidin-5-ylmethyl)-biphenyl-3-ylmethyl]-N-methyl-6-oxo-6-phenyl-hexanamide.

Dans un ballon et sous courant d'azote, on introduit 320 mg (1,55 mmole) de l'acide 5-benzoylpentanoïque dans 5 ml de dichlorométhane. On ajoute 230 mg (1,7 mmole) de l'hydrate-1-hydroxybenzotriazole, 230µl (1,7 mmole) de triéthylamine et 500 mg (1,53 mmole) de 5-(3'-Methylaminomethyl-biphenyl-4-ylmethyl)-thiazolidine-2,4-dione obtenu en 1(f). Puis, à 0°C, 320 mg (1,7 mmole) du chlorhydrate-1-(3-diméthylaminopropyl)-3-éthylcarbodiimide sont ajoutés. Le milieu est agité 3 heures à température ambiante. II est dilué avec du dichlorométhane et lavé avec une solution aqueuse saturée en hydrogencarbonate de sodium. La phase organique est séchée sur sulfate de magnésium, filtrée et évaporée. Le résidu est purifié par chromatographie sur colonne de silice élué avec un mélange heptane et acétate d'éthyle (50/50). On obtient 590 mg (75%) de N-[4'-(2,4-dioxo-thiazolidin-5-ylmethyl)-biphenyl-3-ylmethyl]-N-methyl-6-oxo-6-phenyl-hexanamide sous la forme d'une poudre blanche de point de fusion 48°C.

### EXEMPLE 13 : 4-Dimethylamino-N-[4'-(2,4-dioxo-thiazolidin-5-ylmethyl)-biphenyl-3-ylmethyl]-N-methyl-naphthalene-1-carboxamide.

De manière analogue à l'exemple 12, par réaction de 500 mg (1,53 mmole) de 5-(3'-Methylaminomethyl-biphenyl-4-ylmethyl)-thiazolidine-2,4-dione obtenu en 1(f) avec 330 mg (1,53 mmole) de l'acide 4-diméthylaminonaphtalène-1-carboxylique, on obtient, après purification par chromatographie sur colonne de silice élué avec un mélange heptane et acétate d'éthyle (50/50), 520 mg (65%) de 4-Dimethylamino-N-[4'-(2,4-dioxo-thiazolidin-5-ylmethyl)-biphenyl-3-ylmethyl]-N-methyl-naphthalene-1-carboxamide sous la forme d'une mousse jaunâtre de point de fusion 67°C.

### EXEMPLE 14 : N-[4'-(2,4-Dioxo-thiazolidin-5-ylmethyl)-biphenyl-3-ylmethyl]-4-methanesulfonyl-N-methyl-benzamide.

De manière analogue à l'exemple 12, par réaction de 500 mg (1,53 mmole) de 5-(3'-Methylaminomethyl-biphenyl-4-ylmethyl)-thiazolidine-2,4-dione obtenu en 1(f) avec 310 mg (1,55 mmole) de l'acide 4-(méthylsulfonyl)-benzoïque, on obtient après purification par chromatographie sur colonne de silice élué avec un mélange dichlorométhane et méthanol (98/2), 540 mg (69%) de N-[4'-(2,4-Dioxo-thiazolidin-5-ylmethyl)-biphenyl-3-ylmethyl]-4-methanesulfonyl-N-methyl-benzamide sous la forme d'un solide blanc de point de fusion 172°C.

### EXEMPLE 15 : N-[4'-(2,4-Dioxo-thiazolidin-5-ylmethyl)-biphenyl-3-ylmethyl]-N-methyl-4-(1-phenyl-methanoyl)-benzamide.

### (a) Chlorure de 4-(1-Phenyl-methanoyl)-benzoyle.

De manière analogue à l'exemple 9(a), à partir de 500 mg (2,2 mmoles) de l'acide 4-benzoylbenzoïque, on obtient 490 mg (91 %) du produit attendu sous la forme d'un solide blanc.

### (b) N-[4'-(2,4-Dioxo-thiazolidin-5-ylmethyl)-biphenyl-3 ylmethyl]-N-methyl-4-(1-phenyl-methanoyl)-benzamide.

De manière analogue à l'exemple 1(g), par réaction de 500 mg (1,53 mmole) de 5-(3'-Methylaminomethyl-biphenyl-4-ylmethyl)-thiazolidine-2,4-dione obtenu en 1(f) avec 380 mg (1,55 mmole) de Chlorure de 4-(1-Phenyl-methanoyl)-benzoyle, on obtient, après purification par chromatographie sur colonne de silice élué par un mélange heptane et acétate d'éthyle (50/50), 660 mg (81%) de N-[4'-(2,4-Dioxo-thiazolidin-5-ylmethyl)-biphenyl-3-ylmethyl]-N-methyl-4-(1-phenyl-methanoyl)-benzamide sous la forme d'une poudre blanche de point de fusion 94°C.

### EXEMPLE 16 : 6-(2-Methoxy-ethoxymethoxy)-N-[4'-(2,4-dioxo-thiazolidin-5-ylmethyl)-biphenyl-3-ylmethyl]-N-methyl-naphthalene-2-carboxamide.

### (a) 6-Hydroxy-naphthalene-2-carboxylate de méthyle.

Dans un ballon, on introduit 15,7 g (83 mmoles) d'acide 6-hydroxy-2-naphthoïque dans 160 ml de méthanol. On ajoute 8 ml d'acide sulfurique concentré et on chauffe au reflux pendant 8 heures. A température ambiante, un précipité se forme. Il est filtré, rincé avec de l'éther et séché. On obtient 14,1 g (84%) du produit attendu sous la forme d'une poudre beige.

### (b) 6-(2-Methoxy-ethoxymethoxy)-naphthalene-2-carboxylate de méthyle.

Dans un ballon et sous courant d'azote, on introduit 14 g (69 mmoles) de 6-Hydroxynaphthalene-2-carboxylate de méthyle dans 90 ml de diméthylformamide et 90 ml de tétrahydrofuranne. On additionne, par petites portions, 3,3 g (82 mmoles) d'hydrure de sodium à 60%. Quand le dégagement gazeux est terminé, on rajoute 8,7 ml (76 mmoles) de chlorure 2-méthoxyéthoxyméthyle et le milieu réactionnel est agité 3 heures à température ambiante. Il est, ensuite, versé dans de l'eau glacée et extrait à l'éther. La phase organique est séchée sur sulfate de magnésium, filtrée et évaporée. Le résidu obtenu est purifié par chromatographie sur colonne de silice élué avec un mélange heptane et acétate d'éthyle (80/20) et on obtient 17 g (85%) du produit attendu sous la forme d'une huile incolore.

### (c) Acide 6-(2-Methoxy-ethoxymethoxy)-naphthalene-2-carboxylique.

Dans un ballon, on introduit 16,9 g (58 mmoles) de 6-(2-Methoxy-ethoxymethoxy)-naphthalene-2-carboxylate de méthyle dans 200 ml de tétrahydrofuranne et 20 ml de méthanol. On ajoute 1 ml d'eau et 12,9 g (322 mmoles) de soude en pastilles. Le milieu réactionnel est agité 4 heures à température ambiante. A froid, on ajoute lentement de l'acide chlorhydrique 1N jusqu'à pH 2-3. On extrait à l'acétate d'éthyle. La phase organique est séchée sur sulfate de magnésium, filtrée puis évaporée. Le résidu est trituré dans de l'heptane, filtré et séché. On obtient 14,9 g (92%) du produit attendu sous la forme d'une poudre blanche de point de fusion 110°C.

### (d) 6-(2-Methoxy-ethoxymethoxy)-N-[4'-(2,4-dioxo-thiazolidin-5-ylmethyl)-biphenyl-3-yimethyl]-N-methyl-naphthaiene-2-carboxamide.

De manière analogue à l'exemple 12, par réaction de 1,5 g (4,6 mmoles) de de 5-(3'-Methylaminomethyl-biphenyl-4-ylmethyl)-thiazolidine-2,4-dione obtenu en 1(f) avec 1,27 g (4,6 mmoles) d'acide 6-(2-Methoxy-ethoxymethoxy)-naphthalene-2-carboxylique, on obtient, après purification par chromatographie sur colonne de silice élué avec un mélange heptane et acétate d'éthyle (50/50), 1,97 g (62%) de 6-(2-Methoxy-ethoxymethoxy)-N-[4'-(2,4-dioxo-thiazolidin-5-ylmethyl)-biphenyl-3-ylmethyl]-N-methyl-naphthalene-2-carboxamide sous la forme d'une poudre blanche de point de fusion 68°C.

### EXEMPLE 17: 6-Hydroxy-N-[4'-(2,4-dioxo-thiazolidin-5-ylmethyl)-biphenyl-3-ylmethyl]-N-methyl- naphthalene-2-carboxamide.

Dans un ballon, on introduit 1,5 g (2,5 mmoles) de 6-(2-Methoxy-ethoxymethoxy)-N-[4'-(2,4-dioxo-thiazolidin-5-ylmethyl)-biphenyl-3-ylmethyl]-N-methyl-naphthalene-2-carboxamide (obtenu à l'exemple 16) dans 10 ml de tétrahydrofuranne et 10 ml de méthanol. On ajoute 500 µl d'acide sulfurique concentré et le milieu est agité 2 heures à température ambiante. On ajoute de l'eau et on extrait à l'acétate d'éthyle. La phase organique est séchée sur sulfate de magnésium, filtrée puis évaporée. Le résidu est purifié par chromatographie sur colonne de silice élué avec un mélange heptane et acétate d'éthyle (30/70). On obtient 1,26 g (99%) de 6-Hydroxy-N-[4'-(2,4-dioxo-thiazolidin-5-ylmethyl)-biphenyl-3-ylmethyl]-N-methyl-naphthalene-2-carboxamide sous la forme d'une poudre blanche de point de fusion 218°C.

### EXEMPLE 18: N-[4'-(2,4-Dioxo-thiazolidin-5-ylmethyl)-biphenyl-3-ylmethyl]-N-methyl-4-methylsulfanyl-benzamide.

### (a) chlorure de 4-methylsulfanyl-benzoyle.

De manière analogue à l'exemple 9(a), à partir de 400 mg (2,4 mmoles) de l'acide 4-(méthylthio)-benzoïque, on obtient 440 mg (99%) du produit attendu sous la forme d'un solide jaunâtre.

### (b) N-[4'-(2,4-Dioxo-thiazolidin-5-ylmethyl)-biphenyl-3-ylmethyl]-N-methyl-4-methylsulfanyl-benzamide.

De manière analogue à l'exemple 1(g), par réaction de 500 mg (1,53 mmole) de 5-(3'-Methylaminomethyl-biphenyl-4-ylmethyl)-thiazolidine-2,4-dione obtenu en 1 (f) avec 290 mg (1,55 mmole) de chlorure de 4-methylsulfanyl-benzoyle, on obtient, après recristallisation dans du méthanol, 470 mg (64%) de N-[4'-(2,4-Dioxo-thiazolidin-5-ylmethyl)-biphenyl-3-ylmethyl]-N-methyl-4-methylsulfanyl-benzamide sous la forme d'une poudre blanche de point de fusion 203-7°C.

### EXEMPLE 19 : Acide (S)-3-(3'-{[(1-Biphenyl-4-yl-methanoyl)-methyl-amino]-methyl}-biphenyl-4-yl)-2-ethoxy-propionique.

### (a) chlorure d'éthoxyacétyle.

Dans un ballon et sous courant d'azote, on introduit 25 g (240 mmoles) d'acide éthoxyacétique dans 300 ml de dichlorométhane. On ajoute 47,6 ml (239 mmoles) de dicyclohexylamine. Le milieu est agité 1 heure à température ambiante. On ajoute, alors, 19,2 ml (265 mmoles) de chlorure de thionyle et on agite pendant 3 heures. On rajoute au milieu réactionnel de l'éther éthylique, le précipité formé est filtré et rincé à l'éther. Après évaporation du filtrat, on obtient 29 g (100%) du produit attendu sous la forme d'un liquide marron.

### (b) 3-(2-ethoxy-ethanoyl)-4-benzyloxazolidin-2-one.

Dans un ballon et sous courant d'azote, on introduit 36,7 g (207 mmoles) de (S)-4-benzyloxazolidin-2-one dans 800 ml de THF. Le milieu réactionnel est refroidi à - 78°C et on ajoute, goutte à goutte, 83 ml (207 mmoles) de n-Butyllithium (2,5 M/hexane). 30 minutes après, on ajoute, à -78°C, 25,4 g (207 mmoles) de chlorure d'ethoxyacetyle. Le milieu réactionnel est agité 24 heures puis versé dans une solution aqueuse saturée en chlorure de sodium et extrait à l'acétate d'éthyle. La phase organique est séchée sur sulfate de magnésium, filtrée et évaporée. On obtient, après purification par chromatographie sur colonne de silice élué avec un mélange heptane et acétate d'éthyle (60/40), 30,6 g (56%) du produit attendu sous la forme d'une huile orangée.

### (c) (2S,3R)-3-{3'-[(tert-Butoxycarbonyl-methyl-amino)-methyl]-biphenyl-4-yl}-2-ethoxy-3-hydroxy-propionate de methyle.

Dans un ballon et sous argon, on introduit 21,9 g (83 mmoles) de 3-(2-ethoxy-ethanoyl)-4-benzyloxazolidin-2-one et 100 ml de dichlorométhane. On ajoute, à 0°C, goutte à goutte successivement 103 ml (103 mmoles) de trifluorométhanesulfonate de dibutylborane et 18 ml (104 mmoles) de N-éthyldiisopropylamine et agite pendant une heure. A -78°C, on ajoute une solution de 23,5 g (69 mmoles) de (4'-formyl-biphenyl-3-ylmethyl)-methyl-carbamate de *tert*-butyle obtenu en 1(c) dans 100 ml de dichlorométhane et agite pendant une nuit. On traite avec une solution tampon pH=7 (170 ml) dans 500 ml de méthanol puis avec une solution d'eau oxygénée (170 ml) dans 500 ml de méthanol et on agite 1 heure 30 à 0°C. On verse le milieu réactionnel dans l'eau, extrait avec du dichlorométhane. La phase organique est séchée sur sulfate de magnésium, filtrée et évaporée. Le résidu obtenu est purifié par chromatographie sur colonne de silice élué avec un mélange heptane et acétate d'éthyle (70/30) et on obtient 21 g (51%) du produit attendu.

### (d) (2S,3R)-2-ethoxy-3-hydroxy-3-(3'-methylaminomethyl-biphenyl-4-yl)-propionate de methyle.

Dans un ballon et sous courant d'azote, on introduit 21 g (47,3 mmoles) de (2S,3R)-3-{3'-[(tert-Butoxycarbonyl-methyl-amino)-methyl]-biphenyl-4-yl}-2-ethoxy-3-hydroxy-propionate de methyle, 8,76 ml (54,9 mmoles) de triéthylsilane dans 300 ml d'acide trifluoroacétique. Le milieu réactionnel est agité pendant 4 heures à température ambiante. On ajoute, ensuite, de l'acétate d'éthyle et on neutralise avec de la soude. La phase organique est lavée avec une solution aqueuse saturée en chlorure de sodium, séchée sur sulfate de magnésium, filtrée et évaporée. On obtient 19,6 g (100%) du produit brut attendu.

### (e) (S)-2-ethoxy-3-(3'-methylaminomethyl-biphenyl-4-yl)-propionate de methyle.

19,6 g du produit (2S,3R)-2-ethoxy-3-hydroxy-3-(3'-methylaminomethyl-biphenyl-4-yl)-propionate de methyle brut sont dissous dans 200 ml d'acide trifluoroacétique et on ajoute 41,7 ml (297 mmoles) de triéthylamine. Le milieu réactionnel est agité à température ambiante pendant 48 heures puis extrait à l'acétate d'éthyle. La phase organique est décantée, lavée avec une solution de soude puis avec une solution aqueuse saturée en chlorure de sodium, séchée sur sulfate de magnésium, filtrée et évaporée. Le résidu obtenu est purifié par chromatographie sur colonne de silice élué avec un mélange dichlorométhane et méthanol (95/5). On obtient 1,6 g (10%) du produit attendu.

### (f) (S)-3-(3'-{[(1-Biphenyl-4-yl-methanoyl)-methyl-amino]-methyl}-biphenyl-4-yl)-2-ethoxy-propionate de méthyle.

De manière analogue à l'exemple 1(g), par réaction de 500 mg (1,5 mmole) de (S)-2-ethoxy-3-(3'-methylaminomethyl-biphenyl-4-yl)-propioriate de methyle avec 680 mg (3,1 mmoles) de chlorure de l'acide 4-biphenylcarboxylique, on obtient, après purification par chromatographie sur colonne de silice élué avec un mélange heptane et acétate d'éthyle (70/30), 360 mg (47%) du produit attendu.

### (g) Acide (S)-3-(3'-{[(1-Biphenyl-4-yl-methanoyl)-methyl-amino]-methyl}-biphenyl-4 yl)-2-ethoxy-propionique.

Dans un ballon, on introduit 360 mg (0,7 mmole) de (S)-3-(3'-{[(1-Biphenyl-4-yl-methanoyl)-methyl-amino]-methyl}-biphenyl-4-yl)-2-ethoxy-propionate de méthyle dans 10 ml de THF. On ajoute 60 mg (1,4 mmole) d'hydroxyde de lithium monohydraté, 1 ml d'eau et 1 ml de méthanol et agite 4 heures. On verse le milieu réactionnel dans l'eau, acidifie à pH 1, extrait avec de l'acétate d'éthyle, décante la phase organique, sèche sur sulfate de magnésium, évapore. Le résidu obtenu est purifié par chromatographie sur colonne de silice élué avec un mélange heptane et acétate d'éthyle (50/50) et on obtient 280 mg (80%) de l'acide (S)-3-(3'-{[(1-Biphenyl-4-yl-methanoyl)-methyl-amino]-methyl}-biphenyl-4-yl)-2-ethoxy-propionique sous la forme d'un solide amorphe blanc.
RMN ¹H (CDCl₃) : 1,19 (t, J = 8 Hz, 3H) ; 2,96-3,09 (m, 3H) ; 3,05 (dd, J = 14,1 Hz et J = 7,8 Hz, 1H) ; 3,17 (dd, J = 14,1 Hz et J = 4 Hz, 1H) ; 3,45-3,61 (m, 2H) ; 4,12 (m, 1H) ; 4,64-4,84 (m, 2H) ; 7,25-7,56 (m, 17H).

### EXEMPLE 20 : Acide (S)-2-Ethoxy-3-(3'-{[methyl-(6-oxo-6-phenyl-hexanoyl)-amino]-methyl}-biphenyl-4-yl)-propionique.

### (a) (S)-2-Ethoxy-3-(3'-{[methyl-(6-oxo-6-phenyl-hexanoyl)-amino]-methyl}-biphenyl-4-yl)-propionate de méthyle.

De manière analogue à l'exemple 12, par réaction de 660 mg (2 mmoles) de (S)-2-ethoxy-3-(3'-methylaminomethyl-biphenyl-4-yl)-propionate de methyle obtenu en 19(e) avec 346 mg (1,68 mmole) de l'acide 5-benzoylpentanoïque, on obtient, après purification par chromatographie sur colonne de silice élué avec un mélange heptane et acétate d'éthyle (50/50), 330 mg (33%) du produit attendu.

### (b) Acide (S)-2-Ethoxy-3-(3'-{[methyl-(6-oxo-6-phenyl-hexanoyl)-amino]-methyl}-biphenyl-4-yl)-propionique.

De manière analogue à l'exemple 19(g), à partir de 330 mg (0,64 mmole) de (S)-2-Ethoxy-3-(3'-{[methyl-(6-oxo-6-phenyl-hexanoyl)-amino]-methyl}-biphenyl-4-yl)-propionate de méthyle, on obtient, après purification par chromatographie sur colonne de silice 230 mg (72%) de l'acide (S)-2-Ethoxy-3-(3'-{[methyl-(6-oxo-6-phenyl-hexanoyl)-amino]-methyl}-biphenyl-4-yl)-propionique sous la forme d'une huile jaune.
RMN ¹H (CDCl₃) : 1,20 (m, 3H) ; 1,77-1,85 (m, 4H) ; 2,44 (m, 2H) ; 2,95-2,98 (m, 3H) ; 3,01 (m, 2H) ; 3,05 (m, 1 H) ; 3,15 (m, 1 H) ; 3,46-3,61 (m, 2H) ; 4,10 (2s, 1H); 4,59-4,64 (m, 2H) ; 7,18-7,55 (m, 11H) ; 7,90-7,96 (m, 2H).

### EXEMPLE 21: 1-[4'-(2,4-Dioxo-thiazolidin-5-ylmethyl)-biphenyl-3-yl]-1-methyl-3-naphthalen-2-yl-urée.

### (a) (3-Bromo-phenyl)-methyl-amine.

Dans un ballon et sous courant d'azote, on introduit 10 g (58 mmoles) de 3-bromoaniline et 34 ml (204 mmoles) de triéthylorthoformate. Le milieu réactionnel est porté au reflux pendant 7 heures. Le triéthylorthoformate est, alors, évaporé. Le résidu est dissous dans de l'éthanol et on ajoute, à 0°C, 4,9 g (12,8 mmoles) de borohydrure de sodium. Le milieu est agité une nuit à température ambiante. Il est, alors, versé dans de l'eau et extrait à l'acétate d'éthyle. La phase organique est lavée avec une solution aqueuse saturée en chlorure de sodium, séchée sur sulfate de magnésium, filtrée puis évaporée. Le résidu obtenu est purifié par chromatographie sur colonne de silice élué avec un mélange heptane et acétate d'éthyle (90/10) et on obtient 5 g (46%) du produit attendu sous la forme d'une huile claire.

### (b) 3'-Methylamino-biphenyl-4-carbaldehyde.

De manière analogue à l'exemple 1(c), par réaction de 4,3 g (23,2 mmoles) de (3-Bromo-phenyl)-methyl-amine avec 5,2 g (34,8 mmoles) de l'acide 4-formylbenzèneboronique, on obtient, après purification par chromatographie sur colonne de silice élué avec un mélange heptane et acétate d'éthyle (90/10), 2,9 g (59%) du produit attendu sous la forme d'un solide jaune.

### (c) 5-(3'-Methylamino-biphenyl-4-ylmethylene)-thiazolidine-2,4-dione.

De manière analogue à l'exemple 1(d), par réaction de 2,9 g (13,7 mmoles) de 3'-Methylamino-biphenyl-4-carbaldehyde avec 1,6 g (13,7 mmoles) de 2,4-thiazolidinedione, on obtient, après trituration dans du dichlorométhane et de l'éther, 3,9 g (91%) du produit attendu.

### (d) 1-[4'-(2,4-Dioxo-thiazolidin-5-ylidenemethyl)-biphenyl-3yl]-1-methyl-3-naphthalen-2-yl-urée.

Dans un ballon et sous courant d'azote, on introduit 500 mg (1,6 mmole) de 5-(3'-Methylamino-biphenyl-4-ylmethylene)-thiazolidine-2,4-dione dans 10 ml de dichlorométhane et 540 mg (3,2 mmoles) d'isocyanate de naphthyle. On agite à 35°C pendant 4 heures. Le milieu réactionnel est filtré et le solide est rincé avec du dichlorométhane. Le filtrat est évaporé et on obtient 660 mg (86%) du produit attendu sous la forme d'une poudre jaune.

### (e) 1-[4'-(2,4-Dioxo-thiazolidin-5-ylmethyl)-biphenyl-3-yl]-1-methyl-3-naphthalen-2-yl-urée.

De manière analogue à l'exemple 1(e), à partir 660 mg (1,38 mmole) de 1-[4'-(2,4-Dioxothiazolidin-5-ylidenemethyl)-biphenyl-3-yl]-1-methyl-3-naphthalen-2-yl-urée, on obtient, après purification par chromatographie sur colonne de silice élué avec un mélange heptane et acétate d'éthyle (70/30), 320 mg (48%) de 1-[4'-(2,4-Dioxo-thiazolidin-5-ylmethyl)-biphenyl-3-yl]-1-methyl-3-naphthalen-2-yl-urée sous la forme d'une poudre blanche de point de fusion 196°C.

### EXEMPLE 22: 3-(4-Dimethylamino-phenyl)-1-[4'-(2,4-dioxo-thiazolidin-5-ylmethyl)-biphenyl-3-yl]-1-methyl-urée.

### (a) 3-(4-Dimethylamino-phenyl)-1-[4'-(2,4-dioxo-thiazolidin-5-ylidenemethyl)-biphenyl-3-yl]-1-methyl-urée.

De manière analogue à l'exemple 22(d), par réaction de 500 mg (1,6 mmole) de 5-(3'-Methylamino-biphenyl-4-ylmethylene)-thiazolidine-2,4-dione obtenu en 21(c) avec 520 mg (3,2 mmoles) d' isocyanate de 4-(diméthylamino)-phenyle, on obtient 760 mg (100%) du produit attendu sous la forme d'une poudre jaune.

### (b) 3-(4-Dimethylamino-phenyl)-1-[4 '-(2,4-dioxo-thiazolidin-5-ylmethyl)-biphenyl-3-yl]-1-methyl-urée.

De manière analogue à l'exemple 1(e), à partir de 760 mg (1,6 mmole) de 3-(4-Dimethylamino-phenyl)-1-[4'-(2,4-dioxo-thiazolidin-5-ylidenemethyl)-biphenyl-3-yl]-1-methyl-urée, on obtient, après purification par chromatographie sur colonne de silice élué avec un mélange heptane et acétate d'éthyle (60/40), 330 mg (43%) de 3-(4-Dimethylamino-phenyl)-1-[4'-(2,4-dioxo-thiazol idin-5-ylmethyl)-biphenyl-3-yl]-1-methyl-urée sous la forme d'une poudre blanche de point de fusion 102°C.

### EXEMPLE 23: Acide (S)-2-Ethoxy-3-{3'-[({1-[6-(2-methoxy-ethoxymethoxy)-naphthalen-2-yl]-methanoyl}-methyl-amino)-methyl]-biphenyl-4-yl}-propionique.

### (a) (S)-2-Ethoxy-3-{3'-[({1-[6-(2-methoxy-ethoxymethoxy)-naphthalen-2-yl]-methanoyl}-methyl-amino)-methyl]-biphenyl-4-yl}-propionique.

De manière analogue à l'exemple 13, par réaction de 380 mg (1,16 mmole) de (S)-2-ethoxy-3-(3'-methylaminomethyl-biphenyl-4-yl)-propionate de methyle obtenu en 19(e) avec 350 mg (1,27 mmole) d'acide 6-(2-Methoxy-ethoxymethoxy)-naphthalene-2-carboxylique (préparé en 16(c)), on obtient, après purification par chromatographie sur colonne de silice élué avec un mélange heptane et acétate d'éthyle (50/50), 110 mg (16%) du produit attendu.

### (b) Acide (S)-2-Ethoxy-3-{3'-[({1-[6-(2-methoxy-ethoxymethoxy)-naphthalen-2-yl]-methanoyl}-methyl-amino)-methyl]-biphenyl-4-yl}-propionique.

De manière analogue à l'exemple 19(g), par réaction de 110 mg (0,18 mmole) de (S)-2-Ethoxy-3-{3'-[({ 1-[6-(2-methoxy-ethoxymethoxy)-naphthalen-2-yl]-methanoyl}-methyl-amino)-methyl]-biphenyl-4-yl}-propionate de méthyle avec 16 mg (0,38 mmole) d'hydroxyde de lithium monohydraté, on obtient, après purification par chromatographie sur colonne de silice élué avec un mélange heptane et acétate d'éthyle (50/50) puis avec de l'acétate d'éthyle pur, 30 mg (42%) d'acide (S)-2-Ethoxy-3-{3'-[({1-[6-(2-methoxyethoxymethoxy)-naphthalen-2-yl]-methanoyl}-methyl-amino)-methyl]-biphenyl-4-yl}-propionique sous la forme d'une huile jaune.
RMN ¹H (CDCl₃) : 1,18 (t, J = 6,9 Hz, 3H) ; 2,90-3,17 (m, 5H) ; 3,37 (s, 3H) ; 3,44-3,64 (m, 4H) ; 3,86 (m, 2H) ; 4,11 (m, 1 H) ; 4,70 (m, 2H) ; 5,39 (s, 2H) ; 7,23-7,54 (m, 11 H) ; 7,75 (m, 2H) ; 7,91 (s, 1 H).

### EXEMPLE 24 : 6-(Methoxymethoxy)-N-[4'-(2;4-dioxo-thiazolidin-5-ylmethyl)-biphenyl-3-ylmethyl]-N-methyl-naphthalene-2-carboxamide.

De manière analogue à l'exemple 12, par réaction de 500 mg (1,53 mmole) de 5-(3'-Methylaminomethyl-biphenyl-4-ylmethyl)-thiazolidine-2,4-dione obtenu en 1(f) avec 371 mg (1,6 mmole) de l'acide 6-Methoxymethoxy-naphtalène-2-carboxylique, on obtient, après purification par chromatographie sur colonne de silice éluée avec un mélange heptane et acétate d'éthyle (50/50), 648 mg (75%) de 6-(Methoxymethoxy)-N-[4'-(2,4-dioxo-thiazolidin-5-ylmethyl)-biphenyl-3-ylmethyl]-N-methyl-naphthalene-2-carboxamide sous forme d'une poudre blanche de point de fusion 160°C.
RMN ¹H (DMSO d6 ; 400MHz) : 2,96 (s large, 3H) ; 3,19 (dd, J=9,2 Hz et J=14,1 Hz, 1H) ; 3,41 (s, 3H) ; 3,44 (dd, J=4,2 Hz et J=14,1Hz, 1H) ; 4,58-4,82 (m, 2H) ; 4,97 (dd, J=4,3 Hz zt J=9,1 Hz, 1 H) ; 7,25-8,03 (m, 14H) ; 12,10 (s large, 1 H)

### EXEMPLE 25 : 6-(Methoxycarbonyl)-N-[4'-(2,4-dioxo-thiazolidin-5-ylmethyl)-biphenyl-3-ylmethyl]-N-methyl-naphthalene-2-carboxamide.

De manière analogue à l'exemple 12, par réaction de 500 mg (1,53 mmole) de 5-(3'-Methylaminomethyl-biphenyl-4-ylmethyl)-thiazolidine-2,4-dione obtenu en 1 (f) avec 370 mg (1,6 mmole) de l'acide 6-Methoxycarbonyl-naphtalène-2-carboxylique, on obtient, après purification par chromatographie sur colonne de silice éluée avec un mélange heptane et acétate d'éthyle (50/50), 580 mg (67%) de 6-(Methoxycarbonyl)-N-[4'-(2,4-dioxo-thiazolidin-5-ylmethyl)-biphenyl-3-ylmethyl]-N-methyl-naphthalene-2-carboxamide sous forme d'une poudre blanche de point de fusion 125-127°C.
RMN ¹H (DMSO d6 ; 400MHz) : 2,92-3,01 (m, 3H) ; 3,18 (m, 1H) ; 3,44 (m, 1H); 3,93 (s, 3H); 4,59-4,80 (m, 2H) ; 4,97 (m, 1 H) ; 7,20-7,70 (m, 9H) ; 8,00-8,20 (m, 3H); 8,23 (m, 1H) ; 8,69 (m, 1H) ; 12,10 (s large, 1 H)

### EXEMPLE 26: 6-(Propyloxy)-N-[4'-(2,4-dioxo-thiazolidin-5-ylmethyl)-biphenyl-3-ylmethyl]-N-methyl-naphthalene-2-carboxamide.

De manière analogue à l'exemple 12, par réaction de 500 mg (1,53 mmole) de 5-(3'-Methylaminomethyl-biphenyl-4-ylmethyl)-thiazolidine-2,4-dione obtenu en 1(f) avec 370 mg (1,6 mmole) de l'acide 6-Propyloxy-naphtalène-2-carboxylique, on obtient, après purification par chromatographie sur colonne de silice éluée avec un mélange heptane et acétate d'éthyle (50/50), 530 mg (61%) de 6-(Propyloxy)-N-[4'-(2,4-dioxo-thiazolidin-5-ylmethyl)-biphenyl-3-ylmethyl]-N-methyl-naphthalene-2-carboxamide sous forme d'une poudre blanche de point de fusion 108-110°C.
RMN ¹H (DMSO d6 ; 400MHz) : 1,00 (t, J=7,4 Hz, 3H) ; 1,80 (m, 2H) ; 2,96 (s large, 3H) ; 3,17 (dd, J=9,2 Hz et J=14,1 Hz, 1H); 3,44 (dd, J=4,2 Hz et J=14,1 Hz, 1 H) ; 4,06 (t, J=6,5 Hz, 2H); 4,63-4,77 (m, 2H); 4,96 (dd, J=4,3 Hz zt J=9,1 Hz, 1 H) ; 7,19-7,98 (m, 14H) ; 12,10 (s large, 1H)

### EXEMPLE 27: 6-(Hexyloxy)-N-[4'-(2,4-dioxo-thiazolidin-5-ylmethyl)-biphenyl-3-ylmethyl]-N-methyl-naphthalene-2-carboxamide.

De manière analogue à l'exemple 12, par réaction de 500 mg (1,53 mmole) de 5-(3'-Methylaminomethyl-biphenyl-4-ylmethyl)-thiazolidine-2,4-dione obtenu en 1 (f) avec 436 mg (1,6 mmole) de l'acide 6-Hexyloxy-naphtalène-2-carboxylique, on obtient, après purification par chromatographie sur colonne de silice éluée avec un mélange heptane et acétate d'éthyle (60/40), 520 mg (56%) de 6-(Hexyloxy)-N-[4'-(2,4-dioxo-thiazolidin-5-ylmethyl)-biphenyl-3-ylmethyl]-N-methyl-naphthalene-2-carboxamide sous forme d'une poudre blanche de point de fusion 117°C.
RMN ¹H (DMSO d6 ; 400MHz) : 0,88(m, 3H) ; 1,32 (m, 4H) ; 1,44 (m, 2H) ; 1,79 (m, 2H); 2,96 (s large, 3H) ; 3,17 (dd, J=9,2 Hz et J=14,1 Hz, 1H) ; 3,44 (dd, J=4,2 Hz et J=14,1Hz, 1 H) ; 4,09 (t, J=6,5 Hz, 2H) ; 4,63-4,77 (m, 2H) ; 4,96 (dd, J=4,3 Hz zt J=9,1 Hz, 1H); 7,19-7,98 (m, 14H) ; 12,10 (s large, 1H)

### EXEMPLE 28: 6-(Nonyloxy)-N-[4'-(2,4-dioxo-thiazolidin-5-ylmethyl)-biphenyl-3-ylmethyl]-N-methyl-naphthalene-2-carboxamide.

De manière analogue à l'exemple 12, par réaction de 500 mg (1,53 mmole) de 5-(3'-Methylaminomethyl-biphenyl-4-ylmethyl)-thiazolidine-2,4-dione obtenu en 1(f) avec 503 mg (1,6 mmole) de l'acide 6-Nonyloxy-naphtalène-2-carboxylique, on obtient, après purification par chromatographie sur colonne de silice éluée avec un mélange heptane et acétate d'éthyle (60/40), 590 mg (59%) de 6-(Nonyloxy)-N-[4'-(2,4-dioxo-thiazolidin-5-ylmethyl)-biphenyl-3-ylmethyl]-N-methyl-naphthalene-2-carboxamide sous forme d'une poudre blanche de point de fusion 117°C.
RMN ¹H (DMSO d6 ; 400MHz) : 0,85(m, 3H) ; 1,20-1,40 (m, 10H) ; 1,45 (m, 2H) ; 1,78 (m, 2H) ; 2,96 (s large, 3H) ; 3,17 (dd, J=9,2 Hz et J=14,1 Hz, 1H) ; 3,44 (dd, J=4,2 Hz et J=14,1 Hz, 1 H) ; 4,09 (t, J=6,5 Hz, 2H) ; 4,63-4,77 (m, 2H) ; 4,96 (dd, J=4,3 Hz zt J=9,1 Hz, 1H) ; 7,19-7,98 (m, 14H) ; 12,10 (s large, 1H)

### EXEMPLE 29 : N-[4'-(2,4-dioxo-thiazolidin-5-ylmethyl)-biphenyl-3-ylmethyl]-N-methyl-4'-propyl-biphenyl-2-carboxamide.

De manière analogue à l'exemple 12, par réaction de 500 mg (1,53 mmole) de 5-(3'-Methylaminomethyl-biphenyl-4-ylmethyl)-thiazolidine-2,4-dione obtenu en 1 (f) avec 384 mg (1,6 mmole) de l'acide 4-(4'-propyl-phenyl) benzoïque, on obtient, après purification par chromatographie sur colonne de silice éluée avec un mélange heptane et acétate d'éthyle (60/40), 602 mg (68%) de N-[4'-(2,4-dioxo-thiazolidin-5-ylmethyl)-biphenyl-3-ylmethyl]-N-methyl-4'-Propyl-biphenyl-2-carboxamide sous forme d'une poudre blanche.
RMN¹H (DMSO d6 ; 400MHz) : 0,91 (t, J=7,3 Hz, 3H) ; 1,72 (m, 2H); 2,58 (m, 2H) ; 2,93 (s large, 3H) ; 3,19 (dd, J=9,2 Hz et J=14,1 Hz, 1 H) ; 3,43 (dd, J=4,2 Hz et J=14,1Hz, 1H); 4,58-4,78 (m, 2H) ; 4,96 (dd, J=4,3 Hz zt J=9,1 Hz, 1H) ; 7,20-7,75 (m, 16H) ; 12,10 (s large, 1 H)

### EXEMPLE 30 : N-[4'-(2,4-dioxo-thiazolidin-5-ylmethyl)-biphenyl-3-ylmethyl]-N-methyl-4-phenoxy-benzamide.

De manière analogue à l'exemple 12, par réaction de 500 mg (1,53 mmole) de 5-(3'-, Methylaminomethyl-biphenyl-4-ylmethyl)-thiazolidine-2,4-dione obtenu en 1(f) avec 343 mg (1,6 mmole) de l'acide 4-Phenoxy benzoïque, on obtient, après purification par chromatographie sur colonne de silice éluée avec un mélange heptane et acétate d'éthyle en gradient de polarité de (80/20) à (60/40), 545 mg (68%) de N-[4'-(2,4-dioxo-thiazolidin-5-ylmethyl)-biphenyl-3-ylmethyl]-N-methyl-4-phenoxy-benzamide sous forme d'une poudre blanche de point de fusion 95°C.

### EXEMPLE 31 : N-[4'-(2,4-dioxo-thiazolidin-5-ylmethyl)-biphenyl-3-ylmethyl]-N-methyl-7-oxo-7-phenyl-heptanamide.

De manière analogue à l'exemple 12, par réaction de 500 mg (1,53 mmole) de 5-(3'-Methylaminomethyl-biphenyl-4-ylmethyl)-thiazolidine-2,4-dione obtenu en 1(f) avec 352 mg (1,6 mmole) de l'acide 6-benzoylhexanoïque, on obtient, après purification par chromatographie sur colonne de silice éluée avec un mélange heptane et acétate d'éthyle en gradient de polarité de (70/30) à (50/50), 610 mg (75%) de N-[4'-(2,4-dioxo-thiazolidin-5-ylmethyl)-biphenyl-3-ylmethyl]-N-methyl-7-oxo-7-phenyl-heptanamide sous forme d'une poudre blanche de point de fusion 55-56°C.

### EXEMPLE 32: 4'-(2-methoxy-ethoxymethoxy)-N-[4'-(2,4-dioxo-thiazolidin-5-ylmethyl)-biphenyl-3-ylmethyl]-N-methyl-biphenyl-4-carboxamide.

### (a) 4'-(2-Methoxy-ethoxymethoxy)-biphenyl-4-carboxylate de méthyle.

Dans un ballon tricol de 500 ml, introduire dans l'ordre et sous courant d'azote, 10 g (43,8 mmoles) d'ester méthylique de l'acide 4'-Hydroxy-biphenyl-4-carboxylique, 100 ml de THF et 150 ml de DMF. Additionner par petites portions 1,93g (48,2 mmoles) de NaH à 60% dans l'huile et agiter à l'ambiant pendant 15 minutes. Additionner gouttes à gouttes 5,75 ml de 1-Chloromethoxy-2-methoxy-ethane, et agiter à l'ambiant pendant 30 minutes. Jeter le milieu réactionnel sur une solution d'HCl 1 N et extraire à l'acétate d'éthyle. Laver la phase organique à l'eau et la sécher sur sulfate de magnésium. Après filtration et évaporation, on obtient 12g de l'ester méthylique de l'acide 4'-(2-Methoxy-ethoxymethoxy)-biphenyl-4-carboxylique sous forme d'une poudre beige (Rdt=87%)

### (b) Acide 4'-(2-Methoxy-ethoxymethoxy)-biphenyl-4-carboxylique.

Dans un ballon tricol de 250 ml, introduire dans l'ordre, 10g (31,6 mmoles) d'ester méthylique de l'acide 4'-(2-Methoxy-ethoxymethoxy)-biphenyl-4-carboxylique, 100 ml de méthanol et 31 ml d'une solution NaOH 10M. Agiter à 80°C pendant 30 minutes. Après être revenu à l'ambiant, verser le milieu réactionnel sur de l'eau et acidifier avec une solution HCl 1N. Filtrer le précipité. Le reprendre dans de l'heptane. Après filtration et séchage, on obtient 9g d'acide 4'-(2-Methoxy-ethoxymethoxy)-biphenyl-4-carboxylique sous forme de poudre beige (Rdt=98%)

### (c) 4'-(2-methoxy-ethoxymethoxy)-N-[4'-(2,4-dioxo-thiazolidin-5-ylmethyl)-biphenyl-3-ylmethyl]-N-methyl-biphenyl-4-carboxamide

De manière analogue à l'exemple 12, par réaction de 4 g (12,3 mmole) de 5-(3'-Methylaminomethyl-biphenyl-4-ylmethyl)-thiazolidine-2,4-dione obtenu en 1 (f) avec 3,58 g (12,3 mmole) de l'acide 4'-(2-Methoxy-ethoxymethoxy)-biphenyl-4-carboxylique, on obtient, après purification par chromatographie sur colonne de silice éluée avec un mélange heptane et acétate d'éthyle (50/50), puis recristallisation dans du méthanol, 2,9 g (38%) de 4'-(2-methoxy-ethoxymethoxy)-N-[4'-(2,4-dioxo-thiazolidin-5-ylmethyl)-biphenyl-3-ylmethyl]-N-methyl-biphenyl-4-carboxamide sous forme d'une poudre blanche de point de fusion 126-128°C.
RMN ¹H (DMSO d6 ; 400MHz) : 2,93(m, 3H) ; 3,15-3,22 (m, 4H) ; 3,41-3,47 (m, 3H) ; 3,72 (m, 2H) ; 4,61-4,75 (m, 2H) ; 4,96 (dd, J=4,3 Hz zt J=9,1 Hz, 1 H) ; 5,29 (s large, 2H) ; 7,11-7,68 (m, 16H) ; 12,10 (s large, 1H)

### EXEMPLE 33: 4'-hydroxy-N-[4'-(2,4-dioxo-thiazolidin-5-ylmethyl)-biphenyl-3-ylmethyl]-N-methyl-biphenyl-4-carboxamide.

Dans un ballon tricol de 250 ml, introduire dans l'ordre 1,6g de 4'-(2-methoxyethoxymethoxy)-N-[4'-(2,4-dioxo-thiazolidin-5-ylmethyl)-biphenyl-3-ylmethyl]-N-methyl-biphenyl-4-carboxamide, 100 ml de méthanol et 1 ml d'acide sulfurique à 98%. Agiter à l'ambiant pendant 18 heures. Concentrer le milieu réactionnel. Le reprendre dans l'acétate d'éthyle et le laver deux fois à l'eau. Sécher la phase organique sur sulfate de magnésium. Après filtration et évaporation, on cristallise le produit obtenu dans un mélange acétone /dichlorométhane. Après filtration et séchage, on obtient 1,34g (99%) de 4'-hydroxy-N-[4'-(2,4-dioxo-thiazolidin-5-ylmethyl)-biphenyl-3-ylmethyl]-N-methyl-biphenyl-4-carboxamide sous forme d'une poudre blanche de point de fusion 211-213°C.
RMN ¹H (DMSO d6 ; 400MHz) : 2,93(m, 3H) ; 3,18 (dd, J=9,6 Hz et J=14,1 Hz, 1H) ; 3,43 (dd, J=4,3 Hz et J=14,1 Hz, 1H) ; 4,61-4,75 (m, 2H) ; 4,96 (dd, J=4,3 Hz zt J=9,6 Hz, 1H) ; 6,85-7,63 (m, 16H) ; 9,62 (s large, 1 H) ; 12,10 (s large, 1 H)

### EXEMPLE 34: 1-[4'-(2,4-dioxo-thiazolidin-5-ylmethyl)-biphenyl-3-yl]-3-(4-hexyloxyphenyl)-1-methyl-urée.

### (a) (3-Bromo-phenyl)-carbamate de tert-butyle

Dans un ballon de 10 litres, introduire 667g (3 moles) de di-tert-butyldicarbonate. Additionner sous azote 4,5 litres d'une solution de soude 2M et 453g (2,58 moles) de 3-bromoaniline. Chauffer au reflux pendant 4,5 heures. Le milieu réactionnel est extrait à l'acétate d'éthyle, la phase organique est lavée à l'eau, puis évaporée sous vide. Le solide obtenu est repris sous agitation dans l'heptane. Après filtration et séchage, on obtient 609g (86%) de (3-Bromo-phenyl)-carbamate de tert-butyl sous forme d'une poudre blanche.

### (b) (3-Bromo-phenyl)-methyl-carbamate de tert-butyle

Dans un ballon, introduire sous azote 9,33g (0,22 moles) de NaH à 60% dans l'huile et 250 ml de DMF. Additionner goutte à goutte 53g (0,18 moles) de (3-Bromo-phenyl)-carbamic acid tert-butyl ester en solution dans 150 ml de DMF. Après 10 minutes, additionner goutte à goutte 14,5 ml (0,22 moles) d'iodure de méthyle. Agiter à température ambiante pendant 30 minutes. Après filtration du Nal et évaporation du DMF, le milieu est solubilisé dans 350 ml d'acétate d'éthyle et lavé avec deux fois 300 ml d'eau. Après séchage sur sulfate de sodium et évaporation des solvants, on obtient 55g (98%) de (3-Bromo-phenyl)-methyl-carbamate de tert-butyle sous forme d'un liquide jaune.

### (c) (4'-Formyl-biphenyl-3-yl)-methyl-carbamate de tert-butyle

De manière analogue à l'exemple 1(c), par réaction de 55 g (0,19 moles) de (3-Bromo-phenyl)-methyl-carbamate de tert-butyle avec 50,5 g (0,30 moles) de l'acide 4-formylbenzèneboronique, on obtient, après purification par chromatographie sur colonne de silice éluée avec un mélange heptane et acétate d'éthyle (90/10), 48 g (80%) du (4'-Formyl-biphenyl-3-yl)-methyl-carbamate de tert-butyle.

### (d) [4'-(2,4-Dioxo-thiazolidin-5-ylidenemethyl)-biphenyl-3-yl]-methyl-carbamate de tert-butyle

De manière analogue à l'exemple 1(d), par réaction de 40 g (0,128 moles) de (4'-Formyl-biphenyl-3-yl)-methyl-carbamate de tert-butyle avec 15 g (0,128 moles) de 2,4-thiazolidinedione et 3,7 g (0,025 moles) d'acétate de piperidinium et 0,4 I de toluène. On obtient 42,8 g (81,6%) du [4'-(2,4-Dioxo-thiazolidin-5-ylidenemethyl)-biphenyl-3-yl]-methyl-carbamate de tert-butyle.

### (e) [4'-(2,4-Dioxo-thiazolidin-5-ylmethyl)-biphenyl-3-yl]-methyl-carbamate de tert-butyle

Dans un ballon tricol de 50 ml, introduire dans l'ordre 3,69 g (0,009 moles) de [4'-(2,4-Dioxo-thiazolidin-5-ylidenemethyl)-biphenyl-3-yl]-methyl-carbamate de tert-butyle, 6 ml de THF et 7,3 ml de pyridine. Se placer sous azote et additionner goutte à goutte 10 ml d'une solution 2M de LiBH4 (0,02 moles) dans le THF fraîchement préparée. Après 30 minutes d'agitation à l'ambiant, porter au reflux pendant 16 heures. Verser le milieu réactionnel sur 32 ml d'une solution HCl 1N et évaporer le THF sous vide. Filtrer le précipité obtenu. Après séchage, on chromatographie le produit brut (2,75 g) sur 95g de gel de silice en éluant avec un mélange heptane/acétate d'éthyle = 3/7. On obtient 2,45 g (66%) du [4'-(2,4-Dioxo-thiazolidin-5-ylmethyl)-biphenyl-3-yl]-methyl-carbamate de tert-butyle.

### (f) 5-(3'-Methylamino-biphenyl-4-ylmethyl)-thiazolidine-2,4-dione

Dans un ballon de 100 ml, introduire 2,32 g (5,6 mmoles) de [4'-(2,4-Dioxo-thiazolidin-5-ylmethyl)-biphenyl-3-yl]-methyl-carbamic acid tert-butyl ester, 40 ml de dichlorométhane et 6,4g (56 mmoles) d'acide trichloracétique. Après 24h d'agitation à l'ambiant, le milieu réactionnel est concentré à l'évaporateur rotatif et repris dans de l'éther diisopropylique. Après trituration et agitation, il est filtré, repris dans 50 ml d'eau et neutralisé avec 0,53 g (6,2 mmoles) de bicarbonate de sodium. Le précipité est filtré, laver à l'éther éthylique et séché sous vide. On obtient 1,43 g (81,7%) de 5-(3'-Methylamino-biphenyl-4-ylmethyl)-thiazolidine-2,4-dione sous forme de poudre blanche

### (g) 1-[4'-(2,4-dioxo-thiazolidin-5-ylmethyl)-biphenyl-3-yl]-3-(4-hexyloxy-phenyl)-1-methyl-urée

Dans un ballon 50 ml, introduire 0,67 g (2,14 mmoles) de 5-(3'-Methylamino-biphenyl-4-ylmethyl)-thiazolidine-2,4-dione dans 20 ml de dichlorométhane. Additionner 1 g (4,3 mmoles) de 1-Hexyloxy-4-isocyanato-benzene et chauffer au reflux pendant 16 heures. Verser le milieu réactionnel à l'ambiant sur une solution HCl 1 N et extraire à l'acétate d'éthyle. Laver la phase organique à l'eau et la sécher sur sulfate de magnésium. Après évaporation des solvants, le produit obtenu est purifié par chromatographie sur gel de silice (éluant : heptane/acétate d'éthyle = 3/2). On obtient 0,78 g (68%) de 1-[4'-(2,4-dioxo-thiazolidin-5-ylmethyl)-biphenyl-3-yl]-3-(4-hexyloxy-phenyl)-1-methyl-urée sous forme d'une poudre blanche ayant un point de fusion de 146-148°C.
RMN ¹H (DMSO d6 ; 400MHz) : 0,81 (t, 3H) ; 1,29 (m, 4H) ; 1,39 (m, 2H) ; 1,67 (m, 2H) ; ; 3,18 (dd, J=9,6 Hz et J=14,1 Hz, 1 H) ; 3,31 (s, 3H) ; 3,43 (dd, J=4,3 Hz et J=14,1 Hz, 1H) ; 3,90 (m, 2H) ; 4,96 (dd, J=4,3 Hz zt J=9,6 Hz, 1H) ; 6,80 (m, 2H) ; 7,28-7,66 (m, 10H) ; 8,07 (s, 1 H) ; 12,10 (s large, 1 H)

### EXEMPLE 35 : TEST DE TRANSACTIVATION

L'activité agoniste vis à vis des récepteurs PPARγ des composés selon l'invention peut être évaluée par des tests de transactivation.

La capacité des molécules à activer et/ou inhiber les récepteurs PPARγ est évaluée à partir de cellules Hela transfectées de manière stable avec le récepteur chimère Gal-PPARγ (LBD).

Des plaques 96 puits sont ensemencées à raison de 10 000 cellules /100 µl / puit dans du milieu DMEM 10% SDL puis placées 24 heures à 37°C, 7% CO2.

Pour déterminer l'activité agoniste PPARγ, les cellules sont ensuite traitées par ajout de 5 µl puit des molécules à tester à la concentration finale de 1 µM. Des cellules sont également traitées en parallèle avec un agoniste de référence, l'acide (-)-3-{4-[2-(Benzooxazol-2-yl-methyl-amino)-ethoxy]-phenyl}-2-ethoxy-propionique, 1 µM.

Après une nouvelle incubation de 24 heures à 37°C, 7% CO2, un dosage de la luciférase est effectué à l'aide du kit « Steady-Glo Luciferase Assay System » de Promega. Le comptage de la luminescence se fait sur un lecteur de microplaque Microbéta Trilux (Wallac).

L'activité agoniste du produit testé sera exprimée en pourcentage d'activation par rapport au témoin agoniste l'agoniste de référence à 1 µM.

En appliquant ce même protocole avec le récepteur chimère Gal-PPARα, on peut mesurer l'activité agoniste des composés vis à vis des récepteurs PPAR alpha et ainsi la comparer avec celui du récepteur PPARγ.

L'AC50, exprimée en nM, est déterminée comme la concentration permettant d'obtenir 50% d'activation du signal basal par rapport à l'agoniste de référence.

Les résultats obtenus pour les composés selon l'invention sont regroupés dans le tableau suivant :

| | % d'activation (à 1 microM) | | AC50 (en nM) | |
|---|---|---|---|---|
| | PPARα | PPARγ | PPARα | PPARγ |
| Composé de l'exemple 2 | 13.7 | N.T. | N.T. | 55.5 |
| Composé de l'exemple 3 | 22.3 | N.T. | >50000.0 | 13.1 |
| Composé de l'exemple 4 | 39.8 | N.T. | >50000.0 | 24.2 |
| Composé de l'exemple 5 | 26.6 | N.T. | >50000.0 | 23.3 |
| Composé de l'exemple 6 | 7.9 | N.T. | >50000.0 | 4.0 |
| Composé de l'exemple 7 | 37.8 | 97.2 | N.T. | 63.8 |
| Composé de l'exemple 8 | 33.6 | 97.2 | N.T. | 307.3 |
| Composé de l'exemple 9 | 4.3 | 105.9 | N.T. | 181.9 |
| Composé de l'exemple 10 | 13.75 | 183.2 | >50000.0 | 3.3 |
| Composé de l'exemple 11 | 13.15 | 90.1 | >50000.0 | 15.1 |
| Composé de l'exemple 12 | 12.3 | 167.9 | >50000.0 | 3.2 |
| Composé de l'exemple 13 | 13.4 | 112.3 | >50000.0 | 36.7 |
| Composé de l'exemple 14 | 19.0 | 76.7 | N.T. | 142.8 |
| Composé de l'exemple 15 | 28.8 | 101.6 | >50000.0 | 5.0 |
| Composé de l'exemple 16 | 22.9 | 93.3 | >50000.0 | 0.55 |
| Composé de l'exemple 17 | 15.1 | 96.1 | >50000.0 | 3.8 |
| Composé de l'exemple 18 | 37.3 | 90.7 | >50000.0 | 42.5 |
| Composé de l'exemple 19 | 100.2 | 102.1 | >50000.0 | 2.9 |
| Composé de l'exemple 20 | 41.9 | 113.5 | >50000.0 | 7.7 |
| Composé de l'exemple 21 | -4.2 | 104.2 | N.T. | 22 |
| Composé de l'exemple 22 | -3.9 | 108.3 | N.T. | 4.2 |
| Composé de l'exemple 23 | 11.9 | 90.0 | N.T. | 1.0 |

| | | | | |
|---|---|---|---|---|
| N.T signifie non testé | | | | |

Ces résultats montrent l'activité de transactivation des composés selon l'invention. Ces résultats montrent plus particulièrement la spécificité de l'activation des composés de l'invention pour le sous-type PPAR-γ, comparé à l'activation des composés pour le sous-type PPAR-α.

### EXEMPLE 36 : TEST DE BINDING

L'affinité des composés de l'invention pour le récepteur PPARγ humain a été déterminée dans un test de binding, par compétition de la fixation d'un agoniste de référence, le composé suivant tritié 5-{4-[2-(Methyl-pyridin-2-yl-amino)-ethoxy]-benzyl}-thiazolidine-2,4-dione.

Les récepteurs sont obtenus par infection de cellules d'insecte SF9 par un bacculovirus recombinant. Ils se présentent sous forme d'hétérodimères hPPARγ /RXRα. La présence de RXRα augmente la solubilité et la stabilité du récepteur hPPARγ et, par conséquent, son activité biologique, sans pour autant interférer dans la détermination des constantes de binding.

La technique d'adsorption sur gel d'hydroxylapatite a été utilisée pour séparer le ligand lié au récepteur du ligand libre. Les résultats sont exprimés en valeur de Kd (nM) qui représente la constante de dissociation à l'équilibre obtenue pour chaque composé.

Les résultats obtenus pour les composés selon l'invention sont regroupés dans le tableau suivant :

| | Binding à PPARγ Kd (en nM) |
|---|---|
| Composé de l'exemple 3 | 375.0 |
| Composé de l'exemple 4 | 250.0 |
| Composé de l'exemple 5 | 500.0 |
| Composé de l'exemple 6 | 60.0 |
| Composé de l'exemple 10 | 60.0 |
| Composé de l'exemple 11 | 375.0 |
| Composé de l'exemple 12 | 60.0 |
| Composé de l'exemple 13 | 500.0 |
| Composé de l'exemple 15 | 60.0 |
| Composé de l'exemple 16 | 4.0 |
| Composé de l'exemple 17 | 15.0 |
| Composé de l'exemple 19 | 8.0 |
| Composé de l'exemple 20 | 9.5 |

Ces résultats montrent la très bonne affinité des composés selon la présente invention pour le récepteur PPAR-γ.

### EXEMPLE 37

Dans cet exemple, on a illustré diverses formulations concrètes à base des composés selon l'invention.

### A- VOIE ORALE

(a) Comprimé de 0,2 g
   - Composé de l'exemple 16 0,001 g
   - Amidon 0,114 g
   - Phosphate bicalcique 0,020 g
   - Silice 0,020 g
   - Lactose 0,030 g
   - Talc 0,010 g
   - Stéarate de magnésium 0,005 g
(b) Suspension buvable en ampoules de 5 ml
   - Composé de l'exemple 17 0,001 g
   - Glycérine 0,500 g
   - Sorbitol à 70% 0,500 g
   - Saccharinate de sodium 0,010 g
   - Parahydroxybenzoate de méthyle 0,040 g
   - Arome qs
   - Eau purifiée qsp 5 ml
(c) Comprimé de 0,8 g
   - Composé de l'exemple 19 0,500 g
   - Amidon prégélatinisé 0,100 g
   - Cellulose microcristalline 0,115 g
   - Lactose 0,075 g
   - Stéarate de magnésium 0,010 g
(d) Suspension buvable en ampoules de 10 ml
   - Composé de l'exemple 20 0,200 g
   - Glycérine 1,000 g
   - Sorbitol à 70% 1,000 g
   - Saccharinate de sodium 0,010 g
   - Parahydroxybenzoate de méthyle 0,080 g
   - Arome qs
   - Eau purifiée qsp 10 ml

### B- VOIE TOPIQUE

(a) Onguent
   - Composé de l'exemple 12 0,020 g
   - Myristate d'isopropyle 81,700 g
   - Huile de vaseline fluide 9,100 g
   - Silice ("Aérosil 200" vendue par DEGUSSA) 9,180 g
(b) Onguent
   - Composé de l'exemple 15 0,300 g
   - Vaseline blanche codex qsp 100 g
(c) Crème Eau-dans-Huile non ionique
   - Composé de l'exemple 10 0,100 g
   - Mélange d'alcools de lanoline émulsifs, de cires et d'huiles ("Eucerine anhydre" vendu par BDF) 39,900 g
   - Parahydroxybenzoate de méthyle 0,075 g
   - Parahydroxybenzoate de propyle 0,075 g
   - Eau déminéralisée stérile qsp 100 g
(d) Lotion
   - Composé de l'exemple 19 0,100 g
   - Polyéthylène glycol (PEG 400) 69,900 g
   - Ethanol à 95% 30,000 g
(e) Onguent hydrophobe
   - Composé de l'exemple 20 0,300 g
   - Miristate d'isopropyle 36,400 g
   - Huile de silicone ("Rhodorsil 47 V 300" vendu par RHONE-POULENC) 36,400 g
   - Cire d'abeille 13,600 g
   - Huile de silicone ("Abil 300.000 est" vendu par GOLDSCHMIDT) qsp 100 g
(f) Crème Huile-dans-Eau non ionique
   - Composé de l'exemple 16 1,000 g
   - Alcool cétylique 4,000 g
   - Monostéarate de glycérole 2,500 g
   - Stéarate de PEG 50 2,500 g
   - Beurre de karité 9,200 g
   - Propylène glycol 2,000 g
   - Parahydroxybenzoate de méthyle 0,075 g
   - Parahydroxybenzoate de propyle 0,075 g
   - Eau déminéralisée stérile qsp 100 g

## Revendications

1. Composés **caractérisés par le fait qu'**ils répondent à la formule (I) suivante : 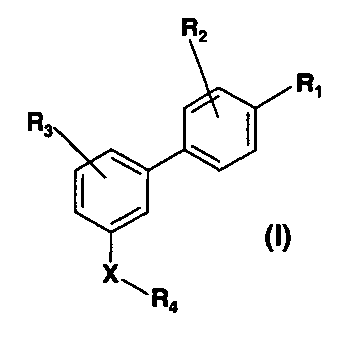 dans laquelle :
- R₁ représente un radical de formules (a) ou (b) suivantes : 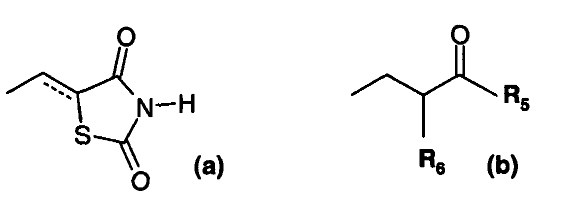 R₅ et R₆ ayant les significations données ci-après,
- R₂ et R₃ identiques ou différents représentent un atome d'hydrogène, un radical alkyle ayant de 1 à 6 atomes de carbone, un radical aryle, un atome d'halogène, un radical -OR₇, un radical polyéther, un radical nitro ou un radical amino pouvant éventuellement être substitué par des radicaux alkyle ayant de 1 à 6 atomes de carbone ;
R₇ ayant la signification donnée ci-après,
- X représente les liaisons de structures suivantes :
-CH₂-N(R₈)-CO-
-N(R₈)-CO-N(R₉)-
-N(R₈)-CO-CH₂-
-N(R₈)-CH₂-CO-
pouvant être lues de gauche à droite ou inversement
R₈ et R₉ ayant les significations données ci-après,
- R₄ représente :
- un radical phényle, benzyle, phénéthyle, thiényle, furyle ou pyridyle, tous ces radicaux étant substitués par un groupement R₁₀,
R₁₀ ayant les significations données ci-après,
- un radical pyrrolyle, pyrazinyle, naphthyle, biphényle, indolyle, indényle, benzothiényle, benzofuryle, benzothiazolyle ou quinolyle, tous ces radicaux pouvant être mono ou di-substitués par un groupement R₁₁ et/ou R₁₂ ;
R₁₁ et R₁₂ ayant les significations données ci-après,
- un radical -(CH₂)ₙ-(CO)_{q}R₁₃,
n, q et R₁₃ ayant les significations données ci-après,
- un radical adamantyle, diphénylméthyl, diphényléthyle, diphénylpropyle, diphénylbutyle, cyclopropylméthyle, cyclopentyléthyle, 2-benzimidazolyléthyle, cyclohexyl méthyle, phénoxyphényl, 9H-fluorényl, benzyloxyphényl, 4-heptyloxyphényl, ou 4-(6-methyl-2-benzothiazolyl)phenyl ;
- un radical -(CH₂)ₙ-O-R₁₃
n et R₁₃ ayant les significations données ci-après,
- R₅ représente un radical hydroxy ou un radical alkoxy ayant de 1 à 9 atomes de carbone,
- R₆ représente un radical alkyle ayant de 1 à 6 atomes de carbone, un radical OR₁₄ ou un radical SR₁₄,
R₁₄ ayant les significations données ci-après,
- R₇ représente un atome d'hydrogène, un radical alkyle ayant de 1 à 6 atomes de carbone, un radical aryle ou un radical aralkyle,
- R₈ représente un atome d'hydrogène ou un radical alkyle ayant de 1 à 6 atomes de carbone,
- R₉ représente un atome d'hydrogène ou un radical alkyle ayant de 1 à 6 atomes de carbone,
- R₁₀ représente : un radical -S(O)ₘR₁₅
un radical -(CH2)ₚ-COR₁₆
un radical -O-R₁₇
m, p, R₁₅, R₁₆, R₁₇ ayant les significations données ci-après,
- R₁₁ et R₁₂ représentent un atome d'halogène, un radical CF₃, un radical alkyle ayant de 1 à 12 atomes de carbone, un radical alkoxy ayant de 1 à 9 atomes de carbone, un radical polyéther, une fonction nitro, un radical hydroxyle éventuellement protégé par un groupe acétyle, benzoyle, une fonction amino éventuellement substituée par au moins un alkyle ayant de 1 à 12 atomes de carbone ou par un radical -CONH-R₂₄, ou protégée par un groupe acétyle ou benzoyle, un radical -S(O)ₘR₁₅, un radical (CH₂)ₚ-COR₁₆ ou un radical -OR₁₇,
m, p, R₁₅, R₁₆, R₁₇, R₂₄ ayant les significations données ci-après,
- n peut prendre les valeurs allant de 1 à 9,
- q peut prendre les valeurs 0 ou 1,
- R₁₃ représente un radical -OR₁₈, un radical -N(R₁₉)(R₂₀), un radical aryle, un radical aralkyle ou un radical hétéroaryle,
R₁₈, R₁₉, R₂₀ ayant les significations données ci-après,
- m peut prendre les valeurs 0, 1 ou 2,
- p peut prendre les valeurs 0, 1 ou 2,
- R₁₄ représente un radical alkyle ayant de 1 à 12 atomes de carbone, un radical CF₃, un radical aryle ou un radical aralkyle,
- R₁₅ représente un radical alkyle ayant de 1 à 12 atomes de carbone, un radical aryle ou un radical aralkyle,
- R₁₆ représente un radical alkyle ayant de 1 à 12 atomes de carbone, un radical - OR₂₁, un radical -N(R₂₂)(R₂₃), un radical aryle ou un radical aralkyle,
R₂₁, R₂₂, R₂₃ ayant les significations données ci-après,
- R₁₇ représente un radical aryle ou un radical aralkyle,
- R₁₈ représente un atome d'hydrogène ou un radical alkyle ayant de 1 à 12 atomes de carbone,
- R₁₉ et R₂₀ identiques ou différents représentent un atome d'hydrogène, un radical alkyle ayant de 1 à 12 atomes de carbone, ou pris ensemble peuvent former un hétérocycle,
- R₂₁ représente un atome d'hydrogène ou un radical alkyle ayant de 1 à 12 atomes de carbone,
- R₂₂ et R₂₃ identiques ou différents représentent un atome d'hydrogène, un radical alkyle ayant de 1 à 12 atomes de carbone, ou pris ensemble peuvent former un hétérocycle ;
- R₂₄ représente un radical phényl, diphénylméthyl, diphénylpropyle, diphénylbutyle, biphénylyl, phénoxyphényl, 9H-fluorényl, 4-benzyloxyphényl, 4-heptyloxyphényl, ou 4-(6-methyl-2-benzothiazolyl)phenyl,
et les sels des composés de formule (I) lorsque R₁ contient une fonction acide carboxylique ainsi que les isomères optiques et géométriques desdits composés de formule (1).

2. Composés selon la revendication 1, **caractérisés par le fait qu'**ils se présentent sous forme de sels d'un métal alcalin ou alcalino-terreux, de sels de zinc, ou de sels d'une amine organique.

3. Composés selon l'une des revendications 1 ou 2, **caractérisés par le fait que** les radicaux alkyles ayant de 1 à 6 atomes de carbone sont choisis parmi les radicaux méthyle, éthyle, isopropyle, butyle, tertiobutyle, hexyle.

4. Composés selon l'une des revendications 1 ou 2, **caractérisés par le fait que** les radicaux alkyles ayant de 1 à 12 atomes de carbone sont choisis parmi les radicaux méthyle, éthyle, isopropyle, butyle, tertiobutyle, hexyle, octyle, décyle, ou dodécyle.

5. Composés selon l'une des revendications 1 ou 2, **caractérisés par le fait que** les radicaux polyethers sont choisis parmi les radicaux polyethers ayant de 1 à 6 atomes de carbone interrompus par au moins un atome d'oxygène tel que les radicaux méthoxyméthoxy, éthoxyméthoxy, ou méthoxyéthoxyméthoxy.

6. Composés selon l'une des revendications 1 ou 2, **caractérisés par le fait que** l'atome d'halogène est choisi dans le groupe constitué par un atome de fluor, de chlore, ou de brome.

7. Composés selon l'une des revendications 1 ou 2, **caractérisés par le fait que** le radical alkoxy ayant de 1 à 9 atomes de carbone est choisi dans le groupe constitué par les radicaux méthoxy, éthoxy, isopropyloxy, tertio-butoxy, ou hexyloxy.

8. Composés selon l'une des revendications 1 ou 2, **caractérisés par le fait que** le radical aryle est choisi parmi un radical phényle ou naphthyle pouvant être mono ou di-substitué par un atome d'halogène, un radical CF₃, un radical alkyle ayant de 1 à 12 atomes de carbone, un radical alkoxy ayant de 1 à 6 atomes de carbone, une fonction nitro, un radical polyéther, un radical hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

9. Composés selon l'une des revendications 1 ou 2, **caractérisés par le fait que** le radical aralkyle est choisi parmi un radical benzyle ou phénethyle pouvant être mono ou di-substitué par un atome d'halogène, un radical CF₃, un radical alkyle ayant de 1 à 12 atomes de carbone, un radical alkoxy ayant de 1 à 6 atomes de carbone, une fonction nitro, un radical polyéther, un radical hydroxyle éventuellement protégé par un groupe acétyle, benzoyle ou une fonction amino éventuellement protégée par un groupe acétyle, benzoyle ou éventuellement substituée par au moins un alkyle ayant de 1 à 12 atomes de carbone.

10. Composés selon l'une des revendications 1 ou 2, **caractérisés par le fait que** le radical hétéroaryle est choisi dans le groupe constitué par un radical pyridyle, furyle, thiényle, ou isoxazolyle, éventuellement substitué par au moins un atome d'halogène, un radical alkyle ayant de 1 à 12 atomes de carbone, un radical alkoxy ayant de 1 à 6 atomes de carbone, une fonction nitro, un radical polyéther, un radical hydroxyle éventuellement protégé par un groupe acétyle, benzoyle ou une fonction amino éventuellement protégée par un groupe acétyle, benzoyle ou éventuellement substituée par au moins un radical alkyle ayant de 1 à 12 atomes de carbone.

11. Composés selon l'une des revendications 1 ou 2, **caractérisés par le fait que** l'hétérocycle est choisi dans le groupe constitué par un radical pipéridino, morpholino, pyrrolidino ou pipérazino éventuellement substitué par un radical alkyle ayant de 1 à 12 atomes de carbone.

12. Composés selon la revendication 1, **caractérisés par le fait qu'**ils sont pris, seuls ou en mélange, dans le groupe constitué par :
1- 7-{[4'-(2,4-Dioxo-thiazolidin-5-ylmethyl)-biphenyl-3-ylmethyl]-methyl-carbamoyl}-heptanoate de méthyle ;
2- 9-{[4'-(2,4-Dioxo-thiazolidin-5-ylmethyl)-biphenyl-3-ylmethyl]-methyl-carbamoyl}-nonanoate de méthyle ;
3- N-[4'-(2,4-Dioxo-thiazolidin-5-ylmethyl)-biphenyl-3-ylmethyl]-N-methyl-terephthalamate de méthyle ;
4- 3-Cyclopentyl-N-[4'-(2,4-dioxo-thiazolidin-5-ylmethyl)-biphenyl-3-ylmethyl]-N-methyl-propionamide ;
5- N-[4'-(2,4-dioxo-thiazolidin-5-ylmethyl)-biphenyl-3-ylmethyl]-N-methyl-naphthalene-1-carboxamide ;
6- N-[4'-(2,4-dioxo-thiazolidin-5-ylmethyl)-biphenyl-3-ylmethyl]-N-methyl-naphthalene-2-carboxamide ;
7- N-[4'-(2,4-Dioxo-thiazolidin-5-ylmethyl)-biphenyl-3-ylmethyl]-N-methyl-2-phenoxy-Acetamide ;
8- N-[4'-(2,4-dioxo-thiazolidin-5-ylmethyl)-biphenyl-3-ylmethyl]-N-methyl-1-Methyl-1 H-pyrrole-2-carboxamide ;
9- N-[4'-(2,4-dioxo-thiazolidin-5-ylmethyl)-biphenyl-3-ylmethyl]-N-methyl-adamantane-1-carboxamide ;
10- N-[4'-(2,4-dioxo-thiazolidin-5-ylmethyl)-biphenyl-3-ylmethyl]-N-methyl-biphenyl-4-carboxamide ;
11- N-[4'-(2,4-dioxo-thiazolidin-5-ylmethyl)-biphenyl-3-ylmethyl]-N-methyl-benzo[b]thiophene-2-carboxamide ;
12- N-[4'-(2,4-dioxo-thiazolidin-5-ylmethyl)-biphenyl-3-ylmethyl]-N-methyl-6-oxo-6-phenyl-hexanamide ;
13- 4-Dimethylamino-N-[4'-(2,4-dioxo-thiazolidin-5-ylmethyl)-biphenyl-3-ylmethyl]-N-methyl-naphthalene-1-carboxamide ;
14- N-[4'-(2,4-Dioxo-thiazolidin-5-ylmethyl)-biphenyl-3-ylmethyl]-4-methanesulfonyl-N-methyl-benzamide ;
15- N-[4'-(2,4-Dioxo-thiazolidin-5-ylmethyl)-biphenyl-3-ylmethyl]-N-methyl-4-(1-phenyl-methanoyl)-benzamide ;
16- 6-(2-Methoxy-ethoxymethoxy)-N-[4'-(2,4-dioxo-thiazolidin-5-ylmethyl)-biphenyl-3-ylmethyl]-N-methyl-naphthalene-2-carboxamide ;
17- 6-Hydroxy-N-[4'-(2,4-dioxo-thiazolidin-5-ylmethyl)-biphenyl-3-ylmethyl]-N-methyl-naphthalene-2-carboxamide ;
18- N-[4'-(2,4-Dioxo-thiazolidin-5-ylmethyl)-biphenyl-3-ylmethyl]-N-methyl-4-methylsulfanyl-benzamide ;
19- Acide (S)-3-(3'-{[(1-Biphenyl-4-yl-methanoyl)-methyl-amino]-methyl}-biphenyl-4-yl)-2-ethoxy-propionique ;
20- Acide (S)-2-Ethoxy-3-(3'-{[methyl-(6-oxo-6-phenyl-hexanoyl)-amino]-methyl}-biphenyl-4-yl)-propionique ;
21- 1-[4'-(2,4-Dioxo-thiazolidin-5-ylmethyl)-biphenyl-3-yl]-1-methyl-3-naphthalen-2-yl-urée ;
22- 3-(4-Dimethylamino-phenyl)-1-[4'-(2,4-dioxo-thiazolidin-5-ylmethyl)-biphenyl-3-yl]-1-methyl-urée ;
23- Acide (S)-2-Ethoxy-3-{3'-[({1-[6-(2-methoxy-ethoxymethoxy)-naphthalen-2-yl]-methanoyl}-methyl-amino)-methyl]-biphenyl-4-yl}-propionique ;
24- 6-(Methoxymethoxy)-N-[4'-(2,4-dioxo-thiazolidin-5-ylmethyl)-biphenyl-3-ylmethyl]-N-methyl-naphthalene-2-carboxamide ;
25- 6-(Methoxycarbonyl)-N-[4'-(2,4-dioxo-thiazolidin-5-ylmethyl)-biphenyl-3-ylmethyl]-N-methyl-naphthalene-2-carboxamide ;
26- 6-(Propyloxy)-N-[4'-(2,4-dioxo-thiazolidin-5-ylmethyl)-biphenyl-3-ylmethyl]-N-methyl-naphthalene-2-carboxamide ;
27- 6-(Hexyloxy)-N-[4'-(2,4-dioxo-thiazolidin-5-ylmethyl)-biphenyl-3-ylmethyl]-N-methyl-naphthalene-2-carboxamide ;
28- 6-(Nonyloxy)-N-[4'-(2,4-dioxo-thiazolidin-5-ylmethyl)-biphenyl-3-ylmethyl]-N-methyl-naphthalene-2-carboxamide ;
29- N-[4'-(2,4-dioxo-thiazolidin-5-ylmethyl)-biphenyl-3-ylmethyl]-N-methyl-4'-propyl-biphenyl-2-carboxamide ;
30- N-[4'-(2,4-dioxo-thiazolidin-5-ylmethyl)-biphenyl-3-ylmethyl]-N-methyl-4-phenoxy-benzamide ;
31- N-[4'-(2,4-dioxo-thiazolidin-5-ylmethyl)-biphenyl-3-ylmethyl]-N-methyl-7-oxo-7-phenyl-heptanamide ;
32- Acide (6-{[4'-(2,4-Dioxo-thiazolidin-5-ylmethyl)-biphenyl-3-ylmethyl]-methyl-carbamoyl}-naphthalen-2-yloxy)-acetique ;
33- Ester méthylique de l'acide (6-{[4'-(2,4-Dioxo-thiazolidin-5-ylmethyl)-biphenyl-3-ylmethyl]-méthyl-carbamoyl}-naphthalen-2-yloxy)-acetique ;
34- 6-Methoxy-N-[4'-(2,4-dioxo-thiazolidin-5-ylmethyl)-biphenyl-3-ylmethyl]-N-methyl-naphtalène-2-carboxamide ;
35- 6-Acetoxy-N-[4'-(2,4-dioxo-thiazolidin-5-ylmethyl)-biphenyl-3-ylmethyl]-N-methyl-naphtalène-2-carboxamide ;
36- 6-Amino-N-[4'-(2,4-dioxo-thiazotidin-5-ylmethyl)-biphenyl-3-ylmethyl]-N-methyl-naphtalène-2-carboxamide ;
37- 6-Acetylamino-N-[4'-(2,4-dioxo-thiazolidin-5-ylmethyl)-biphenyl-3-ylmethyl]-N-methyl-naphtalène-2-carboxamide ;
38- 1-Hydroxy-N-[4'-(2,4-dioxo-thiazolidin-5-ylmethyl)-biphenyl-3-ylmethyl]-N-methyl-naphtalène-2-carboxamide ;
39- 1-Methoxy-N-[4'-(2,4-dioxo-thiazolidin-5-ylmethyl)-biphenyl-3-ylmethyl]-N-methyl-naphtalène-2-carboxamide ;
40- 6-Bromo-N-[4'-(2,4-dioxo-thiazolidin-5-ylmethyl)-biphenyl-3-ylmethyl]-N-methyl-naphtalène-2-carboxamide ;
41- Acide 6-carboxylique-N-[4'-(2,4-dioxo-thiazolidin-5-ylmethyl)-biphenyl-3-ylmethyl]-N-methyl-naphtalène-2-carboxamide ;
42- Ester methylique de l'acide 6-carboxylique-N-[4'-(2,4-dioxo-thiazolidin-5-ylmethyl)-biphenyl-3-ylmethyl]-N-methyl-naphtalène-2-carboxamide ;
43- 6-(3-Phenyl-ureido)-N-[4'-(2,4-dioxo-thiazolidin-5-ylmethyl)-biphenyl-3-ylmethyl]-N-methyl-naphtalène-2-carboxamide ;
44- 3-Hydroxy-N-[4'-(2,4-dioxo-thiazolidin-5-ylmethyl)-biphenyl-3-ylmethyl]-N-methyl-naphtalène-2-carboxamide ;
45- 3-Methoxy-N-[4-(2,4-dioxo-thiazolidin-5-ylmethyl)-biphenyl-3-ylmethyl]-N-methyl-naphtalène-2-carboxamide ;
46- N-[4'-(2,4-dioxo-thiazolidin-5-ylmethyl)-biphenyl-3-ylmethyl]-N-methyl-4'-hydroxy-biphenyl-4-carboxamide ;
47- N-[4'-(2,4-dioxo-thiazolidin-5-ylmethyl)-biphenyl-3-ylmethyl]-N-methyl-4'-methoxy-biphenyl-4-carboxamide ;
48- N-[4'-(2,4-dioxo-thiazolidin-5-ylmethyl)-biphenyl-3-ylmethyl]-N-methyl-4'-propyloxy-biphenyl-4-carboxamide ;
49- N-[4'-(2,4-dioxo-thiazolidin-5-ylmethyl)-biphenyl-3-ylmethyl]-N-methyl-4'-hexyloxy-biphenyl-4-carboxamide ;
50- N-[4'-(2,4-dioxo-thiazolidin-5-ylmethyl)-biphenyl-3-ylmethyl]-N-methyl-4'-acetoxy-biphenyl-4-carboxamide ;
51- Acide (4'-{[4'-(2,4-Dioxo-thiazolidin-5-ylmethyl)-biphenyl-3-ylmethyl]-methyl-carbamoyl}-biphenyl-4-yloxy)-acetique ;
52- Ester méthylique de l'acide (4'-{[4'-(2,4-Dioxo-thiazolidin-5-ylmethyl)-biphenyl-3-ylmethyl]-methyl-carbamoyl}-biphenyl-4-yloxy)-acetique ;
53- N-[4'-(2,4-dioxo-thiazolidin-5-ylmethyl)-biphenyl-3-ylmethyl]-N-methyl-4'-methoxymethoxy-biphenyl-4-carboxamide ;
54- N-[4'-(2,4-dioxo-thiazolidin-5-ylmethyl)-biphenyl-3-ylmethyl]-N-methyl-4'-nonyloxy-biphenyl-4-carboxamide ;
55- N-[4'-(2,4-dioxo-thiazolidin-5-ylmethyl)-biphenyl-3-ylmethyl]-N-methyl-4'-(2-methoxy-ethoxy)-biphenyl-4-carboxamide ;
56- 3-Biphenyl-4-yl-1-[4'-(2,4-dioxo-thiazolidin-5-ylmethyl)-biphenyl-3-yl]-1-methyl-urea ;
57- 1-[4'-(2,4-Dioxo-thiazolidin-5-ylmethyl)-biphenyl-3-yl]-3-(9H-fluoren-2-yl)-1-methylurea ;
58- 1-[4'-(2,4-Dioxo-thiazolidin-5-ylmethyl)-biphenyl-3-yl]-3-(9H-fluoren-9-yl)-1-methylurea ;
59- 3-Benzhydryl-1-[4'-(2,4-dioxo-thiazolidin-5-ylmethyl)-biphenyl-3-yl]-1-methyl-urea ;
60- 1-[4'-(2,4-Dioxo-thiazolidin-5-ylmethyl)-biphenyl-3-yl]-1-methyl-3-(3-phenoxy-phenyl)-urea ;
61- 1-[4'-(2,4-Dioxo-thiazolidin-5-ylmethyl)-biphenyl-3-yl]-3-(4-heptyloxy-phenyl)-1-methyl-urea ;
62- 3-(4-Benzyloxy-phenyl)-1-[4'-(2,4-dioxo-thiazolidin-5-ylmethyl)-biphenyl-3-yl]-1-methyl-urea ;
63- 1-[4'-(2,4-Dioxo-thiazolidin-5-ylmethyl)-biphenyl-3-yl]-1-methyl-3-[4-(6-methyl-benzothiazol-2-yl)-phenyl]-urea ;
64- 4'-(2-methoxy-ethoxymethoxy)-N-[4'-(2,4-dioxo-thiazolidin-5-ylmethyl)-biphenyl-3-ylmethyl]-N-methyl-biphenyl-4-carboxamide ;
65- 4'-hydroxy-N-[4-(2,4-dioxo-thiazolidin-5-ylmethyl)-biphenyl-3-ylmethyl]-N-methyl-biphenyl-4-carboxamide ;
66- 1-[4'-(2,4-dioxo-thiazolidin-5-ylmethyl)-biphenyl-3-yl]-3-(4-hexyloxy-phenyl)-1-methyl-urée.

13. Composés selon la revendication 1 ou 2, **caractérisés par le fait qu'**ils présentent une des caractéristiques suivantes :
- R₁ représente le radical de formule (a) ou le radical de formule (b) où R₅ représente un radical hydroxy et R₆ représente le radical OR₁₄, et/ou
- X représente la liaison de structure -CH₂-N(R₈)-CO- ou N(R₈)-CO-N(R₉)-, lue de gauche à droite ou inversement.

14. Composés selon l'une quelconque des revendications 1 à 13 à titre de médicament.

15. Utilisation d'un composé selon l'une quelconque des revendications 1 à 13 dans la fabrication d'une composition destinée à réguler et/ou à restaurer le métabolisme des lipides cutanés.

16. Utilisation d'un composé selon l'une quelconque des revendications 1 à 13 dans la fabrication d'une composition destinée au traitement :
- des affections dermatologiques liées à un désordre de la kératinisation portant sur la différenciation et sur la prolifération cellulaire ;
- des ichtyoses, des états ichtyosiformes, de la maladie de Darrier, des kératodermies palmoplantaires, des leucoplasies et des états leucoplasiformes, du lichen cutané ou muqueux (buccal) ;
- des affections dermatologiques avec une composante immuno-allergique inflammatoire, avec ou sans trouble de la prolifération cellulaire ;
- des proliférations dermiques ou épidermiques bénignes ou malignes, d'origine virale ou non ;
- des proliférations pouvant être induites par les ultra-violets ;
- des lésions précancéreuses cutanées ;
- des dermatoses immunes ;
- des maladies immunes bulleuses ;
- des maladies du collagène;
- des affections dermatologiques ou générales à composante immunologique ;
- de désordres cutanés dus à une exposition aux rayonnements U.V, du vieillissement de la peau, photo-induit ou chronologique ou des pigmentations et des kératoses actiniques ;
- des pathologies associées au vieillissement chronologique ou actinique ;
- des troubles de la fonction sébacée ;
- des troubles de la cicatrisation ou des vergetures ; ou
- des désordres de la pigmentation.

17. Utilisation selon la revendication 16, **caractérisée en ce que** les affections dermatologiques liées à un désordre de la kératinisation sont les acnés vulgaires, comédoniennes, polymorphes, rosacées, les acnés nodulokystiques, conglobata, les acnés séniles ou les acnés secondaires telles que l'acné solaire, médicamenteuse ou professionnelle.

18. Utilisation selon la revendication 16, **caractérisée en ce que** les affections dermatologiques avec une composante immuno-allergique inflammatoire sont le psoriasis cutané, muqueux ou unguéal, le rhumatisme psoriasique ou l'atopie cutanée, telle que l'eczéma, l'atopie respiratoire ou l'hypertrophie gingivale.

19. Utilisation selon la revendication 16, **caractérisée en ce que** les proliférations dermiques ou épidermiques sont les verrues vulgaires, les verrues planes l'épidermodysplasie verruciforme, les papillomatoses orales ou florides ou le lymphome T.

20. Utilisation selon la revendication 16, **caractérisée en ce que** les proliférations pouvant être induites par les ultra-violets sont des épithélioma baso et spinocellulaires.

21. Utilisation selon la revendication 16, **caractérisée en ce que** les lésions précancéreuses cutanées sont les kératoacanthomes.

22. Utilisation selon la revendication 16, **caractérisée en ce que** les dermatoses immunes sont le lupus érythémateux.

23. Utilisation selon la revendication 16, **caractérisée en ce que** les maladies du collagène sont la sclérodermie.

24. Utilisation selon la revendication 16, **caractérisée en ce que** les pathologies associées au vieillissement chronologique ou actinique sont la xérose.

25. Utilisation selon la revendication 16, **caractérisée en ce que** les troubles de la fonction sébacée sont l'hyperséborrhée de l'acné ou la séborrhée simple.

26. Utilisation selon la revendication 16, **caractérisée en ce que** les désordres de la pigmentation sont l'hyperpigmentation, le mélasma, l'hypopigmentation ou le vitiligo.

27. Composition pharmaceutique, **caractérisée par le fait qu'**elle comprend, dans un support physiologiquement acceptable, au moins l'un des composés tels que définis à l'une quelconque des revendications 1 à 13.

28. Composition selon la revendication 27, **caractérisée en ce que** la concentration en composé(s) selon l'une des revendications 1 à 13 est comprise entre 0,001% et 10% en poids par rapport au poids total de la composition.

29. Composition selon la revendication 27, **caractérisée en ce que** la concentration en composé(s) selon l'une des revendications 1 à 13 est comprise entre 0, 01% et 1 % en poids par rapport au poids total de la composition.

30. Composition cosmétique, **caractérisée par le fait qu'**elle comprend, dans un support cosmétiquement acceptable, au moins un des composés tels que définis à l'une quelconque des revendications 1 à 13.

31. Composition selon la revendication 30, **caractérisée en ce que** la concentration en composé(s) selon l'une des revendications 1 à 13 est comprise entre 0,001 % et 3% en poids par rapport au poids total de la composition.

32. Utilisation cosmétique d'une composition telle que définie à l'une des revendications 30 ou 31 pour prévenir et/ou traiter les signes du vieillissement et/ou la peau sèche.

33. Utilisation cosmétique d'une composition telle que définie à l'une des revendications 30 ou 31 pour l'hygiène corporelle ou capillaire.

## Claims

1. Compounds, **characterized in that** they correspond to the following formula (I): 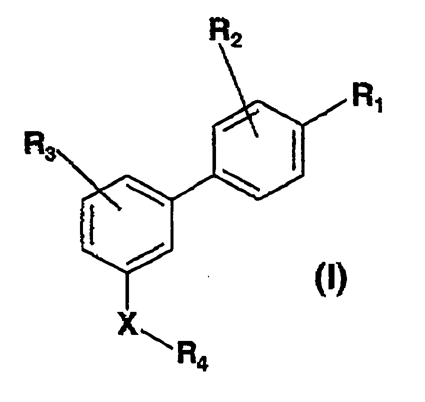 in which:
- R₁ represents a radical of the following formulae (a) or (b): 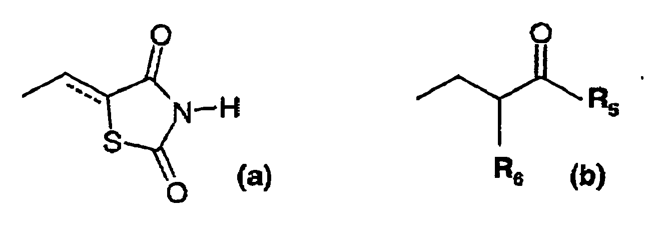 R₅ and R₆ having the meanings given below,
- R₂ and R₃, which may be identical or different, represent a hydrogen atom, an alkyl radical having from 1 to 6 carbon atoms, an aryl radical, a halogen atom, a radical -OR₇, a polyether radical, a nitro radical or an amino radical which may be optionally substituted with alkyl radicals having from 1 to 6 carbon atoms;
R₇ having the meaning given below,
- X represents the bonds having the following structures:
-CH₂-N (R₈) -CO-
-N(R₈)-CO-N(R₉)-
-N(R₈) -CO-CH₂-
-N(R₈)-CH₂-CO-
which may be read from left to right or conversely
R₈ and R₉ having the meanings given below,
- R₄ represents:
- a phenyl, benzyl, phenethyl, thienyl, furyl or pyridyl radical, all these radicals being substituted with a group R₁₀,
R₁₀ having the meanings given below,
- a pyrrolyl, pyrazinyl, naphthyl, biphenyl, indolyl, indenyl, benzothienyl, benzofuryl, benzothiazolyl or quinolyl radical, it being possible for all these radicals to be mono- or disubstituted with a group R₁₁ and/or R₁₂;
R₁₁ and R₁₂ having the meanings given below,
- a radical - (CH₂)ₙ- (CO)_{q}R₁₃,
n, q and R₁₃ having the meanings given below,
- an adamantyl, diphenylmethyl, diphenylethyl, diphenylpropyl, diphenylbutyl, cyclopropylmethyl, cyclopentylethyl, 2-benzimidazolylethyl, cyclohexylmethyl, phenoxyphenyl, 9H-fluorenyl, benzyloxyphenyl, 4-heptyloxyphenyl, or 4-(6-methyl-2-benzothiazolyl)phenyl radical;
- a radical -(CH₂)ₙ-O-R₁₃,
n and R₁₃ having the meanings given below,
- R₅ represents a hydroxyl radical or an alkoxy radical having from 1 to 9 carbon atoms,
- R₆ represents an alkyl radical having from 1 to 6 carbon atoms, a radical OR₁₄ or a radical SR₁₄,
R₁₄ having the meanings given below,
- R₇ represents a hydrogen atom, an alkyl radical having from 1 to 6 carbon atoms, an aryl radical or an aralkyl radical,
- R₈ represents a hydrogen atom or an alkyl radical having from 1 to 6 carbon atoms,
- R₉ represents a hydrogen atom or an alkyl radical having from 1 to 6 carbon atoms,
- R₁₀ represents: a radical -S(O)ₘR₁₅
a radical -(CH₂)ₚ-COR₁₆
a radical -O-R₁₇
m, p, R₁₅, R₁₆ and R₁₇ having the meanings given below,
- R₁₁ and R₁₂ represent a halogen atom, a radical CF₃, an alkyl radical having from 1 to 12 carbon atoms, an alkoxy radical having from 1 to 9 carbon atoms, a polyether radical, a nitro functional group, a hydroxyl radical optionally protected by an acetyl or benzoyl group, an amino functional group optionally substituted with at least one alkyl having from 1 to 12 carbon atoms or with a radical -CONH-R₂₄, or protected by an acetyl or benzoyl group, a radical -S(O)ₘR₁₅, a radical (CH₂)ₚ-COR₁₆ or a radical -OR₁₇,
m, p, R₁₅, R₁₆, R₁₇ and R₂₄ having the meanings given below,
- n may take the values ranging from 1 to 9,
- q may take the values 0 or 1,
- R₁₃ represents a radical -OR₁₈, a radical -N (R₁₉) (R₂₀), an aryl radical, an aralkyl radical or a heteroaryl radical,
R₁₈, R₁₉ and R₂₀ having the meanings given below,
- m may take the values 0, 1 or 2,
- p may take the values 0, 1 or 2,
- R₁₄ represents an alkyl radical having from 1 to 12 carbon atoms, a radical CF₃, an aryl radical or an aralkyl radical,
- R₁₅ represents an alkyl radical having from 1 to 12 carbon atoms, an aryl radical or an aralkyl radical,
- R₁₆ represents an alkyl radical having from 1 to 12 carbon atoms, a radical -OR₂₁, a radical -N(R₂₂)(R₂₃), an aryl radical or an aralkyl radical,
R₂₁, R₂₂ and R₂₃ having the meanings given below,
- R₁₇ represents an aryl radical or an aralkyl radical,
- R₁₈ represents a hydrogen atom or an alkyl radical having from 1 to 12 carbon atoms,
- R₁₉ and R₂₀, which may be identical or different, represent a hydrogen atom, an alkyl radical having from 1 to 12 carbon atoms, or taken together may form a heterocycle,
- R₂₁ represents a hydrogen atom or an alkyl radical having from 1 to 12 carbon atoms,
- R₂₂ and R₂₃, which may be identical or different, represent a hydrogen atom, an alkyl radical having from 1 to 12 carbon atoms, or taken together may form a heterocycle,
- R₂₄ represents a phenyl, diphenylmethyl, diphenylpropyl, diphenylbutyl, biphenylyl, phenoxyphenyl, 9H-fluorenyl, 4-benzyloxyphenyl, 4-heptyloxyphenyl, or 4-(6-methyl-2-benzothiazolyl)phenyl radical,
and the salts of the compounds of formula (I) when R₁ contains a carboxylic acid functional group and the optical and geometric isomers of the said compounds of formula (I).

2. Compounds according to Claim 1, **characterized in that** they are provided in the form of salts of an alkali or alkaline-earth metal, of zinc salts, or of salts of an organic amine.

3. Compounds according to either of Claims 1 and 2, **characterized in that** the alkyl radicals having from 1 to 6 carbon atoms are chosen from the methyl, ethyl, isopropyl, butyl, tert-butyl and hexyl radicals.

4. Compounds according to either of Claims 1 and 2, **characterized in that** the alkyl radicals having from 1 to 12 carbon atoms are chosen from the methyl, ethyl, isopropyl, butyl, tert-butyl, hexyl, octyl, decyl and dodecyl radicals.

5. Compounds according to either of Claims 1 and 2, **characterized in that** the polyether radicals are chosen from the polyether radicals having from 1 to 6 carbon atoms interrupted by at least one oxygen atom such as the methoxymethoxy, ethoxymethoxy and methoxyethoxymethoxy radicals.

6. Compounds according to either of Claims 1 and 2, **characterized in that** the halogen atom is chosen from the group consisting of a fluorine, chlorine and bromine atom.

7. Compounds according to either of Claims 1 and 2, **characterized in that** the alkoxy radical having from 1 to 9 carbon atoms is chosen from the group consisting of the methoxy, ethoxy, isopropyloxy, tert-butoxy and hexyloxy radicals.

8. Compounds according to either of Claims 1 and 2, **characterized in that** the aryl radical is chosen from a phenyl or naphthyl radical which may be mono- or disubstituted with a halogen atom, a radical CF₃, an alkyl radical having from 1 to 12 carbon atoms, an alkoxy radical having from 1 to 6 carbon atoms, a nitro functional group, a polyether radical, a hydroxyl radical optionally protected by an acetyl or benzoyl group or an amino functional group optionally protected by an acetyl or benzoyl group or optionally substituted with at least one alkyl having from 1 to 12 carbon atoms.

9. Compounds according to either of Claims 1 and 2, **characterized in that** the aralkyl radical is chosen from a benzyl or phenethyl radical which may be mono-or disubstituted with a halogen atom, a radical CF₃, an alkyl radical having from 1 to 12 carbon atoms, an alkoxy radical having from 1 to 6 carbon atoms, a nitro functional group, a polyether radical, a hydroxyl radical optionally protected by an acetyl or benzoyl group or an amino functional group optionally protected by an acetyl or benzoyl group or optionally substituted with at least one alkyl having from 1 to 12 carbon atoms.

10. Compounds according to either of Claims 1 and 2, **characterized in that** the heteroaryl radical is chosen from the group consisting of a pyridyl, furyl, thienyl and isoxazolyl radical, optionally substituted with at least one halogen atom, an alkyl radical having from 1 to 12 carbon atoms, an alkoxy radical having from 1 to 6 carbon atoms, a nitro functional group, a polyether radical, a hydroxyl radical optionally protected by an acetyl or benzoyl group or an amino functional group optionally protected by an acetyl or benzoyl group or optionally substituted with at least one alkyl radical having from 1 to 12 carbon atoms.

11. Compounds according to either of Claims 1 and 2, **characterized in that** the heterocycle is chosen from the group consisting of a piperidino, morpholino, pyrrolidino or piperazino radical optionally substituted with an alkyl radical having from 1 to 12 carbon atoms.

12. Compounds according to Claim 1, **characterized in that** they are chosen, alone or in the form of a mixture, from the group consisting of:
1- methyl 7-{[4'-(2,4-dioxothiazolidin-5-ylmethyl)-biphenyl-3-ylmethyl]methylcarbamoyl}heptanoate;
2- methyl 9-{[4'-(2,4-dioxothiazolidin-5-ylmethyl)-biphenyl-3-ylmethyl]methylcarbamoyl}nonanoate;
3- methyl N-[4'-(2,4-dioxothiazolidin-5-ylmethyl)-biphenyl-3-ylmethyl]-N-methylterephthalamate;
4- 3-cyclopentyl-N-[4'-(2,4-dioxothiazolidin-5-ylmethyl)biphenyl-3-ylmethyl]-N-methylpropionamide;
5- N-[4'-(2,4-dioxothiazolidin-5-ylmethyl)biphenyl-3-ylmethyl]-N-methylnaphthalene-1-carboxamide;
6- N-[4'-(2,4-dioxothiazolidin-5-ylmethyl)biphenyl-3-ylmethyl]-N-methylnaphthalene-2-carboxamide;
7- N-[4'-(2,4-dioxothiazolidin-5-ylmethyl)biphenyl-3-ylmethyl]-N-methyl-2-phenoxyacetamide;
8- N-[4'-(2,4-dioxothiazolidin-5-ylmethyl)biphenyl-3-ylmethyl]-N-methyl-1-methyl-1H-pyrrole-2-carboxamide;
9- N-[4'-(2,4-dioxothiazolidin-5-ylmethyl)biphenyl-3-ylmethyl]-N-methyladamantane-1-carboxamide;
10- N-[4'-(2,4-dioxothiazolidin-5-ylmethyl)biphenyl-3-ylmethyl]-N-methylbiphenyl-4-carboxamide;
11- N-[4'-(2,4-dioxothiazolidin-5-ylmethyl)biphenyl-3-ylmethyl]-N-methylbenzo[b]thiophene-2-carboxamide;
12- N-[4'-(2,4-dioxothiazolidin-S-ylmethyl)biphenyl-3-ylmethyl]-N-methyl-6-oxo-6-phenylhexanamide;
13- 4-dimethylamino-N-[4'-(2,4-dioxothiazolidin-5-ylmethyl)biphenyl-3-ylmethyl]-N-methylnaphthalene-1-carboxamide;
14- N-[4'-(2,4-dioxothiazolidin-5-ylmethyl)biphenyl-3-ylmethyl]-4-methanesulphonyl-N-methylbenzamide;
15- N-[4'-(2,4-dioxothiazolidin-5-ylmethyl)biphenyl-3-ylmethyl]-N-methyl-4-(1-phenylmethanoyl)benzamide;
16- 6-(2-methoxyethoxymethoxy)-N-[4'-(2,4-dioxothiazolidin-5-ylmethyl)biphenyl-3-ylmethyl]-N-methylnaphthalene-2-carboxamide;
17- 6-hydroxy-N-[4'-(2,4-dioxothiazolidin-5-ylmethyl)-biphenyl-3-ylmethyl]-N-methylnaphthalene-2-carboxamide;
18- N-[4'-(2,4-dioxothiazolidin-5-ylmethyl)biphenyl-3-ylmethyl]-N-methyl-4-methylsulphariylbenzamide;
19- (S)-3-(3'-{[(1-biphenyl-4-ylmethanoyl)methyl-amino]methyl}biphenyl-4-yl)-2-ethoxypropionic acid;
20- (s) -2-ethoxy-3- (3'-{[methyl- (6-oxo-6-phenylhexanoyl)amino]methyl}biphenyl-4-yl)propionic acid;
21- 1-[4'-(2,4-dioxothiazolidin-5-ylmethyl)biphenyl-3-yl]-1-methyl-3-naphthalen-2-ylurea:
22- 3-(4-dimethylaminophenyl)-1-[4'-(2,4-dioxothiazolidin-5-ylmethyl)biphenyl-3-yl]-1-methylurea;
23- (S)-2-ethoxy-3-{3'-[({1-[6-(2-methoxyethoxymethoxy)naphthalen-2-yl]methanoyl}methylamino)methyl]-biphenyl-4-yl}propionic acid;
24- 6- (methoxymethoxy) -N- [4'- (2, 4-dioxothiazolidin-5-ylmethyl)biphenyl-3-ylmethyl]-N-methylnaphthalene-2-carboxamide;
25- 6-(methoxycarbonyl)-N-[4'y(2,4-dioxothiazolidin-5-ylmethyl)biphenyl-3-ylmethyl]-N-methylnaphthalene-2-carboxamide;
26- 6-(propyloxy)-N-[4'-(2,4-dioxothiazolidin-5-ylmethyl)biphenyl-3-ylmethyl]-N-methylnaphthalene-2-carboxamide;
27- 6-(hexyloxy)-N-[4'-(2,4-dioxothiazolidin-5-ylmethyl)biphenyl-3-ylmethyl]-N-methylnaphthalene-2-carboxamide;
28- 6-(nonyloxy)-N-[4'-(2,4-dioxothiazolidin-5-ylmethyl)biphenyl-3-ylmethyl]-N-methylnaphthalene-2-carboxamide;
29- N-[4'-(2,4-dioxothiazolidin-5-ylmethyl)biphenyl-3-ylmethyl]-N-methyl-4'-propylbiphenyl-2-carboxamide;
30- N-[4'-(2,4-dioxothiazolidin-5-ylmethyl)biphenyl-3-ylmethyl]-N-methyl-4-phenoxybenzamide;
31- N-[4'-(2,4-dioxothiazolidin-5-ylmethyl)biphenyl-3-ylmethyl]-N-methyl-7-oxo-7-phenylheptanamide;
32- (6-{[4'-(2,4-dioxothiazolidin-5-ylmethyl)biphenyl-3-ylmethyl]methylcarbamoyl}naphthalen-2-yloxy)acetic acid;
33- (6-{[4'-(2,4-dioxothiazolidin-5-ylmethyl)biphenyl-3-ylmethyl]methylcarbamoyl}naphthalen-2-yloxy)acetic acid methyl ester;
34- 6-methoxy-N-[4'-(2,4-dioxothiazolidin-5-ylmethyl) biphenyl-3-ylmethyl]-N-methylnaphthalene-2-carboxamide;
35- 6-acetoxy-N-[4'-(2,4-dioxothiazolidin-5-ylmethyl)biphenyl-3-ylmethyl]-N-methylnaphthalene-2-carboxamide;
36- 6-amino-N-[4'-(2,4-dioxothiazolidin-5-ylmethyl) biphenyl-3-ylmethyl] -N-methylnaphthalene-2-carboxamide;
37- 6-acetylamino-N-[4'-(2,4-dioxothiazolidin-5-ylmethyl)biphenyl-3-ylmethyl]-N-methylnaphthalene-2-carboxamide;
38- 1-hydroxy-N-[4'-(2,4-dioxothiazolidin-5-ylmethyl)biphenyl-3-ylmethyl]-N-methylnaphthalene-2-carboxamide;
39- 1-methoxy-N-[4'-(2,4-dioxothiazolidin-5-ylmethyl)biphenyl-3-ylmethyl]-N-methylnaphthalene-2-carboxamide;
40- 6-bromo-N-[4'-(2,4-dioxothiazolidin-5-ylmethyl)biphenyl-3-ylmethyl]-N-methylnaphthalene-2-carboxamide;
41- 6-caxboxyl-N-[4'-(2,4-dioxothiazolidin-5-ylmethyl)biphenyl-3-ylmethyl]-N-methylnaphthalene-2-carboxamide;
42- 6-carboxyl-N-[4'-(2,4-dioxothiazolidin-5-ylmethyl)biphenyl-3-ylmethyl]-N-methylnaphthalene-2-carboxamide methyl ester;
43- 6-(3-phenylureido)-N-[4'-(2,4-dioxothiazolidin-5-ylmethyl)biphenyl-3-ylmethyl]-N-methylnaphthalene-2-carboxamide;
44- 3-hydroxy-N-[4'-(2,4-dioxothiazolidin-5-ylmethyl)biphenyl-3-ylmethyl]-N-methylnaphthalene-2-carboxamide;
45- 3-methoxy-N-[4'-(2,4-dioxothiazolidin-5-ylmethyl)biphenyl-3-ylmethyl]-N-methylnaphthalene-2-carboxamide;
46- N-[4'-(2,4-dioxothiazolidin-5-ylmethyl)biphenyl-3-ylmethyl]-N-methyl-4'-hydroxybiphenyl-4-carboxamide;
47- N-[4'-(2,4-dioxothiazolidin-5-ylmethyl)biphenyl-3-ylmethyl]-N-methyl-4'-methoxybiphenyl-4-carboxamide;
48- N-[4'-(2,4-dioxothiazolidin-5-ylmethyl)biphenyl-3-ylmethyl]-N-methyl-4'-propyloxybiphenyl-4-carboxamide;
49- N-[4'-(2,4-dioxothiazolidin-5-ylmethyl)biphenyl-3-ylmethyl]-N-methyl-4'-hexyloxybiphenyl-4-carboxamide;
50- N-[4'-(2,4-dioxothiazolidin-5-ylmethyl)biphenyl-3-ylmethyl]-N-methyl-4'-acetoxybiphenyl-4-carboxamide;
51- (4'-{[4'-(2,4-dioxothiazolidin-5-ylmethyl)biphenyl-3-ylmethyl]methylcarbamoyl}biphenyl-4-yloxy)acetic acid;
52- (4'-{[4'-(2,4-dioxothiazolidin-5-ylmethyl)biphenyl-3-ylmethyl]methylcarbamoyl}biphenyl-4-yloxy)acetic acid methyl ester;
53- N-[4'-(2,4-dioxothiazolidin-5-ylmethyl)biphenyl-3-ylmethyl]-N-methyl-4'-methoxymethoxybiphenyl-4-carboxamide;
54- N-[4'-(2,4-dioxothiazolidin-5-ylmethyl)biphenyl-3-ylmethyl]-N-methyl-4'-nonyloxybiphenyl-4-carboxamide;
55- N-[4'-(2,4-dioxothiazolidin-5-ylmethyl)biphenyl-3-ylmethyl]-N-methyl-4'-(2-methoxyethoxy)biphenyl-4-carboxamide;
56- 3-biphenyl-4-yl-1-[4'-(2,4-dioxothiazolidin-5-ylmethyl)biphenyl-3-yl]-1-methylurea;
57- 1-[4'-(2,4-dioxothiazolidin-5-ylmethyl)biphenyl-3-yl]-3-(9H-fluoren-2-yl)-1-methylurea;
58- 1-[4'-(2,4-dioxothiazolidin-5-ylmethyl)biphenyl-3-yl]-3-(9H-fluoren-9-yl)-1-methylurea;
59- 3-benzhydryl-1-[4'-(2,4-dioxothiazolidin-5-ylmethyl)biphenyl-3-yl]-1-methylurea;
60- 1-[4'-(2,4-dioxothiazolidin-5-ylmethyl)biphenyl-3-yl]-1-methyl-3-(3-phenoxyphenyl)urea;
61- 1-[4'-(2,4-dioxothiazolidin-5-ylmethyl)biphenyl-3-yl]-3-(4-heptyloxyphenyl)-1-methylurea;
62- 3-(4-benzyloxyphenyl)-1-[4'-(2,4-dioxothiazo7.idin-5-ylmethyl)biphenyl-3-yl]-1-methylurea;
63- 1-[4'-(2,4-dioxothiazolidin-5-ylmethyl)biphenyl-3-yl]-1-methyl-3-[4-(6-methylbenzothiazol-2-yl)phenyl]urea;
64- 4'-(2-methoxyethoxymethoxy)-N-[4'-(2,4-dioxothiazolidin-5-ylmethyl)biphenyl-3-ylmethyl]-N-methylbiphenyl-4-carboxamide;
65- 4'-hydroxy-N-[4'-(2,4-dioxothiazolidin-5-ylmethyl)biphenyl-3-ylmethyl]-N-methylbiphenyl-4-carboxamide;
66- 1-[4'-(2,4-dioxothiazolidin-5-ylmethyl)biphenyl-3-yl]-3-(4-hexyloxyphenyl)-1-methylurea.

13. Compounds according to Claim 1 or 2, **characterized in that** they exhibit one of the following characteristics:
- R₁ represents the radical of formula (a) or the radical of formula (b) where R₅ represents a hydroxyl radical and R₆ represents the radical OR₁₄ and/or
- X represents the linkage having the structure -CH₂-N(R₈)-CO- or -N(R₈)-CO-N(R₉)- read from left to right or conversely.

14. Compounds according to any one of Claims 1 to 13, as a medicament.

15. Use of a compound according to any one of Claims 1 to 13, in the manufacture of a composition intended for regulating and/or restoring skin lipid metabolism.

16. Use of a compound according to any one of Claims 1 to 13, in the manufacture of a composition intended for the treatment:
- of dermatological conditions linked to a keratinization disorder related to cell differentiation and proliferation;
- of ichtyosis, ichtyosiform states, Darrier's disease, keratosis palmaris et plantaris, leukoplasia and leukoplasiform states, cutaneous or mucosal (buccal) lichen;
- of dermatological conditions with an inflammatory immunoallergic component, with or without cell proliferation disorder;
- of benign or malignant dermal or epidermal proliferations, of viral or nonviral origin;
- of proliferations which may be induced by ultraviolet radiation;
- of precancerous skin lesions;
- of immune dermatoses;
- of bullous immune diseases;
- of collagen diseases;
- of dermatological or general conditions with an immunological component;
- of skin disorders due to exposure to UV radiation, skin ageing, photoinduced or chronological or actinic pigmentations and keratoses;
- of pathologies associated with chronological or actinic ageing;
- of sebaceous function disorders;
- of cicatrization disorders or of stretch marks; or
- of pigmentation disorders.

17. Use according to Claim 16, **characterized in that** the dermatological conditions linked to a keratinization disorder are acne vulgaris, comedo-type acne, polymorphic acne, acne rosacea, nodulocystic acne, acne conglobata, senile acne, secondary acne such as solar acne, acne medicamentosa or occupational acne.

18. Use according to Claim 16, **characterized in that** the dermatological conditions with an inflammatory immunoallergic component are cutaneous, mucosal or ungual psoriasis, psoriatic rheumatism or cutaneous atopy, such as eczema, respiratory atopy or gingival hypertrophy.

19. Use according to Claim 16, **characterized in that** the dermal or epidermal proliferations are verruca vulgaris, verruca plana and epidermodysplasia verruciformis, oral or florid papillomatoses or T lymphoma.

20. Use according to Claim 16, **characterized in that** the proliferations which may be induced by ultraviolet radiation are baso- and spinocellular epitheliomas.

21. Use according to Claim 16, **characterized in that** the precancerous skin lesions are keratoacanthomas.

22. Use according to Claim 16, **characterized in that** the immune dermatoses are lupus erythematosus.

23. Use according to Claim 16, **characterized in that** the collagen diseases are scleroderma.

24. Use according to Claim 16, **characterized in that** the pathologies associated with chronological or actinic ageing are xerosis.

25. Use according to Claim 16, **characterized in that** the sebaceous function disorders are acne hyperseborrhoea or simple seborrhoea.

26. Use according to Claim 16, **characterized in that** the pigmentation disorders are hyperpigmentation, melasma, hypopigmentation and vitiligo.

27. Pharmaceutical composition, **characterized in that** it comprises, in a physiologically acceptable carrier, at least one of the compounds as defined in any one of Claims 1 to 13.

28. Composition according to Claim 27, **characterized in that** the concentration of compound(s) according to one of Claims 1 to 13 is between 0.001% and 10% by weight relative to the total weight of the composition.

29. Composition according to Claim 27, **characterized in that** the concentration of compound(s) according to one of Claims 1 to 13 is between 0.01% and 1% by weight relative to the total weight of the composition.

30. Cosmetic composition, **characterized in that** it comprises, in a cosmetically acceptable carrier, at least one of the compounds as defined in any one of Claims 1 to 13.

31. Composition according to Claim 30, **characterized in that** the concentration of compound(s) according to one of Claims 1 to 13 is between 0.001% and 3% by weight relative to the total weight of the composition.

32. Cosmetic use of a composition as defined in either of Claims 30 and 31, for preventing and/or treating the signs of ageing and/or dry skin.

33. Cosmetic use of a composition as defined in either of Claims 30 and 31, for body or hair hygiene.

## Patentansprüche

1. Verbindungen, **dadurch gekennzeichnet, dass** sie der folgenden Formel (I) entsprechen: 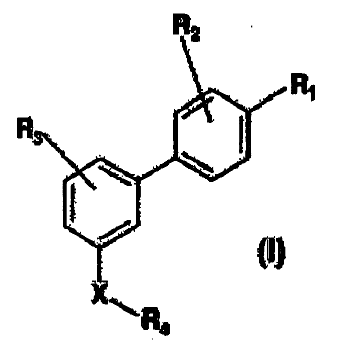 worin bedeuten:
- R₁ eine Gruppe der folgenden Formeln (a) oder (b): 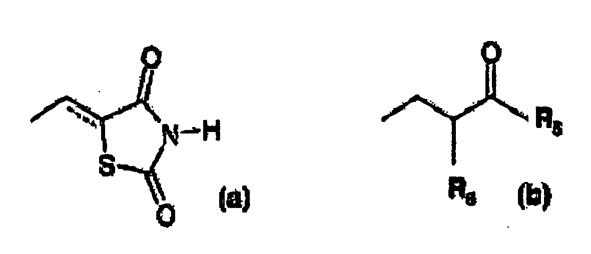
wobei R₅ und R₆ die nachstehend angegebenen Bedeutungen aufweisen,
- R₂ und R₃, die gleich oder verschieden sind, ein Wasserstoffatom, eine Alkylgruppe mit 1 bis 6 Kohlenstoffatomen, eine Arylgruppe, ein Halogenatom, eine Gruppe -OR₇, eine Polyethergruppe, eine Nitrogruppe oder eine Aminogruppe, die gegebenenfalls mit Alkylgruppen, die 1 bis 6 Kohlenstoffatomen aufweisen können, substituiert sein kann; wobei R₇ die nachstehend angegebenen Bedeutungen aufweist,
- X Bindungen der folgenden Strukturen:
-CH₂-N(R₈)-CO-
-N(R₈)-CO-N(R₉)-
-N(R₈)-CO-CH₂-
-N(R₈)-CH₂-CO-
wobei diese Gruppen von links nach rechts oder umgekehrt gelesen werden können und wobei R₈ und R₉ die nachstehend angegebenen Bedeutungen aufweisen,
- R₄ bedeutet:
- Phenyl, Benzyl, Phenethyl, Thienyl, Furyl oder Pyridyl,
wobei alle diese Gruppen mit einer Gruppe R₁₀ substituiert sind,
wobei R₁₀ die nachstehend angegebenen Bedeutungen aufweist,
- Pyrrolyl, Pyrazinyl, Naphthyl, Biphenyl, Indolyl, Indenyl, Benzothienyl, Benzofuryl, Benzothiazolyl oder Chinolyl,
wobei alle diese Gruppen mit einer Gruppe R₁₁ und/ oder R₁₂ mono- oder disubstituiert sein können;
wobei R₁₁ und R₁₂ die nachstehend angegebenen Bedeutungen aufweisen,
- eine Gruppe -(CH₂)ₙ-(CO)_{q}R₁₃,
wobei n, q und R₁₃ die nachstehend angegebenen Bedeutungen aufweisen,
- Adamantyl, Diphenylmethyl, Diphenylethyl, Diphenylpropyl, Diphenylbutyl, Cyclopropylmethyl, Cyclopentylethyl, 2-Benzimidazolylethyl, Cyclohexylmethyl, Phenoxyphenyl, 9H-Fluorenyl, Benzyloxyphenyl, 4-Heptyloxyphenyl oder 4-(6-Methyl-2-benzothiazolyl)phenyl;
- eine Gruppe -(CH₂)ₙ-O-R₁₃,
wobei n und R₁₃ die nachstehend angegebenen Bedeutungen aufweisen,
- R₅ eine Hydroxygruppe oder eine Alkoxygruppe mit 1 bis 9 Kohlenstoffatomen,
- R₆ eine Alkylgruppe mit 1 bis 6 Kohlenstoffatomen, eine Gruppe OR₁₄ oder eine Gruppe SR₁₄,
wobei R₁₄ die nachstehend angegebenen Bedeutungen aufweist,
- R₇ ein Wasserstoffatom, eine Alkylgruppe mit 1 bis 6 Kohlenstoffatomen, eine Arylgruppe oder eine Aralkylgruppe,
- R₈ ein Wasserstoffatom oder eine Alkylgruppe mit 1 bis 6 Kohlenstoffatomen,
- R₉ ein Wasserstoffatom oder eine Alkylgruppe mit 1 bis 6 Kohlenstoffatomen,
- Rio bedeutet: eine Gruppe -S(O)ₘR₁₅
eine Gruppe -(CH₂)ₚ-COR₁₆
eine Gruppe -O-R₁₇,
wobei m, p, R₁₅, R₁₆ und R₁₇ die nachstehend angegebenen Bedeutungen aufweisen,
- R₁₁ und R₁₂ ein Halogenatom, die Gruppe CF₃, eine Alkylgruppe mit 1 bis 12 Kohlenstoffatomen, eine Alkoxygruppe mit 1 bis 9 Kohlenstoffatomen, eine Polyethergruppe, eine Nitrofunktion, eine Hydroxygruppe, die gegebenenfalls geschützt ist mit einer Acetylgruppe, Benzoyl, eine Aminofunktion, die gegebenenfalls mit mindestens einer Alkylgruppe mit 1 bis 12 Kohlenstoffatomen oder mit einer Gruppe -CONH-R₂₄ substituiert oder mit einer Acetylgruppe oder Benzoylgruppe geschützt ist, -S(O)ₘR₁₅, (CH₂)ₚ-COR₁₆ oder -OR₁₇,
wobei m, p, R₁₅, R₁₆, R₁₇ und R₂₄ die nachstehend angegebenen Bedeutungen aufweisen,
- n kann Werte im Bereich von 1 bis 9 annehmen,
- q kann die Werte 0 oder 1 annehmen,
- R₁₃ -OR₁₈, -N(R₁₉)(R₂₀), Aryl, Aralkyl oder Heteroaryl,
wobei R₁₈, R₁₉ und R₂₀ die nachstehend angegebenen Bedeutungen aufweisen,
- m kann die Werte 0, 1 oder 2 annehmen,
- p kann die Werte 0, 1 oder 2 annehmen,
- R₁₄ eine Alkylgruppe mit 1 bis 12 Kohlenstoffatomen, CF₃, Aryl oder Aralkyl,
- R₁₅ eine Alkylgruppe mit 1 bis 12 Kohlenstoffatomen, eine Arylgruppe oder eine Aralkylgruppe,
- R₁₆ eine Alkylgruppe mit 1 bis 12 Kohlenstoffatomen, -OR₂₁, -N(R₂₂)(R₂₃), eine Arylgruppe oder eine Aralkylgruppe,
wobei R₂₁, R₂₂ und R₂₃ die nachstehend angegebenen Bedeutungen aufweisen,
- R₁₇ eine Arylgruppe oder eine Aralkylgruppe,
- R₁₈ ein Wasserstoffatom oder eine Alkylgruppe mit 1 bis 12 Kohlenstoffatomen,
- R₁₉ und R₂₀, die gleich oder verschieden sind, ein Wasserstoffatom, eine Alkylgruppe mit 1 bis 12 Kohlenstoff atomen oder die beiden Gruppen bilden gemeinsam einen Heterocyclus,
- R₂₁ ein Wasserstoffatom oder eine Alkylgruppe mit 1 bis 12 Kohlenstoffatomen,
- R₂₂ und R₂₃, die gleich oder verschieden sind, ein Wasserstoffatom, eine Alkylgruppe mit 1 bis 12 Kohlenstoffatomen oder sie können gemeinsam einen Heterocyclus bilden;
- R₂₄ Phenyl, Diphenylmethyl, Diphenylpropyl, Diphenylbutyl, Biphenylyl, Phenoxyphenyl, 9H-Fluorenyl, 4-Benzyloxyphenyl, 4-Heptyloxyphenyl oder 4-(6-Methyl-2-benzothiazolyl)phenyl,
und die Salze der Verbindungen der Formel (I), wenn R₁ eine Carbonsäurefunktion enthält, sowie die optischen und geometrischen Isomere der Verbindungen der Formel (I).

2. Verbindungen nach Anspruch 1, **dadurch gekennzeichnet, dass** sie in Form von Alkalimetallsalzen oder Erdalkalimetallsalzen, Zinksalzen oder Salzen eines organischen Amins vorliegen.

3. Verbindungen nach einem der Ansprüche 1 oder 2, **dadurch gekennzeichnet, dass** die Alkylgruppen mit 1 bis 6 Kohlenstoffatomen unter den Gruppen Methyl, Ethyl, Isopropyl, Butyl, t-Butyl und Hexyl ausgewählt sind.

4. Verbindungen nach einem der Ansprüche 1 oder 2, **dadurch gekennzeichnet, dass** die Alkylgruppen mit 1 bis 12 Kohlenstoffatomen unter den Gruppen Methyl, Ethyl, Isopropyl, Butyl, *t*-Butyl, Hexyl, Octyl, Decyl oder Dodecyl ausgewählt sind.

5. Verbindungen nach einem der Ansprüche 1 oder 2, **dadurch gekennzeichnet, dass** die Polyethergruppen unter den Polyethergruppen mit 1 bis 6 Kohlenstoffatomen, die durch mindestens ein Sauerstoffatom unterbrochen sind, ausgewählt sind, wie Methoxymethoxy, Ethoxymethoxy oder Methoxyethoxymethoxy.

6. Verbindungen nach einem der Ansprüche 1 oder 2, **dadurch gekennzeichnet, dass** das Halogenatom unter Fluor, Chlor oder Brom ausgewählt ist.

7. Verbindungen nach einem der Ansprüche 1 oder 2, **dadurch gekennzeichnet, dass** die Alkoxygruppe mit 1 bis 9 Kohlenstoffatomen unter den Gruppen Methoxy, Ethoxy, Isopropyloxy, *t*-Butoxy oder Hexyloxy ausgewählt ist.

8. Verbindungen nach einem der Ansprüche 1 oder 2, **dadurch gekennzeichnet, dass** die Arylgruppe unter einer Phenylgruppe oder Naphthylgruppe ausgewählt ist, die mono- oder disubstituiert sein kann mit einem Halogenatom, der Gruppe CF₃, einer Alkylgruppe mit 1 bis 12 Kohlenstoffatomen, einer Alkoxygruppe mit 1 bis 6 Kohlenstoffatomen, einer Nitrofunktion, einer Polyethergruppe, einer Hydroxygruppe, die gegebenenfalls mit einer Acetylgruppe geschützt sein kann, Benzoyl oder einer Aminofunktion, die gegebenenfalls mit einer Acetylgruppe, Benzoyl geschützt oder gegebenenfalls mit mindestens einer Alkylgruppe mit 1 bis 12 Kohlenstoffatomen substituiert ist.

9. Verbindungen nach einem der Ansprüche 1 oder 2, **dadurch gekennzeichnet, dass** die Aralkylgruppe unter Benzyl oder Phenethyl ausgewählt ist, die mono- oder disubstituiert sein können mit einem Halogenatom, einer Gruppe CF₃, einer Alkylgruppe mit 1 bis 12 Kohlenstoffatomen, einer Alkoxygruppe mit 1 bis 6 Kohlenstoffatomen, einer Nitrofunktion, einer Polyethergruppe, einer Hydroxygruppe, die gegebenenfalls geschützt ist mit einer Acetylgruppe, Benzoyl oder einer Aminofunktion, die gegebenenfalls geschützt ist mit einer Acetylgruppe, Benzoyl oder gegebenenfalls mit mindestens einer Alkylgruppe mit 1 bis 12 Kohlenstoffatomen substituiert ist.

10. Verbindungen nach einem der Ansprüche 1 oder 2, **dadurch gekennzeichnet, dass** die Heteroarylgruppe unter den Gruppen Pyridyl, Furyl, Thienyl oder Isoxazolyl ausgewählt ist, die gegebenenfalls substituiert sind mit mindestens einem Halogenatom, einer Alkylgruppe mit 1 bis 12 Kohlenstoffatomen, einer Alkoxygruppe mit 1 bis 6 Kohlenstoffatomen, einer Nitrofunktion, einer Polyethergruppe, einer Hydroxygruppe, die gegebenenfalls geschützt ist mit einer Acetylgruppe, Benzoyl oder einer Aminofunktion, die gegebenenfalls geschützt ist mit einer Acetylgruppe, Benzoyl oder gegebenenfalls mit mindestens einer Alkylgruppe mit 1 bis 12 Kohlenstoffatomen substituiert ist.

11. Verbindung nach einem der Ansprüche 1 oder 2, **dadurch gekennzeichnet, dass** der Heterocyclus unter den Gruppen Piperidino, Morpholino, Pyrrolidino oder Piperazino ausgewählt ist, die gegebenenfalls mit einer Alkylgruppe mit 1 bis 12 Kohlenstoff atomen substituiert sind.

12. Verbindungen nach Anspruch 1, **dadurch gekennzeichnet, dass** sie einzeln oder im Gemisch unter den folgenden Verbindungen ausgewählt sind:
1- Methyl-7-{[4'-(2,4-dioxo-thiazolidin-5-ylmethyl)-biphenyl-3-ylmethyl]-methyl-carbamoyl}-heptanoat;
2- Methyl-9-{[4'-(2,4-dioxo-thiazolidin-5-ylmethyl)-biphenyl-3-ylmethyl]-methyl-carbamoyl}-nonanoat;
3- Methyl-N-[4'-(2,4-dioxo-thiazolidin-5-ylmethyl)-biphenyl-3-ylmethyl]-N-methyl-terephthalamat;
4- 3-Cyclopentyl-N-[4'-(2,4-dioxo-thiazolidin-5-ylmethyl)-biphenyl-3-ylmethyl]-N-methylpropionamid;
5- N-[4'-(2,4-Dioxo-thiazolidin-5-ylmethyl)-biphenyl-3-yl-methyl]-N-methyl-naphthalin-1-carboxamid;
6- N-[4'-(2,4-Dioxo-thiazolidin-5-ylmethyl)-biphenyl-3-yl-methyl]-N-methyl-naphthalin-2-carboxamid;
7- N-[4'-(2,4-Dioxo-thiazolidin-5-ylmethyl)-biphenyl-3-yl-methyl]-N-methyl-2-phenoxy-acetamid;
8- N-[4'-(2,4-Dioxo-thiazolidin-5-ylmethyl)-biphenyl-3-yl-methyl]-N-methyl-1-methyl-1H-pyrrol-2-carboxamid;
9- N-[4'-(2,4-Dioxo-thiazolidin-5-ylinethyl)-biphenyl-3-yl-methyl]-N-methyl-adamantan-1-carboxamid;
10- N-[4'-(2,4-Dioxo-thiazolidin-5-ylmethyl)-biphenyl-3-yl-methyl]-N-methyl-biphenyl-4-carboxamid;
11- N-[4'-(2,4-Dioxo-thiazolidin-5-ylmethyl)-biphenyl-3-yl-methyl]-N-methyl-benzo[b]thiophen-2-carboxamid;
12- N-[4'-(2,4-Dioxo-thiazolidin- 5-ylmethyl)-biphenyl-3-yl-methyl]-N-methyl-6-oxo-6-phenylhexanamid;
13- 4-Dimethylamino-N-[4'-(2,4-dioxo-thiazolidin-5-ylmethyl)-biphenyl-3-ylmethyl]-N-methylnaphthalin-1-carboxamid;
14- N-[4'-(2,4-Dioxo-thiazolidin-5-ylmethyl)-biphenyl-3-yl-methyl]-4-methansulfonyl-N-methylbenzamid;
15- N-[4'-(2,4-Dioxo-thiazolidin-5-ylmethyl)-biphenyl-3-yl-methyl]-N-methyl-4-(1-phenyl-methanoyl)-benzamid;
16- 6-(2-Methoxy-ethoxymethoxy)-N-[4'-(2,4-dioxo-thiazolidin-5-ylmethyl)-biphenyl-3-ylmethyl]-N-methyl-napthalidin-2-carboxamid;
17- 6-Hydroxy-N-[4'-(2,4-dioxo-thiazolidin-5-ylmethyl)-biphenyl-3-ylmethyl]-N-methyl-napthalin-2-carboxamid;
18- N-[4'-(2,4-Dioxo-thiazolidin-5-ylmethyl)-biphenyl-3-yl-methyl]-N-methyl-4-methylsulfanyl-benzamid;
19- (S)-3-(3'-{[(1-Biphenyl-4-yl-methanoyl)-methyl-amino]-methyl}-biphenyl-4-yl)-2-ethoxy-propionsäure;
20- (S)-2-Ethoxy-3-(3'{[methyl-(6-oxo-6-phenyl-hexanoyl)-amino]-methyl}-biphenyl-4-yl)-propiorisäure;
21- 1-[4'-(2,4-Dioxo-thiazolidin-5-ylmethyl)-biphenyl-3-yl]-1-methyl-3-naphthalin-2-yl-harnstoff;
22- 3,(4-Dimethylamino-phenyl)-1-[4'-(2,4-dioxo-thiazolidin-5-ylmethyl)-biphenyl-3-yl]-1-methylharnstoff;
23- (S)-2-Ethoxy-3-{3'-[({1-[6-(2-methoxy-ethoxymethoxy)-naphthalm-2-yl]-methanoyl}-methyl-amino)-methyl]-biphenyl-4-yl}-propionsäure;
24- 6-(Methoxymethoxy)-N-[4'-(2,4-dioxo-thiazolidin-5-ylmethyl)-biphenyl-3-ylmethyl] -N-methyl-naphthalin-2 -carboxamid;
25- 6-(Methoxycarbonyl)-N-[4'-(2,4-dioxo-thiazolidin-5-ylmethyl)-biphenyl-3-ylmethyl]-N-methyl-naphthalin-2-carboxamid;
26- 6-(Propyloxy)-N-[4'-(2,4-dioxo-thiazolidin-5-ylmethyl)-biphenyl-3-ylmethyl]-N-methyl-naphthalin-2-carboxamid;
27- 6-(Hexyloxy)-N-[4'-(2,4-dioxo-thiazolidin-5-yl,methyl)-biphenyl-3-ylmethyl]-N-methyl-naphthalin-2-carboxamid;
28- 6-(Nonyloxy)-N-[4'-(2,4-dioxo-thiazolidin-5-ylmethyl)-biphenyl-3-ylmethyl]-N-methyl-naphthalin-2-carboxamid;
29- N-[4'-(2,4-Dioxo-thiazolidin-5-ylmethyl)-biphenyl-3-yl-methyl]-N-methyl-4'-propyl-biphenyl-2-carboxamid;
30- N-[4'-(2,4-Dioxo-thiazolidin-5-ylmethyl)-biphenyl-3-y1-methyl]-N-rnethyl-4-phenoxy-benzamid;
31- N-[4'-(2,4-Dioxo-thiazolidin-5-ylmethyl)-biphenyl-3-yl-methyl]-N-methyl-7-oxo-7-phenylheptanamid;
32- (6-{[4'-(2,4-Dioxo-thiazolidin-5-yhnethyl)-biphenyl-3-yl-methyl]-methyl-carbomoyl}-napthalin-2-yloxy)-essigsäure;
33- Methylester der (6-{[4'-(2,4-Dioxo-thiazolidin-5-ylmethyl)-biphenyl-'3-ylmethyl]-methyl-carbomoyl}-napthalin-2-yloxy)-essigsäure;
34- 6-Methoxy-N-[4'-(2,4-dioxo-thiazolidin-5-ylmethyl)-biphenyl-3-ylmethyl]-N-methyl-naphthalin-2-carboxamid;
35- 6-Acetoxy-N-[4'-(2,4-dioxo-thiazolidin-5-ylmethyl)-biphenyl-3-ylmethyl]-N-methyl-naphthalin-2-carboxamid;
36- 6-Amino-N-[4'-(2,4-dioxo-thiazolidin-5-ylmethyl)-biphenyl-3-ylmethyl]-N-methyl-naphthalin-2-carboxamid;
37- 6-Acetylaxnino-N-[4'-(2,4-dioxo-thiazolidin-5-ylmethyl)-biphenyl-3-ylmethyl]-N-methyl-naphthalin-2-carboxamid;
38- 1-Hydroxy-N-[4'-(2,4-dioxo-thiazolidin-5-ylmethyl)-biphenyl-3-ylmethyl]-N-methyl-naphthalin-2-carbox.amid;
39- 1-Methoxy-N-[4'-(2,4-dioxo-thiazolidin-5-ylmethyl)-biphenyl-3-ylmethyl]-N-methyl-naphthalin-2-carboxamid;
40- 6-Brom-N-[4'-(2,4-dioxo-thiazolidin-5-ylmethyl)-biphenyl-3-ylmethyl]-N-rnethyl-naphthalin-2-carboxamid;
41- 6-Carboxy-N-[4'-(2,4-dioxo-thiazolidin-5-ylmethyl)-biphenyl-3-ylmethyl]-N-methyl-naphthalin-2-carboxamid;
42- Methylester der 6-Carboxy-N-[4'-(2,4-dioxo-thiazolidin-5-ylmethyl)-biphenyl-3-ylmethyl]-N-methyl-naphthalin-2-carboxamid;
43- 6-(3-Phenyl-ureido)-N-[4'-(2,4-dioxo-thiazolidin-5-ylmethyl)-biphenyl-3-ylmethyl]-N-methyl-napthalin-2-carboxamid;
44- 3-Hydroxy-N-[4'-(2,4-dioxo-thiazolidin-5-ylmethyl)-biphenyl-3-ylmethyl]-N-methyl-napthalin-2-carboxamid;
45- 3-Methoxy-N-[4'-(2,4-dioxo-thiazolidin-5-ylmethyl)-biphenyl-3-ylmethyl]-N-methyl-napthalin-2-carboxamid;
46- N-[4'-(2,4-Dioxo-thiazolidin-5-ylmethyl)-biphenyl-3-yl-methyl]-N-methyl-4'-hydroxy-biphenyl-4-carboxamid;
47- N-[4'-(2,4-Dioxo-thiazolidin-5-ylmethyl)-biphenyl-3-yl-methyl]-N-methyl-4'-methoxy-biphenyl-4-carboxamid;
48- N-[4'-(2,4-Dioxo-thiazolidin-5-ylmethyl)-biphenyl-3-yl-methyl]-N-methyl-4'-propyloxy-biphenyl-4-carboxamid;
49- N-[4'-(2,4-Dioxo-thiazolidin-5-ylmethyl)-biphenyl-3-yl-methyl]-N-methyl-4'-hexyloxy-biphenyl-4-carboxamid;
50- N-[4'-(2,4-Dioxo-thiazolidin-5-ylmethyl)-biphenyl-3-yl-methyl]-N-methyl-4'-acetoxy-biphenyl-4-carboxamid;
51- (4'-{[4'-(2,4-Dioxo-thiazolidin-5-ylmethyl)-biphenyl-3-yl-methyl]-methyl-carbamoyl}-biphenyl-4-yloxy)-essigsäure;
52- Methylester der (4'-{[4'-(2,4-Dioxo-thiazolidin-5-ylmethyl)-biphenyl-3-ylmethyl]-methyl-carbamoyl}-biphenyl-4-yloxy)-essigsäure;
53- N-[4'-(2,4-Dioxo-thiazolidin-5-ylmethyl)-biphenyl-3-yl-methyl]-N-methyl-4'-methoxymethoxy-biphenyl-4-carboxamid;
54- N-[4'-(2,4-Dioxo-thiazolidin-5-ylmethyl)-biphenyl-3-yl-methyl]-N-methyl-4'-nonyloxy-biphenyl-4-carboxamid;
55- N-[4'-(2,4-Dioxo-thiazolidin-5-ylmethyl)-biphenyl-3-yl-methyl]-N-methyl-4'-(2-methoxyethoxy)-biphenyl-4-carboxamid;
56- 3-Biphenyl-4-yl-1-[4'-(2,4-dioxo-thiazolidin-5-ylmethyl)-biphenyl-3-yl]-1-methylharnstoff;
57- 1-[4'-(2,4-Dioxo-thiazolidin-5-ylmethyl)-biphenyl-3-yl]-3-(9H-fluoren-2-yl)-1-methylhamstoff;
58- 1-[4'-(2,4-Dioxo-thiazolidin-5-ylmethyl)-biphenyl-3-yl]-3-(9H-fluoren-9-yl)-1-methylharnstoff;
59- 3-Benzhydryl-1-[4'-(2,4-dioxo-thiazolidin-5-ylmethyl)-biphenyl-3-yl] 1-methylharnstoff;
60- 1-[4'-(2,4-Dioxo-thiazolidin-5-ylmethyl)-biphenyl-3-yl]-1-methyl-3-(3-phenoxy-phenyl)-hamstoff;
61- 1-[4'-(2,4-Dioxo-thiazohdin-5-ylmethyl)-biphenyl-3-yl]-3-(4-heptyloxy-phenyl)-1-methylhamstoff;
62- 3-(4-Benzyloxy-phenyl)-1-[4'-(2,4-dioxo-thiazolidin-5-yl-methyl)-biphenyl-3-yl]-1-methylharnstoff;
63- 1-[4'-(2,4-Dioxo-thiazolidin-5-ylmethyl)-biphenyl-3-yl]-1-methyl-3-[4-(6-methyl-benzothiazol-2-yl)-phenyl]-harnstoff;
64- 4'-(2-Methoxy-ethoxymethoxy)-N-[4'-(2,4-dioxo-thiazolidin-5-ylmethyl)-biphenyl-3-ylmethyl]-N-methyl-biphenyl-4-carboxamid;
65- 4'-Hydroxy-N-[4'-(2,4-dioxo-thiazolidin-5-ylmethyl)-biphenyl-3-ylmethyl]-N-methyl-biphenyl-4-carboxamid;
66- 1-[4'-(2,4-Dioxo-thiazolidin-5-ylmethyl)-biphenyl-3-yl]-3-(4-hexyloxy-phenyl)-1-methyl-harnstoff.

13. Verbindungen nach Anspruch 1 oder 2, **dadurch gekennzeichnet, dass** sie eines der folgenden Merkmale aufweisen:
- R₁ bedeutet die Gruppe der Formel (a) oder die Gruppe der Formel (b), worin R₅ eine Hydroxygruppe und R₆ die Gruppe OR₁₄ bedeutet, und/oder
- X bedeutet die Bindung der Struktur -CH₂-N(R₈)-CO- oder N(R₈)-CO-N(R₉)-, die von links nach rechts oder umgekehrt gelesen werden können.

14. Verbindungen nach einem der Ansprüche 1 bis 13 als Arzneimittel.

15. Verwendung einer Verbindung nach einem der Ansprüche 1 bis 13 für die Herstellung einer Zusammensetzung, die dazu vorgesehen ist, den Metabolismus der Hautlipide zu regulieren und/oder wiederherzustellen.

16. Verwendung einer Verbindung nach einem der Ansprüche 1 bis 13 zur Herstellung einer Zusammensetzung, die vorgesehen ist für die Behandlung von:
- dermatologischen Erkrankungen, die mit einer Verhornungsstörung verbunden sind, die auf der Differenzierung und Proliferation der Zellen beruht;
- Ichtyosis, ichtyosisartigen Zuständen, der Darier Krankheit, Palmoplantarkeratosen, Leukoplakien und leucoplakieformen Zuständen und Lichen der Haut oder der Schleimhäute (buccal);
- dermatologischen Erkrankungen mit entzündlicher immunoallergischer Komponente, mit oder ohne einer Störung der Proliferation der Zellen;
- Proliferationen der Dermis oder Epidermis, die gutartig oder bösartig und gegebenenfalls viralen Ursprungs sein können;
- Proliferationen, die von UV-Strahlung induziert sein können;
- präkanzerösen Hautläsionen;
- Immundermatosen;
- bullösen Immunerkrankungen;
- Kollagenerkrankungen;
- dermatologischen oder allgemeinen Erkrankungen mit immunologischer Komponente;
- Hautstörungen, die mit einer UV-Exposition zusammenhängen; altersbedingter oder lichtinduzierter Hautalterung, aktionischen Pigmentierungen oder Keratosen;
- Pathologien, die mit der altersbedingten oder aktinischen Hautalterung einhergehen;
- Störungen der Talgdrüsenfunktion;
- Störungen der Wundheilung oder Streifen;
- Pigmentierungsstörungen.

17. Verwendung nach Anspruch 16, **dadurch gekennzeichnet, dass** die dermatologischen Erkrankungen, die mit einer Keratinisiexungsstörung zusammenhängen, Acne vulgaris, Acne comedonica, polymorphe Acne, Acne rosaceae, nodulocystische Acne, Acne conglobata, Acne senilis oder sekundäre Akneformen, wie Acne solaris, Acne medicamentosa oder Acne professionalis sind.

18. Verwendung nach Anspruch 16, **dadurch gekennzeichnet, dass** es sich bei den dermatologischen Erkrankungen mit immunoallergischer entzündlicher Komponente um Psoriasis der Haut, der Schleimhäute oder der Nägel, Psoriasis arthropathica, Atopie der Haut, wie Ekzeme, Atopie der Atemwege oder Hypertrophie des Zahnfleisches handelt.

19. Verwendung nach Anspruch 16, **dadurch gekennzeichnet, dass** es sich bei den Proliferationen der Dermis oder der Epidermis um Verrucae vulgares, Verrucae planae und Epidermodysplasia verruciformis, Papillomatosis oralis oder florida oder das T-Lymphom handelt.

20. Verwendung nach Anspruch 16, **dadurch gekennzeichnet, dass** die Proliferationen, die durch UV-Strahlung induziert werden können, Epithelioma basocellulare und spinocellulare sind.

21. Verwendung nach Anspruch 16, **dadurch gekennzeichnet, dass** die präkanzerösen Hautläsionen Keratoacanthome sind.

22. Verwendung nach Anspruch 16, **dadurch gekennzeichnet, dass** es sich bei den Immundermatosen um Lupus erythematodes handelt.

23. Verwendung nach Anspruch 16, **dadurch gekennzeichnet, dass** es sich bei den Collagenerkrankungen um Sklerodermie handelt.

24. Verwendung nach Anspruch 16, **dadurch gekennzeichnet, dass** es sich bei den mit der altersbedingten oder aktinischen Alterung zusammenhängenden Erkrankungen um Xerose handelt.

25. Verwendung nach Anspruch 16, **dadurch gekennzeichnet, dass** die Störungen der Talgdrüsenfunktion Hyperseborrhoe bei Akne oder Seborrhoe simplex sind.

26. Verwendung nach Anspruch 16, **dadurch gekennzeichnet, dass** die Pigznentierungsstörungen Hyperpigmentierung, Melasmen, Hypopigmentierung oder Vitiligo sind.

27. Pharmazeutische Zusammensetzung, **dadurch gekennzeichnet, dass** sie in einem physiologisch akzeptablen Trägerstoff mindestens eine Verbindung nach einem der Ansprüche 1 bis 13 enthält.

28. Zusammensetzung nach Anspruch 27, **dadurch gekennzeichnet, dass** die Konzentration der Verbindung(en) nach einem der Ansprüche 1 bis 13 im Bereich von 0,001 bis 10 Gew.-%, bezogen auf das Gesamtgewicht der Zusammensetzung, liegt.

29. Zusammensetzung nach Anspruch 27, **dadurch gekennzeichnet, dass** die Konzentration der Verbindung(en) nach einem der Ansprüche 1 bis 13 im Bereich von 0,01 bis 1 Gew.-%, bezogen auf das Gesamtgewicht der Zusammensetzung, liegt.

30. Kosmetische Zusammensetzung, **dadurch gekennzeichnet, dass** sie in einem kosmetisch akzeptablen Träger mindestens eine Verbindung nach einem der Ansprüche 1 bis 13 enthält.

31. Zusammensetzung nach Anspruch 30, **dadurch gekennzeichnet, dass** die Konzentrationen der Verbindung(en) nach einem der Ansprüche 1 bis 13 im Bereich von 0,001 bis 3 Gew.-%, bezogen auf das Gesamtgewicht der Zusammensetzung, liegt.

32. Kosmetische Verwendung einer Zusammensetzung nach einem der Ansprüche 30 oder 31 zur Vorbeugung und/oder Behandlung der Alterszeichen und/oder trockener Haut.

33. Kosmetische Verwendung einer Zusammensetzung nach einem der Ansprüche 30 oder 31 für die Körper- oder Haarpflege.
